# EUROPEAN PATENT SPECIFICATION

(11) **EP 2 912 020 B1**
(45) Date of publication and mention of the grant of the patent: **04.12.2019**
(21) Application number: 13783783.7
(22) Date of filing: 17.10.2013
(51) Int. Cl.: A61K 45/06, A61K 31/415, A61K 31/4192, A61K 31/4439

(54) **SUBSTITUTED TOLYL FUNGICIDES**
SUBSTITUIERTE TOLYLFUNGIZIDE
FONGICIDES À BASE DE TOLYLE SUBSTITUÉ

(30) Priority: 25.10.2012 US 201261718221 P
(43) Date of publication of application: 02.09.2015
(73) Proprietor: FMC Corporation, Philadelphia, PA 19104 (US)
(72) Inventor: TAGGI, Andrew, Edmund, New Hope, Pennsylvania 18938 (US); DIETRICH, Robert, F., Wilmington, Delaware 19810 (US); MARCUS, Kimberly, Katherine, Media, Pennsylvania 19063 (US); MCCANN, Stephen, Frederick, Newark, Delaware 19711 (US)
(74) Representative: Dehns
(86) International application number: PCT/US2013/065336
(87) International publication number: WO 2014/066120

(56) References cited:
- WO-A2-2008/124092

## Description

### FIELD OF THE INVENTION

This invention relates to certain substituted tolyl fungicides, their *N*-oxides, salts and compositions, and methods of their use as fungicides.

### BACKGROUND OF THE INVENTION

The control of plant diseases caused by fungal plant pathogens is extremely important in achieving high crop efficiency. Plant disease damage to ornamental, vegetable, field, cereal, and fruit crops can cause significant reduction in productivity and thereby result in increased costs to the consumer. Many products are commercially available for these purposes, but the need continues for new compounds which are more effective, less costly, less toxic, environmentally safer or have different sites of action.

PCT Patent Publication WO2008/124092 discloses fungicidal compounds of Formula i 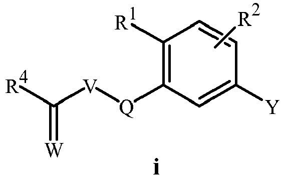 wherein Y is, *inter alia,* selected from twenty-four 5- or 6-membered heteroaromatic rings optionally substituted with a phenyl ring or a 5- or 6-membered heteroaromatic ring.

The particular substituted tolyl fungicides of the present invention are not disclosed in this publication.

### SUMMARY OF THE INVENTION

This invention is directed to compounds of Formula 1 (including all stereoisomers), *N*-oxides, and salts thereof, agricultural compositions containing them and their use as fungicides: 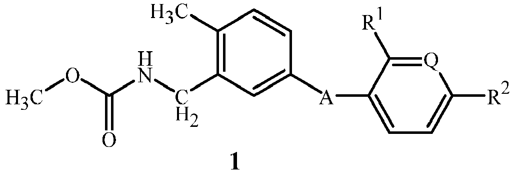 wherein
A is a radical selected from the group consisting of 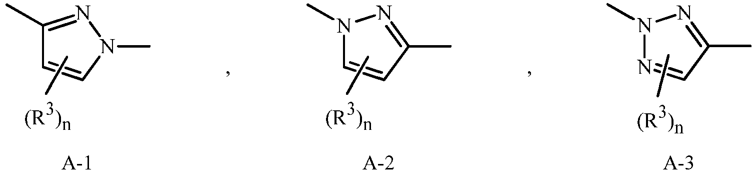 and 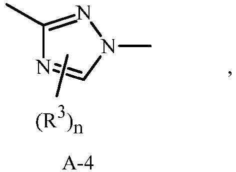 wherein the bond extending to the left of the A group is attached to the phenyl group having the CH₃O(C=O)NHCH₂ and the bond extending to the right of the A group is attached to the phenyl group having R¹ and R² substituents in Formula 1;
Q is CH or N;
R¹ is halogen, cyano, hydroxy, nitro, amino, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
R² is halogen, cyano, hydroxy, nitro, amino, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
each R³ is independently halogen, cyano, C₁-C₃ alkyl, C₁-C₃ haloalkyl, C₁-C₃ alkoxy or C₁-C₃ haloalkoxy; and
n is 0, 1 or 2.

More particularly, this invention pertains to a compound of Formula 1 (including all stereoisomers), an *N*-oxide or a salt thereof.

This invention also relates to a fungicidal composition comprising (a) a compound of the invention (i.e. in a fungicidally effective amount); and (b) at least one additional component selected from the group consisting of surfactants, solid diluents and liquid diluents.

This invention also relates to a fungicidal composition comprising (a) a compound of the invention; and (b) at least one other fungicide (e.g., at least one other fungicide having a different site of action).

This invention further relates to a method for controlling plant diseases caused by fungal plant pathogens comprising applying to the plant or portion thereof, or to the plant seed, a fungicidally effective amount of a compound of the invention (e.g., as a composition described herein).

The aforedescribed method can also be described as a method for protecting a plant or plant seed from diseases caused by fungal pathogens comprising applying a fungicidally effective amount of a compound of Formula 1, an *N*-oxide, or a salt thereof (e.g., as a composition described herein), to the plant (or portion thereof) or plant seed (directly or through the environment (e.g., growing medium) of the plant or plant seed).

### DETAILS OF THE INVENTION

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process, method, article, or apparatus that comprises a list of elements is not necessarily limited to only those elements but may include other elements not expressly listed or inherent to such composition, mixture, process, method, article, or apparatus.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition, method or apparatus that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of'.

Where applicants have defined an invention or a portion thereof with an open-ended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As referred to in the present disclosure and claims, "plant" includes members of Kingdom Plantae, particularly seed plants (Spermatopsida), at all life stages, including young plants (e.g., germinating seeds developing into seedlings) and mature, reproductive stages (e.g., plants producing flowers and seeds). Portions of plants include geotropic members typically growing beneath the surface of the growing medium (e.g., soil), such as roots, tubers, bulbs and corms, and also members growing above the growing medium, such as foliage (including stems and leaves), flowers, fruits and seeds.

As referred to herein, the term "seedling", used either alone or in a combination of words means a young plant developing from the embryo of a seed.

As referred to in this disclosure, the terms "fungal pathogen" and "fungal plant pathogen" include pathogens in the Basidiomycete, Ascomycete, Oomycete and Deuteromycete classes that are the causal agents of a broad spectrum of plant diseases of economic importance, affecting ornamental, turf, vegetable, field, cereal and fruit crops. In the context of this disclosure, "protecting a plant from disease" or "control of a plant disease" includes preventative action (interruption of the fungal cycle of infection, colonization, symptom development and spore production) and/or curative action (inhibition of colonization of plant host tissues).

As referred to in this disclosure, the term mode of action (MOA) is as defined broadly by the Fungicide Resistance Action Committee (FRAC), and is used to distinguish fungicide groups according to their biochemical mode of action in the biosynthetic pathways of plant pathogens. These FRAC-defined MOAs are (A) nucleic acid synthesis, (B) mitosis and cell division, (C) respiration, (D) amino acid and protein synthesis, (E) signal transduction, (F) lipid synthesis and membrane integrity, (G) sterol biosynthesis in membranes, (H) cell wall biosynthesis in membranes, (I) melanin synthesis in cell wall, (P) host plant defense induction, multi-site contact activity and unknown mode of action. Each MOA class consists of one or more groups based either on individual validated target sites of action, or in cases where the precise target site is unknown, based on cross resistance profiles within a group or in relation to other groups. Each of these groupings within a FRAC-defined MOA, whether the target site is known or unknown, is designated by a FRAC code. Additional information on target sites and FRAC codes can be found on the following FRAC website: http://www.frac.info/.

As referred to in this disclosure, the term "cross resistance" refers to a phenomenon wherein a pathogen evolves resistance to one fungicide and in addition acquires resistance to others. These additional fungicides are typically, but not always, in the same chemical class or have the same target site of action, or can be detoxified by the same mechanism.

In the above recitations, the term "alkyl", used either alone or in compound words such as "alkylthio" or "haloalkyl" includes straight-chain or branched alkyl such as methyl, ethyl, n-propyl, *i*-propyl, or the different butyl, pentyl or hexyl isomers. "Alkenyl" includes straight-chain or branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as ethynyl, 1-propynyl, 2-propynyl and the different butynyl, pentynyl and hexynyl isomers. "Alkynyl" also includes moieties comprised of multiple triple bonds such as 2,5-hexadiynyl.

"Alkoxy" includes, for example, methoxy, ethoxy, n-propyloxy, isopropyloxy and the different butoxy, pentoxy and hexyloxy isomers. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. "Alkoxyalkoxy" denotes alkoxy substitution on alkoxy. "Alkenyloxy" includes straight-chain or branched alkenyloxy moieties. Examples of "alkenyloxy" include H₂C=CHCH₂O, (CH₃)₂C=CHCH₂O, (CH₃)CH=CHCH₂O, (CH₃)CH=C(CH₃)CH₂O and CH₂=CHCH₂CH₂O. "Alkynyloxy" includes straight-chain or branched alkynyloxy moieties. Examples of "alkynyloxy" include HC≡CCH₂O, CH₃C≡CCH₂O and CH₃C≡CCH₂CH₂O. "Alkylthio" includes branched or straight-chain alkylthio moieties such as methylthio, ethylthio, and the different propylthio, butylthio, pentylthio and hexylthio isomers. "Alkylsulfinyl" includes both enantiomers of an alkylsulfinyl group. Examples of "alkylsulfinyl" include CH₃S(O)-, CH₃CH₂S(O)-, CH₃CH₂CH₂S(O)-, (CH₃)₂CHS(O)- and the different butylsulfinyl, pentylsulfinyl and hexylsulfinyl isomers. Examples of "alkylsulfonyl" include CH₃S(O)₂-, CH₃CH₂S(O)₂-, CH₃CH₂CH₂S(O)₂-, (CH₃)₂CHS(O)₂-, and the different butylsulfonyl, pentylsulfonyl and hexylsulfonyl isomers.

"Cycloalkyl" includes, for example, cyclopropyl, cyclobutyl, cyclopentyl and cyclohexyl. The term "cycloalkylalkyl" denotes cycloalkyl substitution on an alkyl moiety. Examples of "cycloalkylalkyl" include cyclopropylmethyl, cyclopentylethyl, and other cycloalkyl moieties bonded to straight-chain or branched alkyl groups. The term "cycloalkoxy" denotes cycloalkyl linked through an oxygen atom such as cyclopentyloxy and cyclohexyloxy.

"Cyanoalkyl" denotes an alkyl group substituted with one cyano group. Examples of "cyanoalkyl" include NCCH₂, NCCH₂CH₂ and CH₃CH(CN)CH₂. "Cyanoalkoxy" denotes cyano substitution on alkoxy. Examples of "cyanoalkoxy" include NCCH₂O, NCCH₂CH₂O, and CH₃CH(CN)CH₂O.

The term "halogen", either alone or in compound words such as "haloalkyl", or when used in descriptions such as "alkyl substituted with halogen" includes fluorine, chlorine, bromine or iodine. Further, when used in compound words such as "haloalkyl", or when used in descriptions such as "alkyl substituted with halogen" said alkyl may be partially or fully substituted with halogen atoms which may be the same or different. Examples of "haloalkyl" or "alkyl substituted with halogen" include F₃C-, ClCH₂-, CF₃CH₂- and CF₃CCl₂-. The terms "halocycloalkyl", "haloalkoxy", "haloalkylthio", "haloalkenyl", "haloalkynyl", and the like, are defined analogously to the term "haloalkyl". Examples of "haloalkoxy" include CF₃O-, CCl₃CH₂O-, HCF₂CH₂CH₂O- and CF₃CH₂O-. Examples of "haloalkylthio" include CCl₃S-, CF₃S-, CCl₃CH₂S- and ClCH₂CH₂CH₂S-. Examples of "haloalkylsulfinyl" include CF₃S(O)-, CCl₃S(O)-, CF₃CH₂S(O)- and CF₃CF₂S(O)-. Examples of "haloalkylsulfonyl" include CF₃S(O)₂-, CCl₃S(O)₂-, CF₃CH₂S(O)₂- and CF₃CF₂S(O)₂-. Examples of "haloalkenyl" include (Cl)₂C=CHCH₂- and CF₃CH₂CH=CHCH₂-. Examples of "haloalkynyl" include HC≡CCHCl-, CF₃C≡C-, CCl₃C≡C- and FCH₂C≡CCH₂-. Examples of "haloalkoxyalkoxy" include CF₃OCH₂O-, ClCH₂CH₂OCH₂CH₂O-, Cl₃CCH₂OCH₂O- as well as branched alkyl derivatives.

The total number of carbon atoms in a substituent group is indicated by the "Cᵢ-Cⱼ" prefix where i and j are numbers from 1 to 8. For example, C₁-C₄ alkylsulfonyl designates methylsulfonyl through butylsulfonyl. For example, C₁-C₃ alkoxy designates CH₃O-, CH₃CH₂O-, CH₃CH₂CH₂O and (CH₃)₂CHO)-.

When a compound is substituted with a substituent bearing a subscript that indicates the number of said substituents can exceed 1, said substituents (when they exceed 1) are independently selected from the group of defined substituents, e.g., (R³)ₙ, n is 0, 1 or 2. When a variable group is shown to be optionally attached to a position, for example (R³)ₙ wherein n may be 0, then hydrogen may be at the position even if not recited in the variable group definition. When one or more positions on a group are said to be "not substituted" or "unsubstituted", then hydrogen atoms are attached to take up any free valency.

The terms "carbocyclic ring", "carbocycle" or "carbocyclic ring system" denote a ring or ring system wherein the atoms forming the ring backbone are selected only from carbon. Unless otherwise indicated, a carbocyclic ring can be a saturated, partially unsaturated, or fully unsaturated ring. When a fully unsaturated carbocyclic ring satisfies Hückel's rule, then said ring is also called an "aromatic ring". "Saturated carbocyclic" refers to a ring having a backbone consisting of carbon atoms linked to one another by single bonds; unless otherwise specified, the remaining carbon valences are occupied by hydrogen atoms.

The terms "heterocyclic ring", "heterocycle" or "heterocyclic ring system" denote a ring or ring system in which at least one atom forming the ring backbone is not carbon, e.g., nitrogen, oxygen or sulfur. Typically a heterocyclic ring contains no more than 4 nitrogens, no more than 2 oxygens and no more than 2 sulfurs. Unless otherwise indicated, a heterocyclic ring can be a saturated, partially unsaturated, or fully unsaturated ring. When a fully unsaturated heterocyclic ring satisfies Hückel's rule, then said ring is also called a "heteroaromatic ring" or "aromatic heterocyclic ring". Unless otherwise indicated, heterocyclic rings and ring systems can be attached through any available carbon or nitrogen by replacement of a hydrogen on said carbon or nitrogen.

"Aromatic" indicates that each of the ring atoms is essentially in the same plane and has a *p*-orbital perpendicular to the ring plane, and that (4n + 2) π electrons, where n is a positive integer, are associated with the ring to comply with Hückel's rule. The term "aromatic ring system" denotes a carbocyclic or heterocyclic ring system in which at least one ring of the ring system is aromatic. The term "aromatic carbocyclic ring system" denotes a carbocyclic ring system in which at least one ring of the ring system is aromatic. The term "aromatic heterocyclic ring system" denotes a heterocyclic ring system in which at least one ring of the ring system is aromatic. The term "nonaromatic ring system" denotes a carbocyclic or heterocyclic ring system that may be fully saturated, as well as partially or fully unsaturated, provided that none of the rings in the ring system are aromatic. The term "nonaromatic carbocyclic ring system" in which no ring in the ring system is aromatic. The term "nonaromatic heterocyclic ring system" denotes a heterocyclic ring system in which no ring in the ring system is aromatic.

The term "optionally substituted" in connection with the heterocyclic rings refers to groups which are unsubstituted or have at least one non-hydrogen substituent that does not extinguish the biological activity possessed by the unsubstituted analog. As used herein, the following definitions shall apply unless otherwise indicated. The term "optionally substituted" is used interchangeably with the phrase "substituted or unsubstituted" or with the term "(un)substituted." Unless otherwise indicated, an optionally substituted group may have a substituent at each substitutable position of the group, and each substitution is independent of the other.

A wide variety of synthetic methods are known in the art to enable preparation of aromatic and nonaromatic heterocyclic rings and ring systems; for extensive reviews see the eight volume set of Comprehensive Heterocyclic Chemistry, A. R. Katritzky and C. W. Rees editors-in-chief, Pergamon Press, Oxford, 1984 and the twelve volume set of Comprehensive Heterocyclic Chemistry II, A. R. Katritzky, C. W. Rees and E. F. V. Scriven editors-in-chief, Pergamon Press, Oxford, 1996.

Compounds of this invention can exist as one or more stereoisomers. The various stereoisomers include enantiomers, diastereomers, atropisomers and geometric isomers. Stereoisomers are isomers of identical constitution but differing in the arrangement of their atoms in space and include enantiomers, diastereomers, cis-trans isomers (also known as geometric isomers) and atropisomers. Atropisomers result from restricted rotation about single bonds where the rotational barrier is high enough to permit isolation of the isomeric species. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, individual stereoisomers or as an optically active form. For a comprehensive discussion of all aspects of stereoisomerism, see Ernest L. Eliel and Samuel H. Wilen, Stereochemistry of Organic Compounds, John Wiley & Sons, 1994.

Molecular depictions drawn herein follow standard conventions for depicting stereochemistry. To indicate stereoconfiguration, bonds rising from the plane of the drawing and towards the viewer are denoted by solid wedges wherein the broad end of the wedge is attached to the atom rising from the plane of the drawing towards the viewer. Bonds going below the plane of the drawing and away from the viewer are denoted by dashed wedges wherein the narrow end of the wedge is attached to the atom further away from the viewer. Constant width lines indicate bonds with a direction opposite or neutral relative to bonds shown with solid or dashed wedges; constant width lines also depict bonds in molecules or parts of molecules in which no particular stereoconfiguration is intended to be specified.

Compounds of Formula 1 can comprise chiral centers. For example, substituents such as R¹ and R² may themselves contain chiral centers. This invention comprises racemic mixtures as well as enriched and essentially pure stereoconfigurations at these chiral centers.

Compounds of this invention can exist as one or more conformational isomers due to restricted rotation about the amide bond (e.g., C(W)-N) in Formula 1. This invention comprises mixtures of conformational isomers. In addition, this invention includes compounds that are enriched in one conformer relative to others.

One skilled in the art will appreciate that not all nitrogen containing heterocycles can form *N*-oxides since the nitrogen requires an available lone pair for oxidation to the oxide; one skilled in the art will recognize those nitrogen-containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and m-chloroperbenzoic acid (MCPBA), hydrogen peroxide, alkyl hydroperoxides such as *t*-butyl hydroperoxide, sodium perborate, and dioxiranes such as dimethyldioxirane. These methods for the preparation of *N*-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

One skilled in the art recognizes that because in the environment and under physiological conditions salts of chemical compounds are in equilibrium with their corresponding nonsalt forms, salts share the biological utility of the nonsalt forms. Thus a wide variety of salts of the compounds of Formula **1** are useful for control of plant diseases caused by fungal plant pathogens (i.e. are agriculturally suitable). The salts of the compounds of Formula **1** include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. When a compound of Formula **1** contains an acidic moiety such as a carboxylic acid or phenol, salts also include those formed with organic or inorganic bases such as pyridine, triethylamine or ammonia, or amides, hydrides, hydroxides or carbonates of sodium, potassium, lithium, calcium, magnesium or barium. Accordingly, the present invention comprises compounds selected from Formula **1,** *N*-oxides and agriculturally suitable salts thereof.

Compounds selected from Formula **1,** stereoisomers, tautomers, *N*-oxides, and salts thereof, typically exist in more than one form, and Formula **1** thus includes all crystalline and non-crystalline forms of the compounds that Formula **1** represents. Non-crystalline forms include embodiments which are solids such as waxes and gums as well as embodiments which are liquids such as solutions and melts. Crystalline forms include embodiments which represent essentially a single crystal type and embodiments which represent a mixture of polymorphs (i.e. different crystalline types). The term "polymorph" refers to a particular crystalline form of a chemical compound that can crystallize in different crystalline forms, these forms having different arrangements and/or conformations of the molecules in the crystal lattice. Although polymorphs can have the same chemical composition, they can also differ in composition due to the presence or absence of co-crystallized water or other molecules, which can be weakly or strongly bound in the lattice. Polymorphs can differ in such chemical, physical and biological properties as crystal shape, density, hardness, color, chemical stability, melting point, hygroscopicity, suspensibility, dissolution rate and biological availability. One skilled in the art will appreciate that a polymorph of a compound represented by Formula **1** can exhibit beneficial effects (e.g., suitability for preparation of useful formulations, improved biological performance) relative to another polymorph or a mixture of polymorphs of the same compound represented by Formula **1.** Preparation and isolation of a particular polymorph of a compound represented by Formula **1** can be achieved by methods known to those skilled in the art including, for example, crystallization using selected solvents and temperatures. For a comprehensive discussion of all aspects of stereoisomerism, see Ernest L. Eliel and Samuel H. Wilen, Stereochemistry of Organic Compounds, John Wiley & Sons, 1994.

Embodiments of the present invention as described in the Summary of the Invention include (where Formula **1** as used in the following Embodiments includes *N*-oxides and salts thereof):
Embodiment 1. A compound of Formula 1 wherein A is a radical selected from the group consisting of A¹, A² and A³.
Embodiment 2. A compound of Embodiment 1 wherein A is A¹.
Embodiment 3. A compound of Embodiment 1 wherein A is A².
Embodiment 4. A compound of Embodiment 1 wherein A is A³.
Embodiment 5. A compound of Formula **1** or any one of Embodiments 1 through 4 either alone or in combination, wherein R¹ is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy.
Embodiment 5a. A compound of Embodiment 5 wherein R¹ is halogen, C₁-C₆ alkyl, C₁-C₆ alkoxy or C₁-C₆ haloalkoxy.
Embodiment 6. A compound of Embodiment 5 wherein R¹ is halogen, C₁-C₆ alkyl, or C₁-C₆ alkoxy.
Embodiment 6a. A compound of Embodiment 5a wherein R¹ is C₁-C₆ alkyl, C₁-C₆ alkoxy or C₁-C₆ haloalkoxy.
Embodiment 7. A compound of Embodiment 6 wherein R¹ is fluoro, chloro, C₁-C₃ alkyl or C₁-C₃ alkoxy.
Embodiment 7a. A compound of Embodiment 6a wherein R¹ is C₁-C₃ alkyl or C₁-C₃ alkoxy.
Embodiment 8. A compound of Formula 1 or any one of Embodiments 1 through 7a either alone or in combination, wherein R² is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy.
Embodiment 8a. A compound of Embodiment 8 wherein R² is halogen, C₁-C₆ alkyl, C₁-C₆ alkoxy or C₁-C₆ haloalkoxy.
Embodiment 9. A compound of Embodiment 8 wherein R² is halogen, C₁-C₆ alkyl or C₁-C₆ alkoxy.
Embodiment 9a. A compound of Embodiment 8a wherein R² is C₁-C₆ alkyl, C₁-C₆ alkoxy or C₁-C₆ haloalkoxy.
Embodiment 10. A compound of Embodiment 9 wherein R² is fluoro, chloro, C₁-C₃ alkyl or C₁-C₃ alkoxy.
Embodiment 10a. A compound of Embodiment 9a wherein R² is C₁-C₃ alkyl or C₁-C₃ alkoxy.
Embodiment 11. A compound of Formula **1** or any one of Embodiments 1 through 10a either alone or in combination, wherein each R³ is independently halogen or C₁-C₃ alkyl.
Embodiment 12. A compound of Formula **1** or any one of Embodiments 1 through 11 either alone or in combination, wherein n is 0 or 1.
Embodiment 13. A compound of Embodiment 12 wherein n is 0.
Embodiment 14. A compound of Formula **1** or any one of Embodiments 1 through 13 either alone or in combination, wherein Q is CH.
Embodiment 15. A compound of Formula **1** or any one of Embodiments 1 through 13 either alone or in combination, wherein Q is N.

Also of note is a compound of Formula **1P** 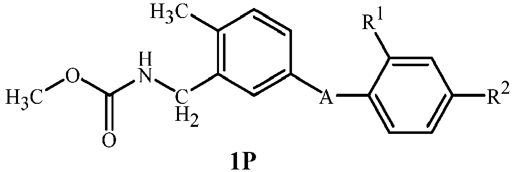 Embodiment AAA. A compound of Formula **1P** wherein
A is a radical selected from the group consisting of 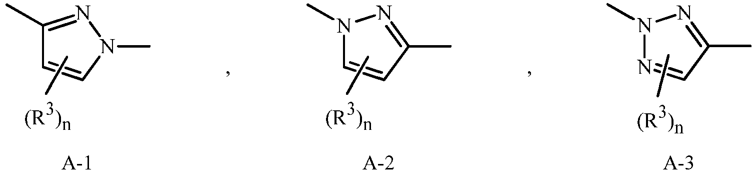 and 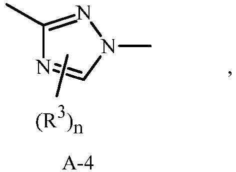 wherein the bond extending to the left of the A group is attached to the phenyl group having the CH₃O(C=O)NHCH₂ and the bond extending to the right of the A group is attached to the phenyl group having R¹ and R² substituents in Formula **1;**
R¹ is halogen, cyano, hydroxy, nitro, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
R² is halogen, cyano, hydroxy, nitro, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
each R³ is independently halogen, cyano, C₁-C₃ alkyl, C₁-C₃ haloalkyl, C₁-C₃ alkoxy or C₁-C₃ haloalkoxy; and
n is 0, 1 or 2.

Embodiments of this invention, including Embodiments 1-15 and AAA above as well as any other embodiments described herein, can be combined in any manner, and the descriptions of variables in the embodiments pertain not only to the compounds of Formula **1** and Formula **1P** but also to the starting compounds and intermediate compounds useful for preparing the compounds of Formula **1** and Formula **1P.** In addition, embodiments of this invention, including Embodiments 1-15 and AAA above as well as any other embodiments described herein, and any combination thereof, pertain to the compositions and methods of the present invention.

Combinations of Embodiments 1-15 and AAA are illustrated by:
Embodiment AA. A compound of Formula **1** as described in the invention wherein
   A is a radical selected from the group consisting of 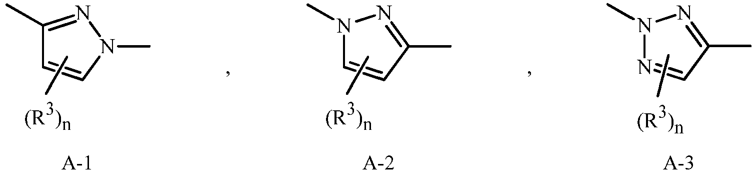 and 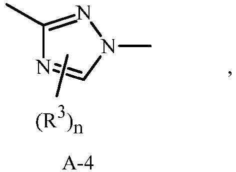 wherein the bond extending to the left of the A group is attached to the phenyl group having the CH₃O(C=O)NHCH₂ and the bond extending to the right of the A group is attached to the phenyl group having R¹ and R² substituents in Formula **1;**
   Q is CH or N;
   R¹ is halogen, cyano, hydroxy, nitro, amino, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
   R² is halogen, cyano, hydroxy, nitro, amino, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
   each R³ is independently halogen, cyano, C₁-C₃ alkyl, C₁-C₃ haloalkyl, C₁-C₃ alkoxy or C₁-C₃ haloalkoxy; and
   n is 0, 1 or 2.
Embodiment A1. A compound of Embodiment AA wherein
   A is a radical selected from the group consisting of A¹, A² and A³;
   Q is CH;
   R¹ is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy;
   R² is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy;
   R³ is halogen or C₁-C₃ alkyl; and
   n is 0 or 1.
Embodiment A. A compound of Embodiment AA or Embodiment AAA wherein
   A is a radical selected from the group consisting of A¹, A² and A³;
   R¹ is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy;
   R² is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy;
   R³ is halogen or C₁-C₃ alkyl; and n is 0 or 1.
Embodiment B. A compound of Embodiment A or A1 wherein
   R¹ is halogen, C₁-C₆ alkyl or C₁-C₆ alkoxy;
   R² is halogen, C₁-C₆ alkyl or C₁-C₆ alkoxy; and
   n is 0.
Embodiment B1. A compound of Embodiment A or A1 wherein
   R¹ is C₁-C₆ alkyl, C₁-C₆ alkoxy or C₁-C₆ haloalkoxy;
   R² is C₁-C₆ alkyl, C₁-C₆ alkoxy or C₁-C₆ haloalkoxy; and
   n is 0.
Embodiment C. A compound of Embodiment B or B1 wherein
   R¹ is fluoro, chloro, C₁-C₃ alkyl or C₁-C₃ alkoxy; and
   R² is fluoro, chloro, C₁-C₃ alkyl or C₁-C₃ alkoxy.
Embodiment C1. A compound of Embodiment B or B1 wherein
   R¹ is C₁-C₃ alkyl or C₁-C₃ alkoxy; and
   R² is C₁-C₃ alkyl or C₁-C₃ alkoxy.
Embodiment D. A compound of Embodiment C or C1 wherein
   A is A¹.
Embodiment E. A compound of Embodiment C or C1 wherein
   A is A².
Embodiment F. A compound of Embodiment C or C1 wherein
   A is A³.

Specific embodiments include compounds of Formula **1** selected from the group consisting of:
methyl *N*-[[5-[1-(4-methoxy-2-methylphenyl)-1*H*-pyrazol-3-yl]-2-methylphenyl]-methyl] carbamate;
methyl *N*-[[5-[3-(4-methoxy-2-methylphenyl)-1*H*-pyrazol-1-yl]-2-methylphenyl]-methyl] carbamate;
methyl *N*-[[5-[1-(2-chloro-4-methoxyphenyl)-1*H*-pyrazol-3-yl]-2-methylphenyl]-methyl] carbamate;
methyl *N*-[[5-[3-(4-chloro-2-methoxyphenyl)-1*H*-pyrazol-1-yl]-2-methylphenyl]-methyl] carbamate;
methyl *N*-[[5-[4-(2,4-dimethoxyphenyl)-2*H*-1,2,3-triazol-2-yl]-2-methylphenyl]-methyl] carbamate;
methyl *N*-[[5-[3-(2,4-dimethoxyphenyl)-1*H*-pyrazol-1-yl]-2-methylphenyl]-methyl] carbamate;
methyl *N-*[[5-[1-(4-chloro-2-methoxyphenyl)-1*H*-pyrazol-3-yl]-2-methylphenyl]-methyl] carbamate;
methyl *N*-[[5-[4-(4-methoxy-2-methylphenyl)-2*H*-1,2,3-triazol-2-yl]-2-methylphenyl]methyl]carbamate; and
methyl *N*-[[5-[3-(2,4-dichlorophenyl)-1*H*-pyrazol-1-yl]-2-methylphenyl]-methyl]carbamate.

Additional specific embodiments include compounds of Formula **1** selected from the group consisting of:
methyl *N*-[[5-[1-(2,4-dimethylphenyl)-1*H*-pyrazol-3-yl]-2-methylphenyl]-methyl] carbamate;
methyl *N*-[[5-[3-(2,4-dimethylphenyl)-1*H*-pyrazol-1-yl]-2-methylphenyl]-methyl] carbamate;
methyl *N*-[[5-[4-(2,4-dimethylphenyl)-2*H*-1,2,3-triazol-2-yl]-2-methylphenyl]-methyl] carbamate;
methyl *N*-[[5-[3-(2,6-dimethoxy-3-pyridinyl)-1*H*-pyrazol-1-yl]-2-methylphenyl]-methyl] carbamate;
methyl *N*-[[5-[3-(6-methoxy-2-methyl-3-pyridinyl)-1*H*-pyrazol-1-yl]-2-methylphenyl]methyl]carbamate;
methyl *N*-[[5-[1-(2,6-dimethoxy-3-pyridinyl)-1*H*-pyrazol-3-yl]-2-methylphenyl]-methyl] carbamate;
methyl *N*-[[5-[1-(6-methoxy-2-methyl-3-pyridinyl)-1*H*-pyrazol-3-yl]-2-methylphenyl] -methyl] carbamate;
methyl *N*-[[5-[4-(2,6-dimethoxy-3-pyridinyl)-2*H*-1,2,3-triazol-2-yl]-2-methylphenyl]methyl] carbamate;
methyl *N*-[[5-[4-(6-methoxy-2-methyl-3-pyridinyl)-2*H*-1,2,3-triazol-2-yl]-2-methylphenyl]methyl] carbamate;
methyl *N*-[[5-[1-(2,4-dimethylphenyl)-1*H*-1,2,4-triazol-3-yl]-2-methylphenyl]methyl] carbamate;
methyl *N*-[[5-[1-(6-methoxy-2-methyl-3-pyridinyl)-1*H*-1,2,4-triazol-3-yl]-2-methylphenyl]methyl] carbamate;
methyl *N*-[[5-[1-(2,6-dimethoxy-3-pyridinyl)-1*H*-1,2,4-triazol-3-yl]-2-methylphenyl]methyl] carbamate;
methyl *N*-[[2-methyl-5-[1-[2-methyl-4-(trifluoromethoxy)phenyl]-1*H*-pyrazol-3-yl]phenyl]methyl]carbamate;
methyl *N*-[[5-[1-[4-(difluoromethoxy)-2-methylphenyl]-1*H*-pyrazol-3-yl]-2-methylphenyl]methyl] carbamate;
methyl *N*-[[2-methyl-5-[3-[2-methyl-4-(trifluoromethoxy)phenyl]-1*H*-pyrazol-1-yl]phenyl]methyl]carbamate;
methyl *N*-[[5-[3-[4-(difluoromethoxy)-2-methylphenyl]-1*H*-pyrazol-1-yl]-2-methylphenyl]methyl]carbamate;
methyl *N*-[[2-methyl-5-[1-[2-methyl-4-(trifluoromethoxy)phenyl]-1*H*-1,2,4-triazol-3 -yl]phenyl]methyl] carbamate;
methyl *N*-[[5-[1-[4-(difluoromethoxy)-2-methylphenyl]-1*H*-1,2,4-triazol-3-yl]-2-methylphenyl]methyl]carbamate;
methyl *N*-[[2-methyl-5-[4-[2-methyl-4-(trifluoromethoxy)phenyl]-2*H*-1,2,3-triazol-2-yl]phenyl]methyl] carbamate; and
methyl *N*-[[5-[4-[4-(difluoromethoxy)-2-methylphenyl]-2*H*-1,2,3-triazol-2-yl]-2-methylphenyl]methyl]carbamate.

This invention provides a fungicidal composition comprising a compound of Formula 1 (including all stereoisomers, *N*-oxides, and salts thereof), and at least one other fungicide. Of note as embodiments of such compositions are compositions comprising a compound corresponding to any of the compound embodiments described above.

This invention provides a fungicidal composition comprising a compound of Formula 1 (including all stereoisomers, *N*-oxides, and salts thereof) (i.e. in a fungicidally effective amount), and at least one additional component selected from the group consisting of surfactants, solid diluents and liquid diluents. Of note as embodiments of such compositions are compositions comprising a compound corresponding to any of the compound embodiments described above.

This invention provides a method for controlling plant diseases caused by fungal plant pathogens comprising applying to the plant or portion thereof, or to the plant seed, a fungicidally effective amount of a compound of Formula 1 (including all stereoisomers, *N*-oxides, and salts thereof). This invention also provides a method for controlling plant diseases caused by Basidiomycete and Ascomycete fungal plant pathogens comprising applying to a plant or portion thereof, or to a plant seed, a fungicidally effective amount of a compound of Formula 1 (including all stereoisomers, *N*-oxides, and salts thereof). This invention also provides a method for controlling plant diseases caused by fungal plant pathogens comprising applying to a transgenic plant or portion thereof, or to a transgenic plant seed, a fungicidally effective amount of a compound of Formula **1** (including all stereoisomers, *N*-oxides, and salts thereof). Of note as embodiments of such methods are methods comprising applying a fungicidally effective amount of a compound corresponding to any of the compound embodiments describe above. Of particular notes are embodiments where the compounds are applied as compositions of this invention.

One or more of the following methods and variations as described in Schemes 1-14 can be used to prepare the compounds of Formula **1.** The definitions of A, Q, R¹, R², R³ and n in the compounds of Formulae **1-24** below are as defined above in the Summary of the Invention unless otherwise noted. Compounds of Formulae **1a-1d** are various subsets of the compounds of Formula **1,** and all substituents for Formulae **1a-1d** are as defined above for Formula **1.**

As shown in Scheme 1, compounds of Formula **1a** (i.e. compounds of Formula **1** wherein A is A-2) with a substituted 3-phenyl pyrazole ring can be prepared via a copper or palladium catalyzed cross-coupling reaction using compounds of Formula **2** wherein X is a bromine, iodine or triflate under Ullmann (Chemical Reviews, 2002, 102, 1359-1470) or Buchwald-Hartwig (Angew. Chem. Int. Ed. 2008, 47, 6338-6361, Chem. Sci., 2010, 1, 13-31) conditions. These reactions traditionally require the presence of a base, such as a metal carbonate like potassium carbonate, a suitable catalyst and ligand combination, such as copper (I) iodide and a ligand such as *trans*-1,2-diamino-*N,N'*-dimethylcyclohexane, and the desired substituted heterocycle (a compound of Formula **3**) in an aprotic solvent such as dioxane or toluene at a temperature between ambient and the boiling point of the solvent.

When X is a boronic acid, then heterocycles of Formula **3** can be coupled to compounds of Formula **2** using Chan-Lam conditions (Tetrahedron Lett., 1998, 39, 2933-2936). These conditions require the presence of a suitable base such as pyridine or triethyl amine, a catalyst such as copper (II) acetate, in an aprotic solvent like dichloromethane or chloroform, at a temperature between ambient and the boiling point of the solvent, in the presence of oxygen.

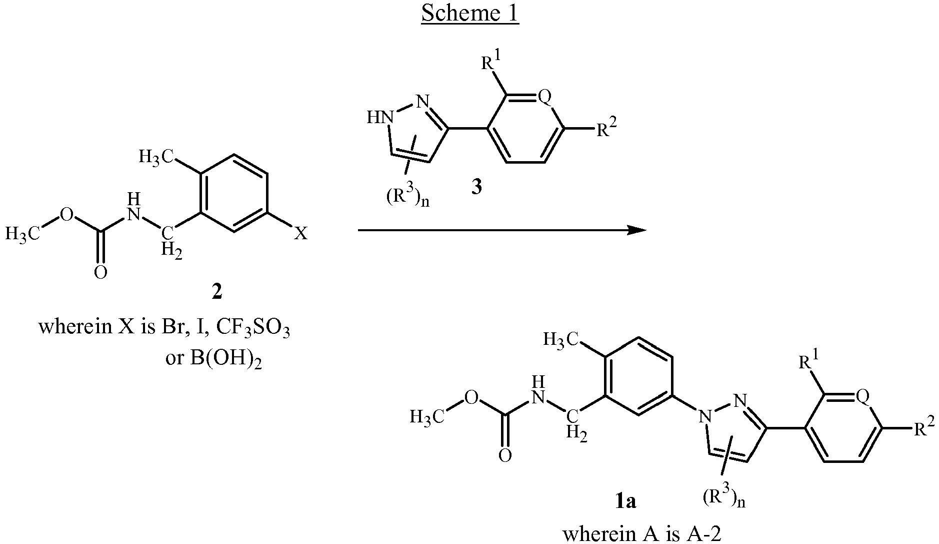

As shown in Scheme 2, compounds of Formula **2a** can be prepared from compounds of Formula **4** by first converting the benzylic alcohol into a suitable leaving group like a chlorine or bromine using a reagent such as thionyl chloride in an aprotic solvent such as dichloromethane or dichloro ethane at a temperature between 0° C and the boiling point of the solvent.

In the second step, treatment of the benzyl halide with potassium cyanate or sodium cyanate and methanol provides compounds of Formula **2.** The reaction is typically carried out in a solvent such as *N,N*-dimethylformamide at temperatures ranging from about room temperature to 120 °C according to the procedure described in U.S. patent 6,313,071. Compounds of Formula **2a** wherein X is Br or I can be further converted into compounds of Formula **2** wherein X is boronic acid (B(OH)₂) using methods known in the chemical literature.

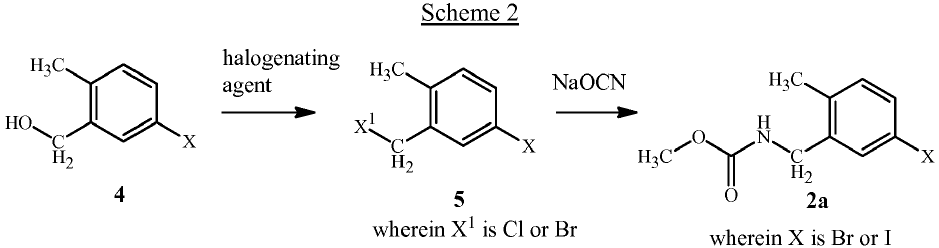

As shown in Scheme 3, compounds of Formula **4** wherein X is a bromine or iodine can be prepared from the corresponding commercially available compounds of Formula **6** by converting the carboxylic acid to the benzyl alcohol using many procedures known in the art. In one example, a suitable reducing agent such as borane/tetrahydrofuran complex, in an aprotic solvent such as diethyl ether or tetrahydrofuran, at a temperature between ambient and the boiling point of the solvent, results in the transformation of compounds of Formula **6** to compounds of Formula **4.**

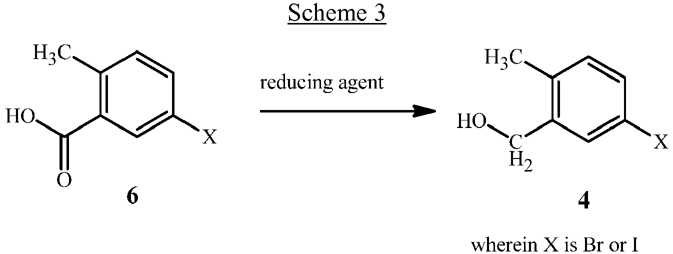

As shown in Scheme 4, compounds of Formula **3a** wherein n is 0, can be prepared using various methods known to one skilled in the art. In particular the methods described in EP 538156 will allow for the conversion of compounds of Formula **7** to be converted to compounds of Formula **3** proceeding through intermediates of Formula **8**.

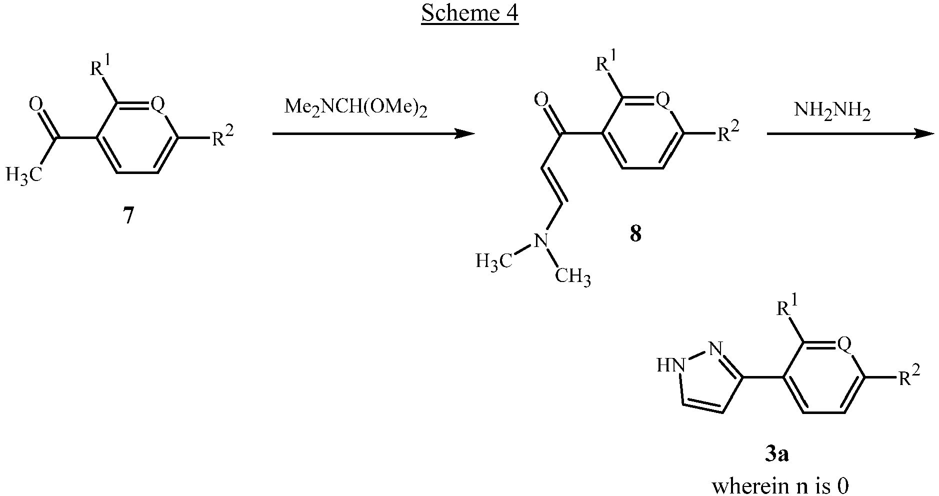

As shown in Scheme 5, compounds of Formula **1b** (i.e. compounds of Formula **1** wherein A is A-1) can be prepared by reaction of pyrazoles of Formula **9** with compounds of Formula **10** wherein X² is a bromine, iodine, trifluoromethanesulfonate or boronic acid. Conditions for this reaction are similar to that in Scheme 1 and can be found in PCT Patent Publication WO2008/124092.

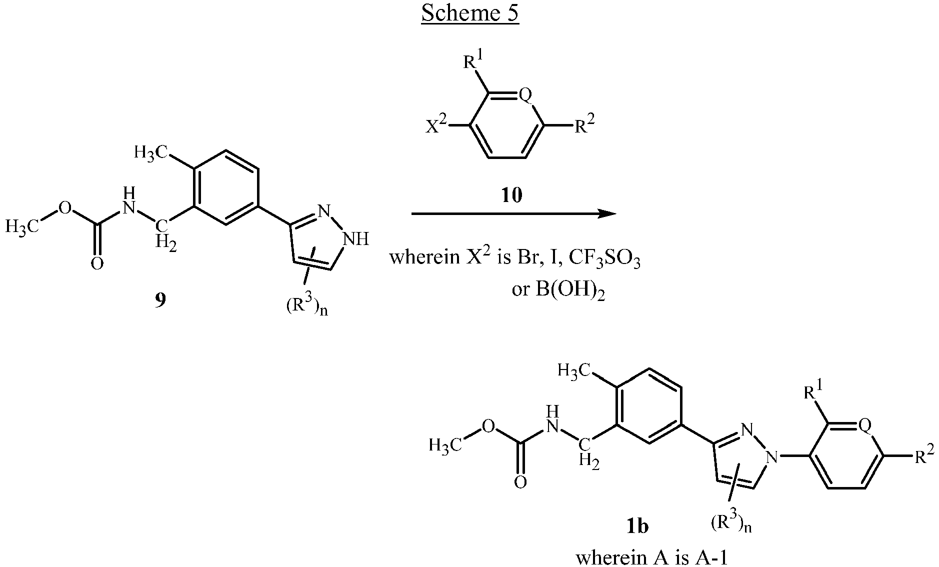

As shown in Scheme 6, compounds of Formula **9a** wherein n is 0 can be prepared by first reacting a compound of Formula **11** with *N,N*-dimethylformamide dimethyl acetal (DMF-DMA) at temperatures ranging from about 40 to about 100 °C in a solvent such as benzene or toluene, to provide an intermediate compound of Formula **12.** In a subsequent step, Formula **12** is reacted with hydrazine or a hydrazine salt in a lower alcohol solvent such as methanol or ethanol to provide a compound of Formula **9a.** One skilled in the art will recognize that there are other methods for performing transformations of this type, for example, the method described by Barrett et al., Bioorganic and Medicinal Chemistry Letters 2005, 15, 3540-3546.

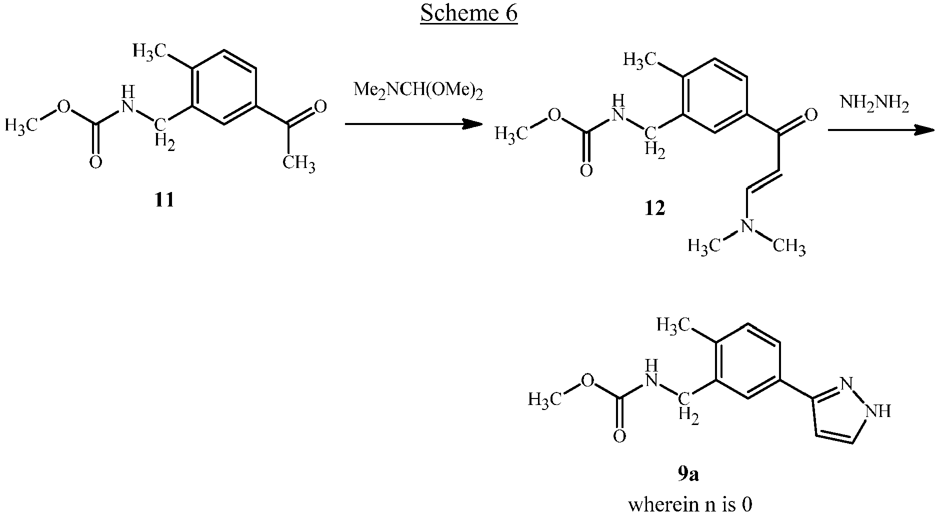

A compound of Formula **11** can be prepared by a simple four-step procedure from the commercially available amine of Formula **13** as outlined in Scheme 7. In the first step, an amine of Formula **13** is reacted with acetic anhydride with or without an aprotic solvent such as dichloromethane, chloroform, diethyl ether or tetrahydrofuran at temperatures ranging from about 0 to about 100 °C, in the presence of a base such as triethylamine or pyridine with or without a nucleophilic catalyst such as 4-dimethylaminopyridine to provide a compound of Formula **14.** The compound of Formula **14** can then be reacted according to Friedel-Crafts conditions to provide a compound of Formula **15** which can then be deprotected to yield a compound of Formula **16.** For typical reactions conditions see, EP 1586552. The compound of Formula **16** is then reacted with methyl carbonyl chloride in an aprotic solvent such as dichloromethane in the presence of a base such as triethyl amine at a temperature between ambient and the 40° C to yield a compound of Formula compound **11.**

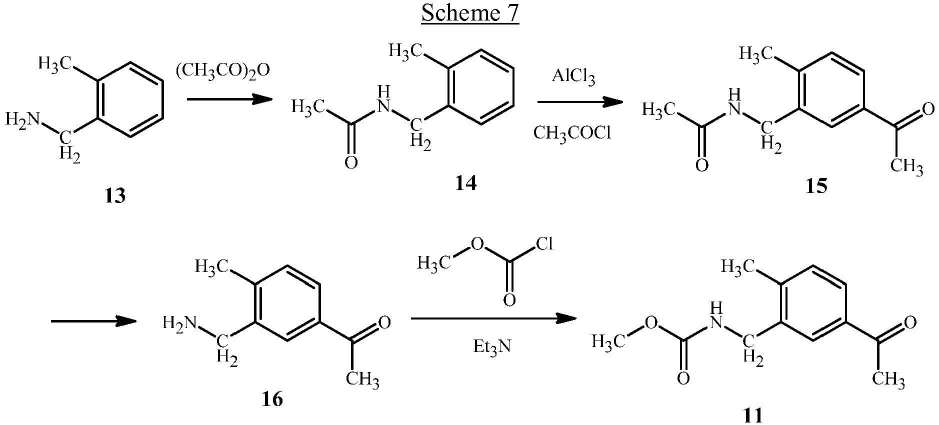

As shown in Scheme 8, compounds of Formula **1c** (i.e. a compound of Formula 1 wherein A is A-4) can be prepared from compounds of Formula **17** through reaction with methyl carbonyl chloride in an aprotic solvent such as dichloromethane in the presence of a base such as triethyl amine at a temperature between ambient and the 40° C.

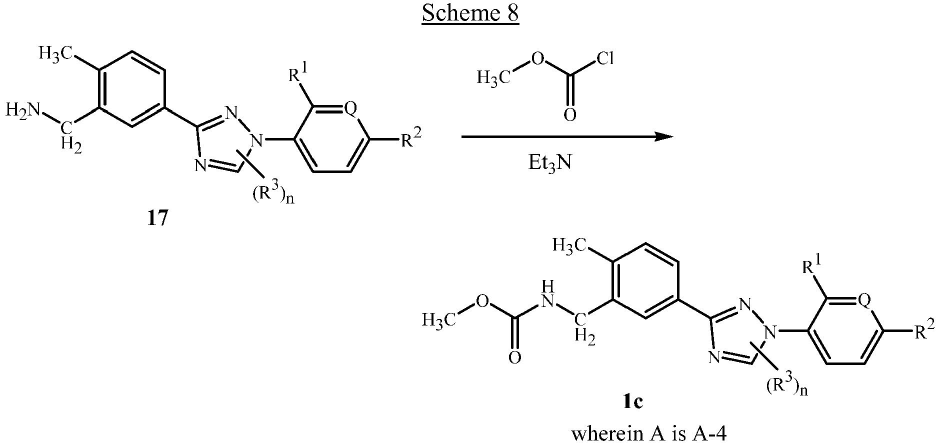

As shown in Scheme 9, compounds of Formula **17** can be prepared from nitriles of Formula **18** using an appropriate reducing agent such as borane or lithium aluminum hydride in an aprotic solvent such as tetrahydrofuran at a temperature between ambient and the boiling point of the solvent. For related examples, see the procedures and references contained within PCT patent applications WO 2011079102 and WO 2011073444.

Nitriles of Formula **18** can also be converted to amines of Formula **17** by catalytic hydrogenation. These reactions are traditionally carried out in the presence of a transition metal catalyst such as palladium (0) on carbon, Raney nickel, or platinum oxide in a lower alcohol solvent such as methanol or ethanol at a temperature between ambient and 100 °C under an atmosphere of hydrogen gas at a pressure between 1 and 75 bars. For related examples, see the procedures and references contained within PCT patent applications WO 2009152868 and WO 2010023161.

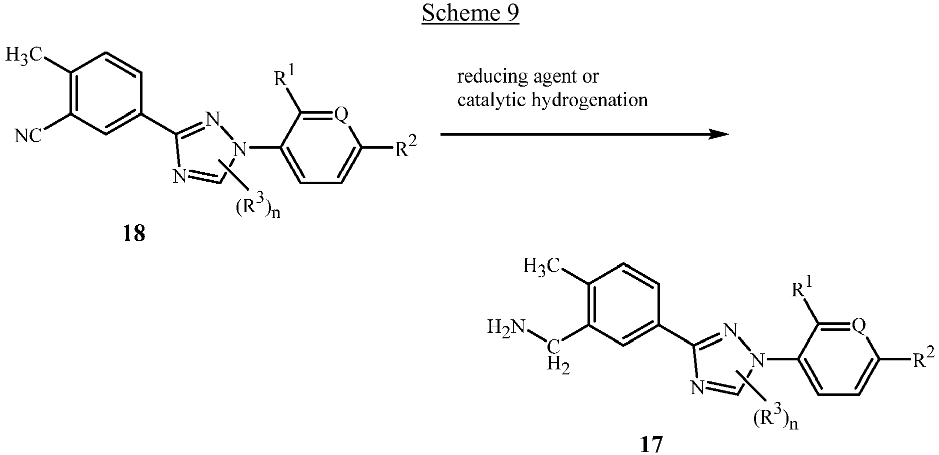

As shown in Scheme 10, compounds of Formula **18** can be prepared from compounds of Formula **19** via coupling with commercially available intermediates of Formula **10** (where X² is a bromine, iodine, triflate or boronic acid) using the methods described in Scheme 1

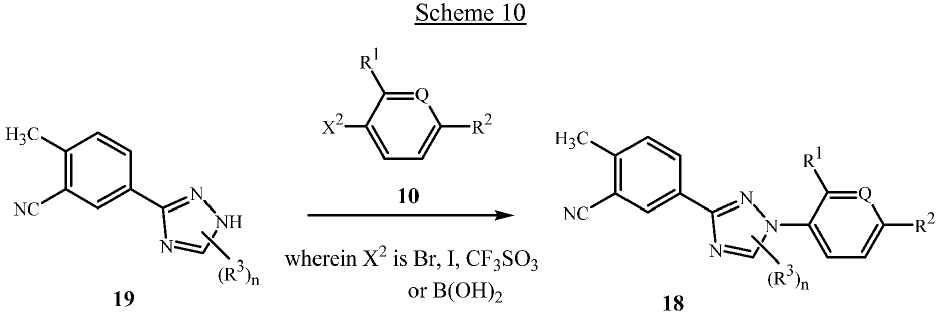

Compounds of Formula **19** can be prepared from compounds of Formula **20** as shown in Scheme 11. In a typical procedure, a compound of Formula **20** is contacted with a cyanide salt such as copper (I) cyanide or zinc (II) cyanide, in the presence of a suitable transition metal catalyst such as copper (I) iodide or Tetrakis(triphenylphosphine) palladium (0), in a polar aprotic solvent such as N,N'-dimethylformamide or dimethyl sulfoxide, at a temperature between 50° C and 150° C. For related procedures see PCT patent applications WO 2012032528 and WO 2011133882 and references contained within.

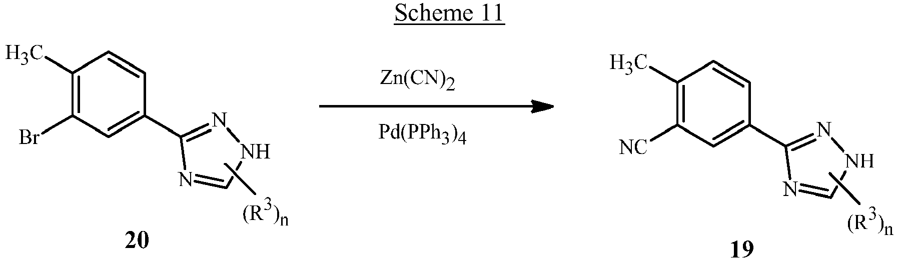

As shown in Scheme 12, compounds of Formula **20a** (wherein n is 0) can be prepared by first reacting commercially available compounds of Formula **21** with *N,N*-dimethylformamide dimethyl acetal (DMF-DMA) at temperatures ranging from about 40 to about 100 °C in a solvent such as toluene or benzene, to provide an intermediate compound of Formula **22.** In a subsequent step, the compound of Formula **22** is reacted with hydrazine or a hydrazine salt in a lower alcohol solvent such as methanol or ethanol to provide a compound of Formula **20a.**

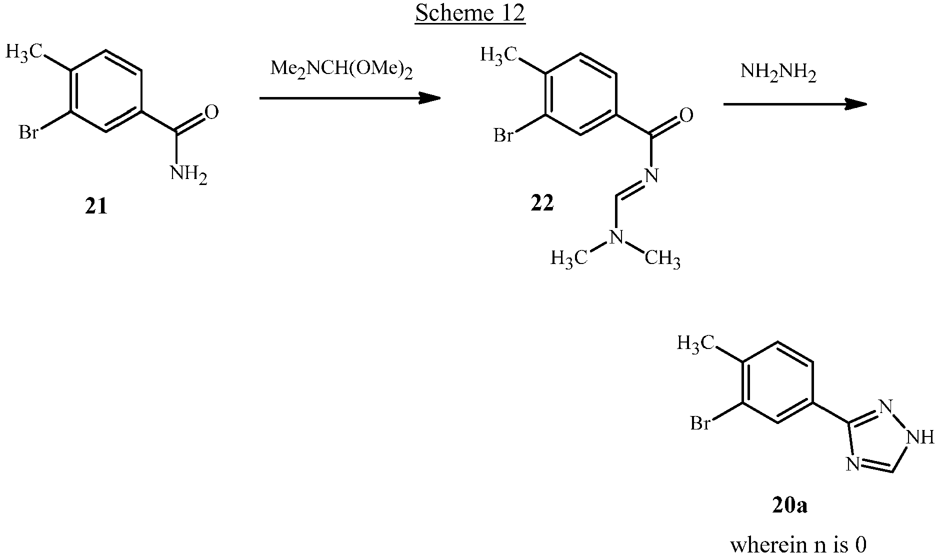

As shown in Scheme 13 compounds of Formula **1d** (i.e. compounds of Formula **1** wherein A is A-3) can be prepared by coupling compounds of Formula **2** with a 4-phenyl-1,2,3-tiazole of Formula **23** using procedures analogous to those described for Scheme 1 reactions.

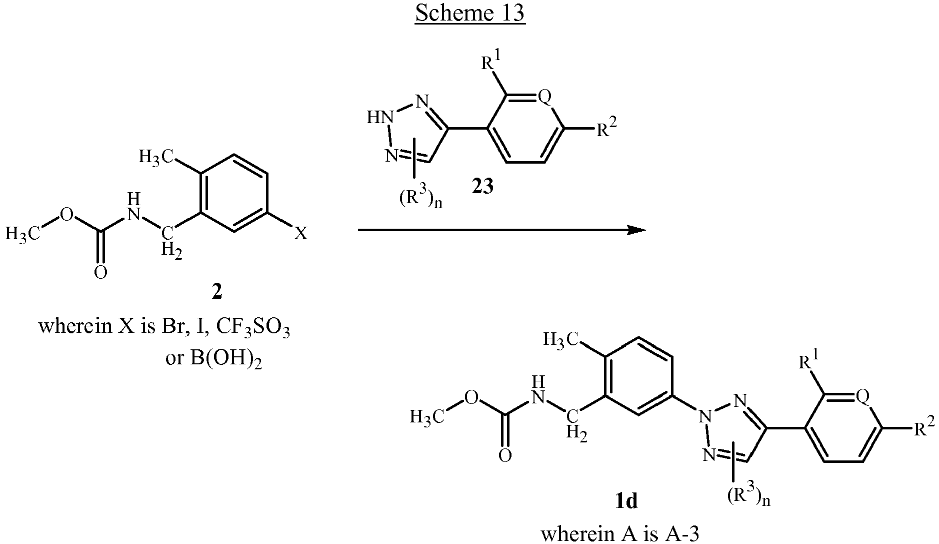

4-Phenyl-1,2,3 triazoles of Formula **23a** (wherein n is 0) can be prepared using the method illustrated by Scheme 14. A 2-nitrostyrene compound of Formula **24** reacts with a source of azide ion such as sodium azide or trimethylsilylazide in a polar solvent such as DMSO, DMF or EtOH at temperatures ranging from ambient up to about 100 °C. An example of the use of trimethylsilylazide/tetrabutylammonium fluoride combination can be found in J. Med. Chem. 2004, 47, 4645. Many examples exist for the preparation of 2-nitrostyrenes of Formula **24** via Henry reaction of benzaldehydes and nitromethane followed by dehydration: see, e.g., Tetrahedron, 1987, 43, 4803.

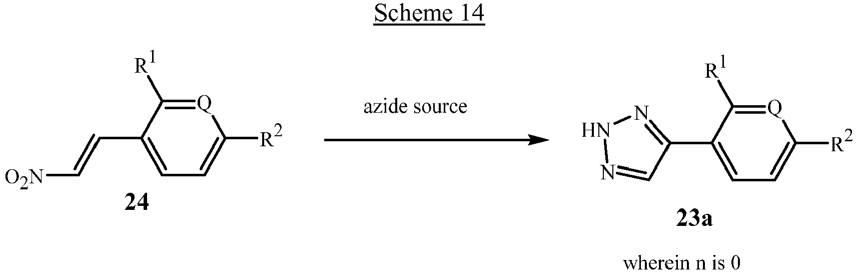

It is recognized that some reagents and reaction conditions described above for preparing compounds of Formula **1** may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. Protective Groups in Organic Synthesis, 2nd ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula **1.** One skilled in the art will also recognize that it may be necessary to perform a combination of the steps illustrated in the above schemes in an order other than that implied by the particular sequence presented to prepare the compounds of Formula **1.**

One skilled in the art will also recognize that compounds of Formula **1** and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative, and not limiting of the disclosure in any way whatsoever. Steps in the following Examples illustrate a procedure for each step in an overall synthetic transformation, and the starting material for each step may not have necessarily been prepared by a particular preparative run whose procedure is described in other Examples or Steps. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane; "s" means singlet, "d" means doublet, "t" means triplet, "q" means quartet, "m" means multiplet, "dd" means doublet of doublets, "dt" means doublet of triplets, "br s" means broad singlet. Mass spectra are reported as the molecular weight of the highest isotopic abundance parent ion (M+1) formed by addition of H⁺ (molecular weight of 1) to the molecule, or (M-1) formed by the loss of H⁺ (molecular weight of 1) from the molecule, observed by using liquid chromatography coupled to a mass spectrometer (LCMS) using either atmospheric pressure chemical ionization (AP⁺) or electrospray ionization (ESI⁺), where "amu" stands for atomic mass units.

### EXAMPLE 1

### Preparation of methyl N-[[5-[3-(2,4-dimethoxyphenyl)-1H-pyrazol-1-yl]-2-methylphenyl]methyl]carbamate (Compound 23)

### Step A: Preparation of 5-bromo-2-methylbenzenemethanol

To a mixture of 5-bromo-2-methyl-benzoic acid (6.0 g, 28 mmol) in 32 mL diethyl ether at 0 °C was added a 1M solution of borane-tetrahydrofuran complex (34 mL, 34 mmol) over 10 minutes. The reaction mixture was warmed to room temperature and then heated to reflux for 10 minutes. Methanol was added to react with the excess borane and then the mixture was extracted five times with saturated aqueous sodium bicarbonate solution. The organic phases was combined, dried over magnesium sulfate and concentrated under reduced pressure to provide the title compound (5.6 g).
¹H NMR (CDCl₃) δ 7.53 (d, 1H), 7.31 (m, 1H), 7.03 (d, 1H), 4.66 (s, 2H), 2.27 (s, 3H).

### Step B: Preparation of methyl N-[(5-bromo-2-methylphenyl)methyl]carbamate

To a solution of 5-bromo-2-methylbenzenemethanol (i.e. the product of Step A) (5.6 g, 28 mmol) in 60 mL dichloromethane was added thionyl chloride (3.8 g, 32 mmol). The mixture was stirred over night and then concentrated under reduced pressure, redissolved in 60 mL of dichloromethane and then reconcentrated to yield 4-bromo-2-(chloromethyl)-1-methylbenzene which was subsequently dissolved in 27 mL N,N'-dimethyl formamide and 6.0 mL methanol. To this solution was added potassium cyanate (3.2 g, 39 mmol). The resulting mixture was heated to 100 °C for 5 hours and then concentrated under reduced pressure. The crude residue was suspended in ethyl acetate and then washed two times with sodium bicarbonate. The organic phase was separated, dried over magnesium sulfate and concentrated under reduced pressure to provide the title compound (4.67 g) as a solid which was used without further purification.
¹H NMR (CDCl₃) δ 7.37 (d, 1H), 7.30 (m, 1H), 7.03 (d, 1H), 4.88 (bs, 1H), 4.33 (d, 2H), 3.71 (s, 3H), 2.27 (s, 3H).

### Step C: Preparation of methyl N-[[5-[3-(2,4-dimethoxyphenyl)-1H-pyrazol-1-yl]-2-methylphenyl]methyl]carbamate

Commercially available 3-(2,4-dimethoxyphenyl)-1*H*-pyrazole (0.13 g, 0.62 mmol), methyl N-[(5-bromo-2-methylphenyl)methyl]carbamate (i.e. the product of Step B) (0.20 g, 0.77 mmol), copper(I) iodide (0.03 g, 0.16 mmol) and potassium carbonate (0.21 g, 1.6 mmol) were suspended in 3 mL of a 50/50 mixture of toluene and dioxane. Nitrogen gas was bubbled through the reaction mixture for 5 minutes, and then *N,N'*-dimethyl-1,2-cyclohexanediamine (0.04 g, 0.31 mmol) was added. Nitrogen gas was bubbled through the reaction mixture for another 10 minutes, and then the reaction was heated to 110 °C for approximately 16 hours. The reaction mixture was concentrated under reduced pressure and then purified by medium pressure liquid chromatography on silica gel eluting with 0 to 100% ethyl acetate in hexanes to yield the title compound, a compound of the current invention, as a solid (0.17 g).
¹H NMR (CDCl₃) δ 7.99 (d, 1H), 7.88 (d, 1H), 7.66 (d, 1H), 7.53 (m, 1H), 7.22 (d, 1H), 6.91 (d, 1H), 6.59 (m, 1H), 6.55 (d, 1H), 4.97 (bs, 1H), 4.42 (d, 2H), 3.90 (s, 3H), 3.85 (s, 3H), 3.71 (s, 3H), 2.34 (s, 3H).

### EXAMPLE 2

### Preparation of methyl N-[[5-[1-(4-methoxy-2-methylphenyl)-1H-pyrazol-3-yl]-2-methylphenyl]methyl]carbamate (Compound 18)

### Step A: Preparation of N-[(2-methylphenyl)methyl]acetamide

To a solution of 2-methyl benzyl amine (25 g, 120 mmol) in 150 mL of toluene at 0 °C was added a solution of acetic anhydride (29 g, 310 mmol) in 70 mL of toluene over approximately 15 minutes. The reaction was heated to 100 °C for 1 hour and then cooled to room temperature. Water was added and the reaction mixture was extracted with ethyl acetate. The combined organic phases were washed with IN hydrochloric acid and saturated aqueous sodium chloride solution, then dried over magnesium sulfate and concentrated under reduced pressure to yield a solid. The solid was washed with a solution of 150 mL of 1-chlorobutane and 250 mL of hexanes provide the title compound (26 g) as a solid.
¹H NMR (CDCl₃) δ 7.20 (m, 4H), 5.56 (bs, 1H), 4.43 (d, 2H), 2.33 (s, 3H), 2.01 (s, 3H).

### Step B: Preparation of N-[(5 -acetyl-2-methylphenyl)methyl] acetamide

To a solution of *N*-[(2-methylphenyl)methyl]acetamide (i.e. the product of Step A) (26 g, 160 mmol) in 110 mL dichloromethane at 0 °C was added aluminum trichloride (64 g, 480 mmol) portion-wise in order to minimize the exothermic reaction. Acetyl chloride (25 g, 320 mmol) was added over 20 minutes, and then the reaction mixture was heated to reflux over night. The reaction was cooled to room temperature, and then poured into ice water. The phases were separated and the aqueous phase was extracted with dichlormethane. The combined organic phases were dried over magnesium sulfate and concentrated under reduced pressure. The crude residue was then purified by medium pressure liquid chromatography on silica gel eluting with 50 to 100% ethyl acetate in hexanes to provide the title compound (18 g) as a solid.
¹H NMR (CDCl₃) δ 7.81 (d, 1H), 7.76 (m, 1H), 7.27 (m, 1H), 5.84 (bs, 1H), 4.47 (d, 2H), 2.56 (s 3H), 2.38 (s, 3H), 2.04 (s 3H).

### Step C: Preparation of methyl N-[(5-acetyl-2-methylphenyl)methyl]carbamate

*N*-[(5-acetyl-2-methylphenyl)methyl]acetamide (i.e. the product of Step B) (10 g, 49 mmol) was added to a solution of concentrated sulfuric acid (17 g, 170 mmol) in 20 mL water. The reaction mixture was heated to reflux for 6 hours and then filtered through celite while still warm. The filtrate was extracted with diethyl ether. The aqueous phase was then made basic with 50 mL of 25% aqueous sodium hydroxide solution and extracted two times with ethyl acetate. The ethyl acetate phases were combined, dried over magnesium sulfate and concentrated under reduced pressure to yield 6.3 g of 1-[3-(aminomethyl)-4-methylphenyl]ethanone which was immediately dissolved in 75 mL of tetrahydrofuran and cooled to 0 °C. To this solution was added diisopropylethylamine (5.5 g, 43 mmol) and then a solution of methylchloroformate (3.8 g, 39 mmol) in 20 mL of tetrahydrofuran. The reaction mixture was allowed to warm to room temperature and stir over night. The reaction mixture was concentrated under reduced pressure, dissolved in ethyl acetate and washed with water, saturated aqueous ammonium chloride solution, saturated aqueous sodium chloride solution and then dried over magnesium sulfate and concentrated under reduced pressure to provide the title compound (7.6 g) as a solid.
¹H NMR (CDCl₃) δ 7.84 (d, 1H), 7.77 (m, 1H), 7.26 (m, 1H), 4.97 (bs, 1H), 4.41 (d, 2H), 3.71 (s, 3H), 2.58 (d, 3H), 2.38 (s, 3H).

### Step D: Preparation of methyl N-[[2-methyl-5-(1H-pyrazol-3-yl)phenyl]-methyl]carbamate

methyl *N*-[(5-acetyl-2-methylphenyl)methyl]carbamate (i.e. the product of Step C) (23 g, 107 mmol) and *N,N'*-dimethylformamide dimethyl acetal (38 g, 320 mmol) were refluxed in 250 mL toluene for approximately 16 hours. The reaction mixture was concentrated under reduced pressure and the resultant solids were triturated with 1-chlorobutane and acetone to yield 6 .0 g of solid intermediate methyl *N*-[[5-[3-(dimethylamino)-1-oxo-2-propen-1-yl]-2-methylphenyl]methyl]carbamate and a filtrate which also contained desired intermediate. The solid intermediate was then dissolved in 120 mL methanol and treated with hydrazine hydrate (1.7 g, 34 mmol) and stirred for approximately 16 hours. The reaction mixture was concentrated and the resultant solids triturated with acetone to provide the title compound (6.0 g) as a solid.
¹H NMR (CDCl₃) δ 7.65 (s, 1H), 7.61 (d, 1H), 7.55 (d, 1H), 7.21 (d, 1H), 6.58 (d, 1H), 5.05 (bs, 1H), 4.40 (d, 2H), 3.70 (s, 3H), 2.34 (s, 3H).

### Step E: Preparation of methyl N-[[5-[1-(4-methoxy-2-methylphenyl)-1H-pyrazol-3-yl]-2-methylphenyl]methyl]carbamate

Methyl *N*-[[2-methyl-5-(1*H*-pyrazol-3-yl)phenyl]-methyl]carbamate (i.e. the product of Step D) (1.0 g, 4.1 mmol), 1-bromo-4-methoxy-2-methyl- benzene (1.0 g, 5.1 mmol), copper(I) iodide (0.40 g, 2.0 mmol) and potassium carbonate (0.1.4 g, 10 mmol) were suspended in 50 mL of a 50/50 mixture of toluene and dioxane. Nitrogen gas was bubbled through the reaction mixture for 5 minutes, and then *N,N'*-dimethyl-1,2-cyclohexanediamine (0.58 g, 4.1 mmol) was added. Nitrogen gas was bubbled through the reaction mixture for another 10 minutes, and then the reaction mixture was heated to 110 °C for approximately 40 hours. The reaction mixture was concentrated under reduced pressure and then purified by medium pressure liquid chromatography on silica gel eluting with 0 to 100% ethyl acetate in hexanes to provide the title compound, a compound of the current invention, (0.30 g) as a solid.
¹H NMR (CDCl₃) δ 7.76 (s, 1H), 7.69 (d, 1H), 7.54 (d, 1H), 7.30 (d, 1H), 7.22 (d, 1H), 6.82 (m, 2H), 6.69 (m, 1H), 4.83 (bs, 1H), 4.42 (d, 2H), 3.87 (s, 3H), 3.70 (s, 3H), 2.37 (s, 3H), 2.25 (s, 3H).

### EXAMPLE 3

### Preparation of methyl N-[[5-[4-(2,4-dimethoxyphenyl)-2H-1,2,3-triazol-2-yl]-2-methylphenyl]methyl]carbamate (Compound 17)

### Step A: Preparation of 5-iodo-2-methylbenzenemethanol

Borane-methylsulfide solution (50.0 mL of a 2.0M solution in THF, 100mmol) was added dropwise to a solution of 5-iodo-2-methyl benzoic acid (13.1g, 50 mmol) and anhydrous THF (100 mL) at 35-45 °C at a rate to moderate the gas evolution. The resulting mixture was heated at 60 °C for 3 h and was then cooled to 25 °C and quenched by the careful, dropwise addition of methanol (50 mL) which was added at such a rate that gas evolution was moderated and the temperature was maintained below 35 °C. The resulting mixture was stirred at 25 °C for 2 h and was then concentrated under vacuum. The residue was partitioned between ethyl acetate and saturated aqueous sodium bicarbonate solution, the organic phase was washed with saturated aqueous sodium bicarbonate solution, dried over anhydrous magnesium sulfate and concentrated to provide the title compound (11.58 g) as a white solid
¹H NMR (CDCl₃) δ 7.71 (d, 1H), 7.52 (d, 1H), 6.91 (d, 1H), 4.64 (s, 2H), 2.27 (s, 3H), 1.62 (br s, 1H).

### Step B: Preparation of 2-(bromomethyl)-4-iodo-1-methylbenzene

Phosphorus tribromide (2.16 mL, 22.9 mmol) was added to a solution of 5-iodo-2-methylbenzenemethanol (i.e. the product of Step A) (11.38 g, 45.9 mmol) and anhydrous diethyl ether (73 mL) at 0-5 °C. The resulting mixture was stirred at 0 °C for 1.5 h and was then quenched by the addition of water (25 mL) added at 0-5 °C. The resulting mixture was stirred at 0 °C for 15 min and was then partitioned between ethyl acetate and water. The organic phase was washed with water, saturated aqueous sodium bicarbonate solution, dried over anhydrous magnesium sulfate and concentrated under vacuum to provide the title compound (12.1 g) as a white solid.
¹H NMR (CDCl₃) δ 7.63 (d, 1H), 7.51 (d, 1H), 6.93 (d, 1H), 4.41 (s, 2H), 2.35 (s, 3H).

### Step C: Preparation of methyl N-[(5-iodo-2-methylphenyl)methyl]carbamate

A solution 2-(bromomethyl)-4-iodo-1-methylbenzene (i.e. the product of Step B) (8.50 g, 27.3 mmol), methanol (8 mL) and DMF (82 mL) was treated with potassium cyanate (5.54 g, 68.3 mmol) added in 4 equal portions over 15 min at 35-45 °C. The resulting mixture was heated at 55 °C for 2 h, cooled to 25 °C and partitioned between ethyl ether and water. The organic phase was washed with water, dried over anhydrous magnesium sulfate, and concentrated to provide the title compound (8.43 g) as a white solid.
¹H NMR (CDCl₃) δ 7.56 (d, 1H), 7.50 (d, 1H), 6.90 (d, 1H), 4.92 (br s, 1H), 4.31 (br d, 1H), 3.71 (s, 3H), 2.26 (s, 3H).

### Step D: Preparation of 4-(2,4-dimethoxyphenyl)-2H-1,2,3-triazole

A solution of 2,4-dimethoxy-1-(2-nitroethenyl)benzene (3.35 g, 16.0 mmol), azidotrimethylsilane (2.76 g, 24.0 mmol) and anhydrous DMF (100 mL) was treated with tetrabutylammonium fluoride (17.6 mL of a 1.0 M solution in THF, 17.6 mmol) added dropwise over 20 min at 50 °C. The resulting solution was heated at 50 °C for an additional 1 h, cooled to 25 °C and treated with methanol (25 mL). The resulting solution was concentrated under vacuum to about 25 mL volume and the residue was partioned between ethyl acetate and water. The organic phase was washed with water, dried over anhydrous magnesium sulfate, and concentrated to provide the title compound (2.28 g) as a beige solid.
¹H NMR (CDCl₃) δ 8.03 (s, 1H), 7.76 (br s, 1H), 6.63 (d, 1H), 6.58 (s, 1H), 3.98 (s, 3H), 3.87 (s, 3H).

### Step E: Preparation of methyl N-[[5-[4-(2,4-dimethoxyphenyl)-2H-1,2,3-triazol-2-yl]-2-methylphenyl]methyl] carbamate

A solution of methyl *N*-[(5-iodo-2-methylphenyl)methyl]carbamate (i.e. the product of Step C) (203 mg, 1.0 mmol) and 4-(2,4-dimethoxyphenyl)-2*H*-1,2,3-triazole (i.e. the product of Step D) (204 mg, 1.0 mmol), *N N'*-dimethylethylene diamine (0.16 mL, 1.5 mmol), and dioxane (15 mL) was de-gassed with bubbling nitrogen for 10 min, treated with Cs₂CO₃ (648 mg, 2.0 mmol) and CuI (19 mg, 0.1 mmol) and further de-gassed with nitrogen for 10 min. The resulting mixture was heated at reflux under nitrogen for 72 h. The resulting mixture was cooled to ambient temperature, diluted with ethyl acetate (20 mL) and concentrated onto Celite® diatomaceous filter aid (2 g). Purification by chromatography on a 12 g silica column eluting with a solvent gradient of 0% to 100% ethyl acetate in hexanes gave a yellow solid which was further triturated with diethyl ether to provide the title compound, a compound of the current invention, (75 mg) as a light yellow solid.
¹H NMR (CDCl₃) δ 8.17 (s, 1H), 8.04 (d, 1H), 8.00 (d, 1H), 7.92 (dd, 1H), 7.25 (d, 1H), 6.61 (dd, 1H), 6.55 (d, 1H), 5.03 (br s, 1H), 4.44 (br d, 2H), 3.93 (s, 3H), 3.86 (s, 3H), 3.72 (br s, 3H), 2.36 (br s, 3H).

### EXAMPLE 4

### Preparation of methyl N-[[5-[3-(2,6-dimethyl-3-pyridinyl)-1H-pyrazol-1-yl]-2-methylphenyl]methyl]carbamate (Compound 130)

### Step A: Preparation of methyl N-[[5-(3-bromo-1H-pyrazol-1-yl)-2-methylphenyl]methyl]carbamate

Methyl *N*-[(2-methyl-5-iodophenyl)methyl]carbamate (i.e. the product of Example 3, Step C) (5.00 g, 16.4 mmol), 3-bromopyrazole (3.11 g, 21.3 mmol), potassium carbonate (5.65 g, 41.0 mmol), and copper (I) iodide (623 mg, 3.28 mmol) were combined in toluene (16 mL) and *N,N'*-dimethylformamide (16 mL). A stream of nitrogen gas was bubbled into the mixture for 30 min., *N,N'*-dimethyl-1,2-cyclohexanediamine (1.0 mL, 6.6 mmol) was added and a stream of nitrogen gas was bubbled through the mixture for an additional 30 min. The nitrogen line was then raised above the reaction mixture and the mixture was stirred at room temperature for 16 hours. The reaction mixture was filtered through a fritted-glass funnel and then concentrated under vacuum. The resultant residue was purified by medium pressure liquid chromatography using a gradient of 10 to 50% ethyl acetate in hexanes to provide the title compound (4.35 g) as an off-white solid.
¹H NMR (500 MHz, CDCl₃) δ 7.76 (d, *J*=2.5 Hz, 1 H), 7.55 (d, *J*=2.0 Hz, 1 H), 7.42 (dd, *J*=8.1, 2.0 Hz, 1 H), 7.23 (d, *J*=8.2 Hz, 1 H), 6.45 (d, *J*=2.6 Hz, 1 H), 5.00 (bs, 1 H), 4.40 (d, *J*=5.7 Hz, 2 H), 3.71 (s, 3 H), 2.34 (s, 3 H).

### Step B: Preparation of methyl N-[[5-[3-(2,6-dimethyl-3-pyridinyl)-1H-pyrazol-1-yl]-2-methylphenyl]methyl] carbamate

Methyl *N*-[[5-(3-bromo-1*H*-pyrazol-1-yl)-2-methylphenyl]methyl]carbamate (i.e. the product of Step A) (150 mg, 0.464 mmol), 2,6-dimethylpyridine-3-boronic acid (250 mg, 1.67 mmol), PdCl₂(PPh₃)₂ (33 mg, 0.046 mmol), and potassium carbonate (320 mg, 2.32 mmol) were taken up in acetonitrile (4 mL) and water (1 mL) in a microwave reactor vial. The reaction mixture was then heated at 120 °C in a microwave reactor for 30 min. After the reaction had cooled to room temperature, the mixture was diluted with dichloromethane, filtered through a ChemElut cartridge (diatomaceous earth sorbent material), and concentrated under vacuum. The residue was purified by medium pressure liquid chromatography using a gradient of 20 to 100% ethyl acetate in hexanes. The resulting material was further purified by trituration with diethyl ether to yield the title compound, a compound of the present invention, as a white solid (125 mg).
¹H NMR (500 MHz, CDCl₃) δ 7.95 (d, *J*=2.5 Hz, 1 H), 7.85 (d, *J*=7.9 Hz, 1 H), 7.66 (d, *J*=2.0 Hz, 1 H), 7.57 - 7.50 (m, 1 H), 7.28 - 7.24 (m, 1 H), 7.10 - 7.04 (m, 1 H), 6.63 (d, *J*=2.4 Hz, 1 H), 4.94 (bs, 1 H), 4.44 (d, *J*=5.8 Hz, 2 H), 3.72 (s, 3 H), 2.77 (s, 3 H), 2.57 (s, 3 H), 2.37 (s, 3 H).

### EXAMPLE 5

### Preparation of methyl N-[[5-[1-(2,6-dimethyl-3-pyridinyl)-1H-pyrazol-3-yl]-2-methylphenyl]methyl]carbamate (Compound 114)

### Step A: Preparation of 5-acetyl-2-methylbenzonitrile

3-Bromo-4-methylacetophenone (2.45 g, 11.5 mmol) was dissolved in *N,N'-*dimethylformamide (25 mL) and copper (I) cyanide (2.06 g, 23 mmol) was added. A stream of nitrogen was bubbled into the reaction mixture for 20 min. The nitrogen line was removed from the solution, and the reaction was heated under positive nitrogen pressure at reflux for 7 hours. The reaction mixture was cooled to room temperature, diluted with water, and filtered through a fritted-glass funnel. The resulting solution was extracted with ethyl acetate (3x). The combined organic phases were washed with saturated aqueous sodium chloride (5x), dried over magnesium sulfate, and concentrated under vacuum to yield a light brown solid (1.30 g), which was sufficiently pure to be used directly in the next reaction.
¹H NMR (500 MHz, CDCl₃) δ 8.18 (d, *J*=1.9 Hz, 1 H), 8.06 (dd, *J*=8.1, 1.8 Hz, 1 H), 7.44 (d, *J*=8.0 Hz, 1 H), 2.62 (s, 3 H), 2.61 (s, 3 H).

### Step B: Preparation of 2-methyl-5-(1H-pyrazol-3-yl)benzonitrile

5-Acetyl-2-methylbenzonitrile (i.e. the product of Step A) (3.0 g, 18.8 mmol) was dissolved in *N,N'*-dimethylformamide dimethyl acetal (10 mL) and heated to 110 °C for 16 hours. The reaction mixture was allowed to cool to room temperature and was then concentrated under vacuum. The resulting residue containing intermediate 5-[(2E)-3-(dimethylamino)-1-oxo-2-propen-1-yl]-2-methylbenzonitrile was taken up in ethanol (40 mL) and hydrazine hydrate (3.4 mL, 56 mmol) was added. The reaction mixture was heated to reflux for 1 hour. The mixture was allowed to cool to room temperature and was then concentrated under vacuum. The residue was taken up in ethyl acetate, washed with water and saturated aqueous sodium chloride (4x), dried over magnesium sulfate, and concentrated under vacuum to yield a pale yellow solid (3.20 g), which was sufficiently pure to be used directly in the next reaction.
¹H NMR (500 MHz, CDCl₃) δ 13.01 (bs, 1 H), 8.16 (d, *J*=1.6 Hz, 1 H), 8.05 (d, *J*=7.7 Hz, 1 H), 7.81 (s, 1 H), 7.49 (d, *J*=8.0 Hz, 1 H), 6.83 (d, *J*=1.9 Hz, 1 H), 2.50 (s, 3 H).

### Step C: Preparation of 5-[1-(2,6-dimethyl-3-pyridinyl)-1H-pyrazol-3-yl]-2-methylbenzonitrile

2-Methyl-5-(1*H*-pyrazol-3-yl)benzonitrile (i.e. he product of Step B) (0.20 g, 1.1 mmol), copper (I) iodide (42 mg, 0.22 mmol), potassium carbonate (376 mg, 2.72 mmol), and 3-bromo-2,6-dimethylpyridine (0.18 mL, 1.4 mmol) were taken up in *N,N'-*dimethylformamide (2 mL). A stream of nitrogen gas was bubbled into the mixture for 10 min., *N,N'*-dimethyl-1,2-cyclohexanediamine (0.07 mL, 0.4 mmol) was added and a stream of nitrogen gas was bubbled through the mixture for an additional 10 min. The nitrogen line was then raised above the reaction mixture, and the mixture was heated at 100 °C for 16 hours. The reaction mixture allowed to cool to room temperature, diluted with water and ethyl acetate, and filtered through a fritted-glass funnel. The phases were separated and the aqueous phase was extracted with ethyl acetate (3x). The combined organic phases were washed with saturated aqueous sodium chloride, dried over magnesium sulfate, and concentrated under vacuum. The residue was purified by medium pressure liquid chromatography using a gradient of 20-100% ethyl acetate in hexanes to yield an off-white solid (0.25 g).
¹H NMR (500 MHz, CDCl₃) δ 8.10 (d, *J*=1.9 Hz, 1 H), 7.98 (dd, *J*=8.0, 1.9 Hz, 1 H), 7.65 (d, *J*=2.4 Hz, 1 H), 7.61 (d, *J*=8.0 Hz, 1 H), 7.37 (d, *J*=8.0 Hz, 1 H), 7.13 (d, *J*=8.0 Hz, 1 H), 6.76 (d, *J*=2.4 Hz, 1 H), 2.61 (s, 3 H), 2.58 (s, 3 H), 2.51 (s, 3 H).

### Step D: Preparation of methyl N-[[5-[1-(2,6-dimethyl-3-pyridinyl)-1H-pyrazol-3-yl]-2-methylphenyl]methyl] carbamate

5-[1-(2,6-Dimethyl-3-pyridinyl)-1*H*-pyrazol-3-yl]-2-methylbenzonitrile (i.e. the product of Step C) (0.25 g, 0.87 mmol), was taken up in ethanol (20 mL) and warmed to 60 °C. Nickel (II) chloride (110 mg, 0.87 mmol) and then sodium borohydride (230 mg, 6.1 mmol) were added. The reaction mixture was stirred at 60 °C for 45 min. The reaction mixture was cooled to room temperature, diluted with ethyl acetate, and was filtered through Celite® filtering agent. The filtrate was washed with saturated aqueous sodium chloride (4x), dried over magnesium sulfate, and concentrated under vacuum. The resulting residue was taken up in ethyl acetate (5 mL) and water (1 mL) and potassium carbonate (168 mg, 1.22 mmol) was added. The mixture was cooled in an ice water bath and methyl chloroformate (0.08 mL, 1 mmol) was added. The reaction mixture was allowed to stir while warming to room temperature over 16 hours. The mixture was then diluted with ethyl acetate and water and the phases separated. The combined organic phases were washed with water (3x), dried over magnesium sulfate, and concentrated under vacuum. The resulting residue was purified by medium pressure liquid chromatography using a gradient of 40-100% ethyl acetate in hexanes to yield the title compound, a compound of the present invention, as a beige solid (100 mg).
¹H NMR (500 MHz, CDCl₃) δ 7.76 (d, *J*=1.7 Hz, 1 H), 7.69 (dd, *J*=7.7, 1.7 Hz, 1 H), 7.64 - 7.59 (m, 2 H), 7.23 (d, *J*=7.7 Hz, 1 H), 7.12 (d, *J*=7.9 Hz, 1 H), 6.75 (d, *J*=2.4 Hz, 1 H), 4.91 - 4.80 (bs, 1 H), 4.43 (d, *J*=5.4 Hz, 2 H), 3.70 (s, 3 H), 2.60 (s, 3 H), 2.51 (s, 3 H), 2.37 (s, 3 H).

### EXAMPLE 6

### Preparation of methyl N-[[5-[1-(4-methoxy-2-methylphenyl)-1H-1,2,4-triazol-3-yl]-2-methylphenyl]methyl]carbamate (Compound 70)

### Step A: Preparation of methyl N-[(5-cyano-2-methylphenyl)methyl]carbamate

To a solution of methyl *N*-[(2-methyl-5-iodophenyl)methyl]carbamate (i.e. the product of Example 3, Step C) (5.0 g, 16 mmol) in 50 mL *N,N'*-dimethylformamide was added copper (I) cyanide (2.9 g, 33 mmol). The reaction mixture was heated at 125 °C for 16 hours, then cooled to room temperature and passed through Celite® filtration agent. The filtrate was diluted with ethyl acetate and washed with water, saturated aqueous sodium chloride and dried over sodium sulfate. The solvent was concentrated under reduced pressure to give the title compound (2.0 g) as a solid.
¹H NMR (500 MHz, DMSO-D₆) δ 2.40 (s, 3H), 3.62(s, 3H), 4.20 (d, 2H), 7.38 (dd, 1H), 7.54 (s, 1H), 7.62 (d, 1H), 7.68 (bs,1H).

### Step B: Preparation of methyl N-[[5-(aminocarbonyl)-2-methylphenyl)methyl]-carbamate

To a solution of methyl *N*-[(5-cyano-2-methylphenyl)methyl]carbamate (i.e. the product of Step A) (5.0 g, 25 mmol) in 40 mL EtOH was added 30% aqueous hydrogen peroxide (0.7 mL) drop-wise at 0 °C. Then 6N sodium hydroxide (0.3 mL) solution was added, and the reaction mixture was stirred at 25 °C for 16 hours. The solvent was concentrated under reduced pressure, water was added, and the remaining solid was filtered off to give the title compound (4.3 g).
¹H NMR (500 MHz, DMSO-D₆) δ 2.6 (s, 3H), 3.52 (s, 3H), 4.20 (d, 2H), 7.20 (d, 1H), 7.30 bs, 1H), 7.60 (b, 1H), 7.70 (dd, 1H), 7.80 (s, 1H), 7.90 (bs,1H).

### Step C: Preparation of methyl N-[[5-(1H-1,2,4-triazol-3-yl)-2-methylphenyl]-methyl]carbamate

A mixture of methyl *N*-[[5-(aminocarbonyl)-2-methylphenyl)methyl]-carbamate (i.e. the product of Step B) (1.2 g, 5.4 mmol), *N,N'*-dimethylformamide dimethyl acetal (1.9 g, 16 mmol) and 20 mL toluene were heated to 100 °C for 16 hours. The reaction mixture was then concentrated, water was added, and the mixture was extracted three times with ethyl acetate. The combined organic phases were washed with water and saturated aqueous sodium chloride, dried over sodium sulfate, filtered and concentrated under reduced pressure to give an intermediate (1.0 g, 3.6 mmol). The intermediate was taken up in ethanol (20 mL) and treated with hydrazine hydrate (0.23 mL 7.2 mmol). The reaction mixture was stirred at room temperature for 3 hours and then concentrated under reduced pressure. The resulting crude residue was purified with neutral alumina column chromatography using 1% methanol in chloroform as eluent to give the title compound (0.45 g) as a solid.
¹H NMR (500 MHz, DMSO-D₆) δ 2.20 (s, 3H), 3.60 (s, 3H), 4.20 (d, 2H), 7.20 (d, 2H), 7.80 (d, 1H), 7.90 (s, 1H), 8.20 (s, 1H), 12.52 (bs, 1H).

### Step D: Preparation of methyl N-[[5-[1-(4-methoxy-2-methylphenyl)-1H-1,2,4-triazol-3-yl]-2-methylphenyl]methyl]carbamate

To a solution of methyl *N*-[[5-(1*H*-1,2,4-triazol-3-yl)-2-methylphenyl]-methyl]carbamate (i.e. the product of Step C) (0.20 g, 0.81 mmol) in dioxane (16 mL) was added trans-*N,N'*-dimethyl-1,2-cyclohexanediamine (23 mg, 0.16 mmol) *N,N'*-dimethyl-1,2-ethanediamine, (70 mg, 0.81 mmol), copper(I) iodide (30 mg, 0.16 mmol) and potassium carbonate (340 mg, 2.4 mmol) under a nitrogen atmosphere. To the reaction mixture was added 1-bromo-4-methoxy-2-methylbenzene (200 mg, 0.98 mmol). The reaction mixture was heated at 100 °C for 48 hours, then cooled to room temperature and concentrated under reduced pressure. The resulting crude residue was purified by silica gel column chromatography using 1% methanol in chloroform as eluent to yield the title compound, a compound of the present invention, as a solid (110 mg).
¹H NMR (500 MHz, CDCl₃) δ 2.20 (s, 3H), 2.40 (s, 3H), 3.70 (s, 3H),3.90 (s, 3H), 4.50 (d, 2H), 4.90 (bs, 1H), 6.84 (dd, 1H), 6.88 (d, 1H), 7.32 (m, 2H), 8.00 (d, 1H), 8.10 (m, 1H), 8.32 (m, 1H).

By the procedures described herein together with methods known in the art, the following compounds of Tables 1 to 47Hcan be prepared. The following abbreviations are used in the Tables which follow: *n* means normal, *i* means iso, c means cyclo, Me means methyl, Et means ethyl, Pr means propyl, OMe means methoxy, OEt means ethoxy, SMe means methylthio, SEt means ethylthio, -CN means cyano and -NO₂ means nitro.

Tables 1A-47A pertain to the structure shown below.

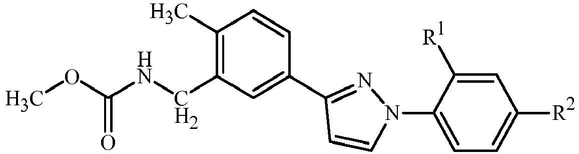

**TABLE 1A**

| R¹ is F | | | |
|---|---|---|---|
| R² | R² | R² | R² |
| F | CH=CH₂ | OCF₃ | OCH₂OCH₃ |
| Cl | CH=CHCH₃ | OCHF₂ | OCH₂CH₂OCH₃ |
| Br | CH₂CH=CH₂ | O(*c*-Pr) | SMe |
| I | C≡CH | OCH₂(*c*-Pr) | SEt |
| CN | C≡CCH₃ | CH₂(*c*-Pr) | CH₂CN |
| OH | CH₂C≡CH | OCH₂CH=CH₂ | OCH₂CN |
| NO₂ | CF₃ | OCH₂CH=CH(CH₃) | CH₂OCH₃ |
| Me | CHF₂ | OCH₂C≡CH | CH₂CH₂OCH₃ |
| Et | CH₂Cl | OCH₂C≡CCH₃ | CH₂OCH₂CH₃ |
| *n*-Pr | OMe | OCH₂CH=CHCl | OCH₂CF₃ |
| *i*-Pr | OEt | OCH₂CH=CCl₂ | OCF₂CF₂H |
| *c*-Pr | O(*n*-Pr) | OCH₂C≡CCF₃ | |

The present disclosure also includes Tables 2A through 47A, each of which is constructed the same as Table 1A above except that the row heading in Table 1A (i.e. "R¹ is F") below the Markush structure is replaced with the respective row heading shown below. For example, in Table 2A the row heading is "R¹ is Cl, and R² is as defined in Table 1A above. Thus, the first entry in Table 2A specifically discloses methyl *N*-[[5-[1-(2-chloro-4-fluorophenyl)-1*H*-pyrazol-3-yl]-2-methylphenyl]methyl]carbamate.

**TABLE 1B**

| Table | Table Headings | Table | Table Headings |
|---|---|---|---|
| 2A | R¹ is Cl | 25A | R¹ is OCF₃ |
| 3A | R¹ is Br | 26A | R¹ is OCHF₂ |
| 4A | R¹ I | 27A | R¹ is O(*c*-Pr) |
| 5A | R¹ is CN | 28A | R¹ is OCH₂(*c*-Pr) |
| 6A | R¹ is OH | 29A | R¹ is CH₂(*c*-Pr) |
| 7A | R¹ is NO₂ | 30A | R¹ is OCH₂CH=CH₂ |
| 8A | R¹ is Me | 31A | R¹ is OCH₂CH=CH(CH₃) |
| 9A | R¹ is Et | 32A | R¹ is OCH₂C≡CH |
| 10A | R¹ is *n*-Pr | 33A | R¹ is OCH₂C≡CCH₃ |
| 11A | R¹ is *i*-Pr | 34A | R¹ is OCH₂CH=CHCl |
| 12A | R¹ is *c*-Pr | 35A | R¹ is OCH₂CH=CCl₂ |
| 13A | R¹ is CH=CH₂ | 36A | R¹ is OCH₂C≡CCF₃ |
| 14A | R¹ is CH=CHCH₃ | 37A | R¹ is OCH₂OCH₃ |
| 15A | R¹ is CH₂CH=CH₂ | 38A | R¹ is OCH₂CH₂OCH₃ |
| 16A | R¹ is C≡CH | 39A | R¹ is SMe |
| 17A | R¹ is C≡CCH₃ | 40A | R¹ SEt |
| 18A | R¹ CH₂C≡CH | 41A | R¹ is CH₂CN |
| 19A | R¹ is CF₃ | 42A | R¹ is OCH₂CN |
| 20A | R¹ is CHF₂ | 43A | R¹ is CH₂OCH₃ |
| 21A | R¹ is CH₂Cl | 44A | R¹ is CH₂CH₂OCH₃ |
| 22A | R¹ is OMe | 45A | R¹ is CH₂OCH₂CH₃ |
| 23A | R¹ is OEt | 46A | R¹ is OCH₂CF₃ |
| 24A | R¹ is O(*n*-Pr) | 47A | R¹ is OCF₂CF₂H |

Table 1B is identical to Table 1A, except that the chemical structure in the Table 1B heading is replaced with the following structure: 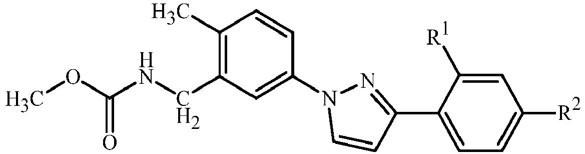 For example, the first compound in Table 1B is the structure shown immediately above wherein R¹ is F and R² is F.

### TABLES 2B-47B

Tables 2B through 47B are constructed in a similar manner as Tables 2A through 47A.

### TABLE 1C

Table 1C is identical to Table 1A, except that the chemical structure in the Table 1C heading is replaced with the following structure: 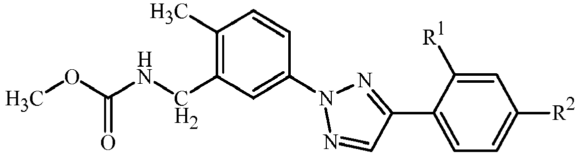 For example, the first compound in Table 1C is the structure shown immediately above wherein R¹ is F and R² is F.

### TABLES 2C-47C

Tables 2C through 47C are constructed in a similar manner as Tables 2A through 47A.

### TABLE ID

Table 1D is identical to Table 1A, except that the chemical structure in the Table 1D heading is replaced with the following structure: 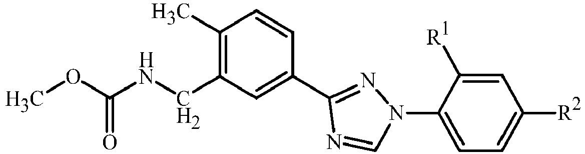 For example, the first compound in Table 1D is the structure shown immediately above wherein R¹ is F and R² is F.

### TABLES 2D-47D

Tables 2D through 47D are constructed in a similar manner as Tables 2A through 47A.

### TABLE IE

Table IE is identical to Table 1A, except that the chemical structure in the Table IE heading is replaced with the following structure: 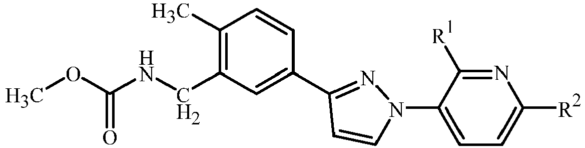 For example, the first compound in Table IE is the structure shown immediately above wherein R¹ is F and R² is F.

### TABLES 2E-47E

Tables 2E through 47E are constructed in a similar manner as Tables 2A through 47A.

### TABLE 1F

Table IF is identical to Table 1A, except that the chemical structure in the Table 1F heading is replaced with the following structure: 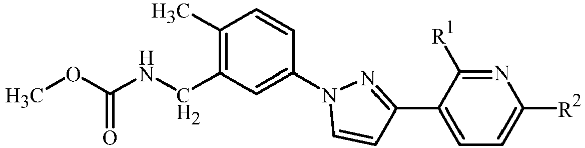 For example, the first compound in Table IF is the structure shown immediately above wherein R¹ is F and R² is F.

### TABLES 2F-47F

Tables 2F through 47F are constructed in a similar manner as Tables 2A through 47A.

### TABLE 1G

Table 1G is identical to Table 1A, except that the chemical structure in the Table 1G heading is replaced with the following structure: 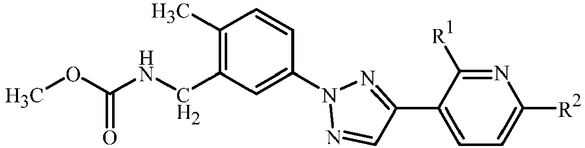 For example, the first compound in Table 1G is the structure shown immediately above wherein R¹ is F and R² is F.

### TABLES 2G-47G

Tables 2G through 47G are constructed in a similar manner as Tables 2A through 47A.

### TABLE 1H

Table 1H is identical to Table 1A, except that the chemical structure in the Table 1H heading is replaced with the following structure: 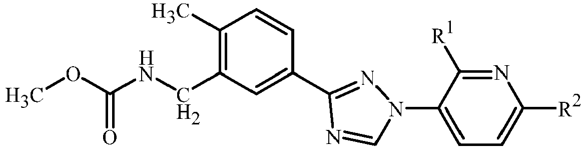 For example, the first compound in Table 1H is the structure shown immediately above wherein R¹ is F and R² is F.

### TABLES 2H-47H

Tables 2H through 47H are constructed in a similar manner as Tables 2A through 47A.

### Formulation/Utility

A compound of Formula **1** of this invention (including *N*-oxides and salts thereof) will generally be used as a fungicidal active ingredient in a composition, i.e. formulation, with at least one additional component selected from the group consisting of surfactants, solid diluents and liquid diluents, which serve as a carrier. The formulation or composition ingredients are selected to be consistent with the physical properties of the active ingredient, mode of application and environmental factors such as soil type, moisture and temperature.

Useful formulations include both liquid and solid compositions. Liquid compositions include solutions (including emulsifiable concentrates), suspensions, emulsions (including microemulsions, oil-in-water emulstions, flowable concentrates and/or suspoemulsions) and the like, which optionally can be thickened into gels. The general types of aqueous liquid compositions are soluble concentrate, suspension concentrate, capsule suspension, concentrated emulsion, microemulsion, oil-in-water emulstion, flowable concentrate and suspo-emulsion. The general types of nonaqueous liquid compositions are emulsifiable concentrate, microemulsifiable concentrate, dispersible concentrate and oil dispersion.

The general types of solid compositions are dusts, powders, granules, pellets, prills, pastilles, tablets, filled films (including seed coatings) and the like, which can be water-dispersible ("wettable") or water-soluble. Films and coatings formed from film-forming solutions or flowable suspensions are particularly useful for seed treatment. Active ingredient can be (micro)encapsulated and further formed into a suspension or solid formulation; alternatively the entire formulation of active ingredient can be encapsulated (or "overcoated"). Encapsulation can control or delay release of the active ingredient. An emulsifiable granule combines the advantages of both an emulsifiable concentrate formulation and a dry granular formulation. High-strength compositions are primarily used as intermediates for further formulation.

Sprayable formulations are typically extended in a suitable medium before spraying. Such liquid and solid formulations are formulated to be readily diluted in the spray medium, usually water, but occasionally another suitable medium like an aromatic or paraffinic hydrocarbon or vegetable oil. Spray volumes can range from about one to several thousand liters per hectare, but more typically are in the range from about ten to several hundred liters per hectare. Sprayable formulations can be tank mixed with water or another suitable medium for foliar treatment by aerial or ground application, or for application to the growing medium of the plant. Liquid and dry formulations can be metered directly into drip irrigation systems or metered into the furrow during planting. Liquid and solid formulations can be applied onto seeds of crops and other desirable vegetation as seed treatments before planting to protect developing roots and other subterranean plant parts and/or foliage through systemic uptake.

The formulations will typically contain effective amounts of active ingredient, diluent and surfactant within the following approximate ranges which add up to 100 percent by weight.

| | Weight Percent | | |
|---|---|---|---|
| | Active Ingredient | Diluent | Surfactant |
| Water-Dispersible and Water-soluble Granules, Tablets and Powders | 0.001-90 | 0-99.999 | 0-15 |
| Oil Dispersions, Suspensions, Emulsions, Solutions (including Emulsifiable Concentrates) | 1-50 | 40-99 | 0-50 |
| Dusts | 1-25 | 70-99 | 0-5 |
| Granules and Pellets | 0.001-95 | 5-99.999 | 0-15 |
| High Strength Compositions | 90-99 | 0-10 | 0-2 |

Solid diluents include, for example, clays such as bentonite, montmorillonite, attapulgite and kaolin, gypsum, cellulose, titanium dioxide, zinc oxide, starch, dextrin, sugars (e.g., lactose, sucrose), silica, talc, mica, diatomaceous earth, urea, calcium carbonate, sodium carbonate and bicarbonate, and sodium sulfate. Typical solid diluents are described in Watkins et al., Handbook of Insecticide Dust Diluents and Carriers, 2nd Ed., Dorland Books, Caldwell, New Jersey.

Liquid diluents include, for example, water, *N,N-*dimethylalkanamides (e.g., *N,N-*dimethylformamide), limonene, dimethyl sulfoxide, *N*-alkylpyrrolidones (e.g., *N*-methylpyrrolidinone), alkyl phosphates (e.g., triethyl phosphate), ethylene glycol, triethylene glycol, propylene glycol, dipropylene glycol, polypropylene glycol, propylene carbonate, butylene carbonate, paraffins (e.g., white mineral oils, normal paraffins, isoparaffins), alkylbenzenes, alkylnaphthalenes, glycerine, glycerol triacetate, sorbitol, aromatic hydrocarbons, dearomatized aliphatics, alkylbenzenes, alkylnaphthalenes, ketones such as cyclohexanone, 2-heptanone, isophorone and 4-hydroxy-4-methyl-2-pentanone, acetates such as isoamyl acetate, hexyl acetate, heptyl acetate, octyl acetate, nonyl acetate, tridecyl acetate and isobornyl acetate, other esters such as alkylated lactate esters, dibasic esters, alkyl and aryl benzoates and γ-butyrolactone, and alcohols, which can be linear, branched, saturated or unsaturated, such as methanol, ethanol, n-propanol, isopropyl alcohol, n-butanol, isobutyl alcohol, *n*-hexanol, 2-ethylhexanol, n-octanol, decanol, isodecyl alcohol, isooctadecanol, cetyl alcohol, lauryl alcohol, tridecyl alcohol, oleyl alcohol, cyclohexanol, tetrahydrofurfuryl alcohol, diacetone alcohol and benzyl alcohol. Liquid diluents also include glycerol esters of saturated and unsaturated fatty acids (typically C₆-C₂₂), such as plant seed and fruit oils (e.g., oils of olive, castor, linseed, sesame, corn (maize), peanut, sunflower, grapeseed, safflower, cottonseed, soybean, rapeseed, coconut and palm kernel), animal-sourced fats (e.g., beef tallow, pork tallow, lard, cod liver oil, fish oil), and mixtures thereof. Liquid diluents also include alkylated fatty acids (e.g., methylated, ethylated, butylated) wherein the fatty acids may be obtained by hydrolysis of glycerol esters from plant and animal sources, and can be purified by distillation. Typical liquid diluents are described in Marsden, Solvents Guide, 2nd Ed., Interscience, New York, 1950.

The solid and liquid compositions of the present invention often include one or more surfactants. When added to a liquid, surfactants (also known as "surface-active agents") generally modify, most often reduce, the surface tension of the liquid. Depending on the nature of the hydrophilic and lipophilic groups in a surfactant molecule, surfactants can be useful as wetting agents, dispersants, emulsifiers or defoaming agents.

Surfactants can be classified as nonionic, anionic or cationic. Nonionic surfactants useful for the present compositions include, but are not limited to: alcohol alkoxylates such as alcohol alkoxylates based on natural and synthetic alcohols (which may be branched or linear) and prepared from the alcohols and ethylene oxide, propylene oxide, butylene oxide or mixtures thereof; amine ethoxylates, alkanolamides and ethoxylated alkanolamides; alkoxylated triglycerides such as ethoxylated soybean, castor and rapeseed oils; alkylphenol alkoxylates such as octylphenol ethoxylates, nonylphenol ethoxylates, dinonyl phenol ethoxylates and dodecyl phenol ethoxylates (prepared from the phenols and ethylene oxide, propylene oxide, butylene oxide or mixtures thereof); block polymers prepared from ethylene oxide or propylene oxide and reverse block polymers where the terminal blocks are prepared from propylene oxide; ethoxylated fatty acids; ethoxylated fatty esters and oils; ethoxylated methyl esters; ethoxylated tristyrylphenol (including those prepared from ethylene oxide, propylene oxide, butylene oxide or mixtures thereof); fatty acid esters, glycerol esters, lanolin-based derivatives, polyethoxylate esters such as polyethoxylated sorbitan fatty acid esters, polyethoxylated sorbitol fatty acid esters and polyethoxylated glycerol fatty acid esters; other sorbitan derivatives such as sorbitan esters; polymeric surfactants such as random copolymers, block copolymers, alkyd peg (polyethylene glycol) resins, graft or comb polymers and star polymers; polyethylene glycols (pegs); polyethylene glycol fatty acid esters; silicone-based surfactants; and sugar-derivatives such as sucrose esters, alkyl polyglycosides and alkyl polysaccharides.

Useful anionic surfactants include, but are not limited to: alkylaryl sulfonic acids and their salts; carboxylated alcohol or alkylphenol ethoxylates; diphenyl sulfonate derivatives; lignin and lignin derivatives such as lignosulfonates; maleic or succinic acids or their anhydrides; olefin sulfonates; phosphate esters such as phosphate esters of alcohol alkoxylates, phosphate esters of alkylphenol alkoxylates and phosphate esters of styryl phenol ethoxylates; protein-based surfactants; sarcosine derivatives; styryl phenol ether sulfate; sulfates and sulfonates of oils and fatty acids; sulfates and sulfonates of ethoxylated alkylphenols; sulfates of alcohols; sulfates of ethoxylated alcohols; sulfonates of amines and amides such as *N,N-*alkyltaurates; sulfonates of benzene, cumene, toluene, xylene, and dodecyl and tridecylbenzenes; sulfonates of condensed naphthalenes; sulfonates of naphthalene and alkyl naphthalene; sulfonates of fractionated petroleum; sulfosuccinamates; and sulfosuccinates and their derivatives such as dialkyl sulfosuccinate salts.

Useful cationic surfactants include, but are not limited to: amides and ethoxylated amides; amines such as *N*-alkyl propanediamines, tripropylenetriamines and dipropylenetetramines, and ethoxylated amines, ethoxylated diamines and propoxylated amines (prepared from the amines and ethylene oxide, propylene oxide, butylene oxide or mixtures thereof); amine salts such as amine acetates and diamine salts; quaternary ammonium salts such as quaternary salts, ethoxylated quaternary salts and diquaternary salts; and amine oxides such as alkyldimethylamine oxides and bis-(2-hydroxyethyl)-alkylamine oxides.

Also useful for the present compositions are mixtures of nonionic and anionic surfactants or mixtures of nonionic and cationic surfactants. Nonionic, anionic and cationic surfactants and their recommended uses are disclosed in a variety of published references including McCutcheon's Emulsifiers and Detergents, annual American and International Editions published by McCutcheon's Division, The Manufacturing Confectioner Publishing Co.; Sisely and Wood, Encyclopedia of Surface Active Agents, Chemical Publ. Co., Inc., New York, 1964; and A. S. Davidson and B. Milwidsky, Synthetic Detergents, Seventh Edition, John Wiley and Sons, New York, 1987.

Compositions of this invention may also contain formulation auxiliaries and additives, known to those skilled in the art as formulation aids (some of which may be considered to also function as solid diluents, liquid diluents or surfactants). Such formulation auxiliaries and additives may control: pH (buffers), foaming during processing (antifoams such polyorganosiloxanes), sedimentation of active ingredients (suspending agents), viscosity (thixotropic thickeners), in-container microbial growth (antimicrobials), product freezing (antifreezes), color (dyes/pigment dispersions), wash-off (film formers or stickers), evaporation (evaporation retardants), and other formulation attributes. Film formers include, for example, polyvinyl acetates, polyvinyl acetate copolymers, polyvinylpyrrolidone-vinyl acetate copolymer, polyvinyl alcohols, polyvinyl alcohol copolymers and waxes. Examples of formulation auxiliaries and additives include those listed in McCutcheon's Volume 2: Functional Materials, annual International and North American editions published by McCutcheon's Division, The Manufacturing Confectioner Publishing Co.; and PCT Publication WO 03/024222.

The compound of Formula **1** and any other active ingredients are typically incorporated into the present compositions by dissolving the active ingredient in a solvent or by grinding in a liquid or dry diluent. Solutions, including emulsifiable concentrates, can be prepared by simply mixing the ingredients. If the solvent of a liquid composition intended for use as an emulsifiable concentrate is water-immiscible, an emulsifier is typically added to emulsify the active-containing solvent upon dilution with water. Active ingredient slurries, with particle diameters of up to 2,000 µm can be wet milled using media mills to obtain particles with average diameters below 3 µm. Aqueous slurries can be made into finished suspension concentrates (see, for example, U.S. 3,060,084) or further processed by spray drying to form water-dispersible granules. Dry formulations usually require dry milling processes, which produce average particle diameters in the 2 to 10 µm range. Dusts and powders can be prepared by blending and usually grinding (such as with a hammer mill or fluid-energy mill). Granules and pellets can be prepared by spraying the active material upon preformed granular carriers or by agglomeration techniques. See Browning, "Agglomeration", Chemical Engineering, December 4, 1967, pp 147-48, Perry's Chemical Engineer's Handbook, 4th Ed., McGraw-Hill, New York, 1963, pages 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see T. S. Woods, "The Formulator's Toolbox - Product Forms for Modern Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pp 81-96; Hance et al., Weed Control Handbook, 8th Ed., Blackwell Scientific Publications, Oxford, 1989; and Developments in formulation technology, PJB Publications, Richmond, UK, 2000.

In the following Examples, all percentages are by weight and all formulations are prepared in conventional ways. Compound numbers refer to compounds in Index Tables A-G. Without further elaboration, it is believed that one skilled in the art using the preceding description can utilize the present invention to its fullest extent. The following Examples are, therefore, to be constructed as merely illustrative, and not limiting of the disclosure in any way whatsoever. Percentages are by weight except where otherwise indicated.

### Example A

**High Strength Concentrate**

| | |
|---|---|
| Compound 18 | 98.5% |
| silica aerogel | 0.5% |
| synthetic amorphous fine silica | 1.0% |

### Example B

**Wettable Powder**

| | |
|---|---|
| Compound 20 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0% |

### Example C

**Granule**

| | |
|---|---|
| Compound 11 | 10.0% |
| attapulgite granules (low volatile matter, 0.71/0.30 mm; U.S.S. No. 25-50 sieves) | 90.0% |

### Example D

**Extruded Pellet**

| | |
|---|---|
| Compound 23 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

### Example E

**Emulsifiable Concentrate**

| | |
|---|---|
| Compound 8 | 10.0% |
| polyoxyethylene sorbitol hexoleate | 20.0% |
| C₆-C₁₀ fatty acid methyl ester | 70.0% |

### Example F

**Microemulsion**

| | |
|---|---|
| Compound 31 | 5.0% |
| polyvinylpyrrolidone-vinyl acetate copolymer | 30.0% |
| alkylpolyglycoside | 30.0% |
| glyceryl monooleate | 15.0% |
| water | 20.0% |

### Example G

**Seed Treatment**

| | |
|---|---|
| Compound 118 | 20.00% |
| polyvinylpyrrolidone-vinyl acetate copolymer | 5.00% |
| montan acid wax | 5.00% |
| calcium ligninsulfonate | 1.00% |
| polyoxyethylene/polyoxypropylene block copolymers | 1.00% |
| stearyl alcohol (POE 20) | 2.00% |
| polyorganosilane | 0.20% |
| colorant red dye | 0.05% |
| water | 65.75% |

### Example H

**Suspension Concentratet**

| | |
|---|---|
| Compound 46 | 35.0% |
| butyl polyoxyethylene/polypropylene block copolymers | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3-one | 0.1% |
| water | 53.7% |

Water-soluble and water-dispersible formulations are typically diluted with water to form aqueous compositions before application. Aqueous compositions for direct applications to the plant or portion thereof (e.g., spray tank compositions) typically at least about 1 ppm or more (e.g., from 1 ppm to 100 ppm) of the compound(s) of this invention.

One embodiment of the present invention relates to a method for controlling fungal pathogens, comprising diluting the fungicidal composition of the present invention (a compound of Formula **1** formulated with surfactants, solid diluents and liquid diluents or a formulated mixture of a compound of Formula **1** and at least one other fungicide) with water, and optionally adding an adjuvant to form a diluted composition, and contacting the fungal pathogen or its environment with an effective amount of said diluted composition.

Although a spray composition formed by diluting with water a sufficient concentration of the present fungicidal composition can provide sufficient efficacy for controlling fungal pathogens, separately formulated adjuvant products can also be added to spray tank mixtures. These additional adjuvants are commonly known as "spray adjuvants" or "tank-mix adjuvants", and include any substance mixed in a spray tank to improve the performance of a pesticide or alter the physical properties of the spray mixture. Adjuvants can be anionic or nonionic surfactants, emulsifying agents, petroleum-based crop oils, crop-derived seed oils, acidifiers, buffers, thickeners or defoaming agents. Adjuvants are used to enhancing efficacy (e.g., biological availability, adhesion, penetration, uniformity of coverage and durability of protection), or minimizing or eliminating spray application problems associated with incompatibility, foaming, drift, evaporation, volatilization and degradation. To obtain optimal performance, adjuvants are selected with regard to the properties of the active ingredient, formulation and target (e.g., crops, fungal pathogens).

The amount of adjuvants added to spray mixtures is generally in the range of about 2.5% to 0.1 % by volume. The application rates of adjuvants added to spray mixtures are typically between about 1 to 5 L per hectare. Representative examples of spray adjuvants include: Adigor® (Syngenta) 47% methylated rapeseed oil in liquid hydrocarbons, Silwet® (Helena Chemical Company) polyalkyleneoxide modified heptamethyltrisiloxane and Assist® (BASF) 17% surfactant blend in 83% paraffin based mineral oil.

The compounds of this invention are useful as plant disease control agents. The present invention therefore further comprises a method for controlling plant diseases caused by fungal plant pathogens comprising applying to the plant or portion thereof to be protected, or to the plant seed to be protected, an effective amount of a compound of the invention or a fungicidal composition containing said compound. The compounds and/or compositions of this invention provide control of diseases caused by a broad spectrum of fungal plant pathogens in the Basidiomycete, Ascomycete, Oomycete and Deuteromycete classes. Accordingly, mixtures and compositions described herein can control a broad spectrum of plant diseases, foliar caused by fungal pathogens of inafflicting crops and other desired vegetation including: cereal grain crops such as wheat, barley, oats, rye, triticale, rice, maize, sorghum and millet; vine crops such as table and wine grapes; field crops such as oilseed rape (canola), sunflower; , sugar beets, sugar cane, soybean, peanuts (groundnut) and, tobacco; legume forage crops such as, alfalfa, clover, lespedeza, trefoil and vetch; pome fruits such as apple, pear, crabapple, loquat, mayhaw and quince; stone fruits such as peaches, cherries, plums, apricots, nectarines and almonds; citrus fruits such as lemons, limes, oranges, grapefruit, mandarin (tangerines) and kumquat; root and tuber vegetables and field crops (and their foliage) such as artichoke, garden and sugar beet, carrot, cassava, ginger, ginseng, horseradish, parsnip, potato, radish, rutabaga, sweet potato, turnip and yam; bulb vegetables such as garlic, leek, onion and shallot; leafy vegetables such as arugula (roquette), celery, celery, cress, endive (escarole), fennel, head and leaf lettuce, parsley, radicchio (red chicory), rhubarb, spinach and Swiss chard; brassica (cole) leafy vegetables such as broccoli, broccoli raab (rapini), Brussels sprouts, cabbage, bok choy, cauliflower, collards, kale, kohlrabi and, mustard and greens; legume vegetables (succulent or dried) such as lupin, bean (Phaseolus spp.) (including field bean, kidney bean, lima bean, navy bean, pinto bean, runner bean, snap bean, tepary bean and wax bean), bean (Vigna spp.) (including adzuki bean, asparagus bean, blackeyed pea, catjang, Chinese longbean, cowpea, crowder pea, moth bean, mung bean, rice bean, southern pea, urd bean and yardlong bean), broad bean (fava), chickpea (garbanzo), guar, jackbean, lablab bean, lentil and pea (Pisum spp.) (including dwarf pea, edible-podded pea, English pea, field pea, garden pea, green pea, snowpea, sugar snap pea, pigeon pea and soybean); fruiting vegetables such as eggplant, groundcherry (Physalis spp.), pepino and pepper (including bell pepper, chili pepper, cooking pepper, pimento, and sweet pepper); tomatillo and tomato); cucurbit vegetables such as Chayote (fruit), Chinese waxgourd (Chinese preserving melon), citron melon, cucumber, gherkin, edible gourd (including hyotan, cucuzza, hechima, and Chinese okra), Momordica spp. (including balsam apple, balsam pear, bittermelon and Chinese cucumber), muskmelon (including cantaloupe and pumpkin), summer and winter squash (including butternut squash, calabaza, hubbard squash, acorn squash, spaghetti squash and pumpkin) and watermelon; berries such as blackberry (including bingleberry, boysenberry, dewberry, lowberry, marionberry, olallieberry and youngberry), blueberry, cranberry, currant, elderberry, gooseberry, huckleberry, loganberry, raspberry and strawberry; tree nuts such as almond, beech nut, Brazil nut, butternut, cashew, chestnut, chinquapin, filbert (hazelnut), hickory nut, macadamia nut, pecan and walnut; tropical fruits and other crops such as bananas, plantains, mangos, coconuts, papaya, guava, avocado, lichee, agave, coffee, cacao, sugar cane, oil palm, sesame, rubber and spices; fiber crops such as cotton, flax and hemp; turfgrasses (including warm- and cool-season turfgrasses) such as bentgrass, Kentucky bluegrass, St. Augustine grass, tall fescue and Bermuda grass.

Compounds of Formula **1,** *N*-oxides and salts thereof, and compositions comprising said compounds are useful in treating all plants, plant parts and seeds. Plant and seed varieties and cultivars can be obtained by conventional propagation and breeding methods or by genetic engineering methods. Genetically modified plants or seeds (transgenic plants or seeds) are those in which a heterologous gene (transgene) has been stably integrated into the plant"s or seed'''s genome. A transgene that is defined by its particular location in the plant genome is called a transformation or transgenic event.

Genetically modified plant and seed cultivars which can be treated according to the invention include those that are resistant against one or more biotic stresses (pests such as nematodes, insects, mites, fungi, etc.) or abiotic stresses (drought, cold temperature, soil salinity, etc.), or that contain other desirable characteristics. Plants and seeds can be genetically modified to exhibit traits of, for example, herbicide tolerance, insect-resistance, modified oil profiles or drought tolerance. Useful genetically modified plants and seeds containing single gene transformation events or combinations of transformation events are listed in Table I. Additional information for the genetic modifications listed in Table I can be obtained from the following databases: http://www2.oecd.org/biotech/byidentifier.aspx, http://www.aphis.usda.gov, and http://gmoinfo.jrc.ec.europa.eu .

The following abbreviations are used in Table I which follows: tol. is tolerance, res. is resistance, SU is sulfonylurea, ALS is acetolactate synthase, HPPD is 4-Hydroxyphenylpyruvate Dioxygenase, NA is Not Available.

**Table I**

| Crop | Event Name | Event Code | Trait(s) | Gene(s) |
|---|---|---|---|---|
| Alfalfa | J101 | MON-00101-8 | Glyphosate tol. | cp4 epsps (aroA:CP4) |
| Alfalfa | J163 | MON-∅∅163-7 | Glyphosate tol. | cp4 epsps (aroA:CP4) |
| Canola* | 23-18-17 (Event 18) | CGN-89465-2 | High lauric acid oil | te |
| Canola* | 23-198 (Event 23) | CGN-89465-2 | High lauric acid oil | te |
| Canola* | 61061 | DP-∅61∅61-7 | Glyphosate tol. | gat4621 |
| Canola* | 73496 | DP-∅73496-4 | Glyphosate tol. | gat4621 |
| Canola* | GT200 (RT200) | MON-89249-2 | Glyphosate tol. | cp4 epsps (aroA:CP4); goxv247 |
| Canola* | GT73 (RT73) | MON-∅∅∅73-7 | Glyphosate tol. | cp4 epsps (aroA:CP4); goxv247 |
| Canola* | HCN10 (Topas 19/2) | NA | Glufosinate tol. | bar |
| Canola* | HCN28 (T45) | ACS-BN∅∅8-2 | Glufosinate tol. | pat (syn) |
| Canola* | HCN92 (Topas 19/2) | ACS-BN∅∅7-1 | Glufosinate tol. | bar |
| Canola* | MON88302 | MON-883∅2-9 | Glyphosate tol. | cp4 epsps (aroA:CP4) |
| Canola* | MPS961 | NA | Phytate breakdown | phyA |
| Canola* | MPS962 | NA | Phytate breakdown | phyA |
| Canola* | MPS963 | NA | Phytate breakdown | phyA |
| Canola* | MPS964 | NA | Phytate breakdown | phyA |
| Canola* | MPS965 | NA | Phytate breakdown | phyA |
| Canola* | MS1 (B91-4) | ACS-BN∅∅4-7 | Glufosinate tol. | bar |
| Canola* | MS8 | ACS-BN∅∅5-8 | Glufosinate tol. | bar |
| Canola* | OXY-235 | ACS-BN∅11-5 | Oxynil tol. | bxn |
| Canola* | PHY14 | NA | Glufosinate tol. | bar |
| Canola* | PHY23 | NA | Glufosinate tol. | bar |
| Canola* | PHY35 | NA | Glufosinate tol. | bar |
| Canola* | PHY36 | NA | Glufosinate tol. | bar |
| Canola* | RF1 (B93-101) | ACS-BN∅∅1-4 | Glufosinate tol. | bar |
| Canola* | RF2 (B94-2) | ACS-BN∅∅2-5 | Glufosinate tol. | bar |
| Canola* | RF3 | ACS-BN∅∅3-6 | Glufosinate tol. | bar |
| Bean | EMBRAPA 5.1 | EMB-PV051-1 | Disease res. | ac1 (sense and antisense) |
| Brinjal (Eggplant) | EE-1 | | Insect res. | crylAc |
| Carnation | 11 (7442) | FLO-07442-4 | SU tol..; modified flower color | surB; dfr; hfl (f3'5'h) |
| Carnation | 11363 (1363A) | FLO-11363-1 | SU tol.; modified flower color | surB; dfr; bp40 (f3'5'h) |
| Carnation | 1226A (11226) | FLO-11226-8 | SU tol.; modified flower color | surB; dfr; bp40 (f3'5'h) |
| Carnation | 123.2.2 (40619) | FLO-4∅619-7 | SU tol.; modified flower color | surB; dfr; hfl (f3'5'h) |
| Carnation | 123.2.38 (40644) | FLO-4∅644-4 | SU tol.; modified flower color | surB; dfr; hfl (f3'5'h) |
| Carnation | 123.8.12 | FLO-4∅689-6 | SU tol.; modified flower color | surB; dfr; bp40 (f3'5'h) |
| Carnation | 123.8.8 (40685) | FLO-4∅685-1 | SU tol.; modified flower color | surB; dfr; bp40 (f3'5'h) |
| Carnation | 1351A (11351) | FLO-11351-7 | SU tol.; modified flower color | surB; dfr; bp40 (f3'5'h) |
| Carnation | 1400A (11400) | FLO-114∅∅-2 | SU tol.; modified flower color | surB; dfr; bp40 (f3'5'h) |
| Carnation | 15 | FLO-∅∅∅15-2 | SU tol.; modified flower color | surB; dfr; hfl (f3'5'h) |
| Carnation | 16 | FLO-∅∅∅16-3 | SU tol.; modified flower color | surB; dfr; hfl (f3'5'h) |
| Carnation | 4 | FLO-∅∅∅∅4-9 | SU tol.; modified flower color | surB; dfr; hfl (f3'5'h) |
| Carnation | 66 | FLO-∅∅∅66-8 | SU tol.; delayed senescence | surB; acc |
| Carnation | 959A (11959) | FLO-11959-3 | SU tol.; modified flower color | surB; dfr; bp40 (f3'5'h) |
| Carnation | 988A (11988) | FLO-11988-7 | SU tol.; modified flower color | surB; dfr; bp40 (f3'5'h) |
| Carnation | 26407 | IFD-26497-2 | SU tol.; modified flower color | surB; dfr; bp40 (f3'5'h) |
| Carnation | 25958 | IFD-25958-3 | SU tol.; modified flower color | surB; dfr; bp40 (f3'5'h) |
| Chicory | RM3-3 | NA | Glufosinate tol. | bar |
| Chicory | RM3-4 | NA | Glufosinate tol. | bar |
| Chicory | RM3-6 | NA | Glufosinate tol. | bar |
| Cotton | 19-51a | DD-01951A-7 | ALS herbicide tol. | S4-HrA |
| Cotton | 281-24-236 | DAS-24236-5 | Glufosinate tol.; insect res. | pat (syn); cry1F |
| Cotton | 3006-210-23 | DAS-21∅23-5 | Glufosinate tol.; insect res. | pat (syn); cry1Ac |
| Cotton | 31707 | NA | Oxynil tol.; insect res. | bxn; cry1Ac |
| Cotton | 31803 | NA | Oxynil tol.; insect res. | bxn; cry1Ac |
| Cotton | 31807 | NA | Oxynil tol.; insect res. | bxn; cry1Ac |
| Cotton | 31808 | NA | Oxynil tol.; insect res. | bxn; cry1Ac |
| Cotton | 42317 | NA | Oxynil tol.; insect res. | bxn; cry1Ac |
| Cotton | BNLA-601 | NA | Insect res. | cry1Ac |
| Cotton | BXN10211 | BXN10211-9 | Oxynil tol. | bxn; cry1Ac |
| Cotton | BXN10215 | BXN10215-4 | Oxynil tol. | bxn; cry1Ac |
| Cotton | BXN10222 | BXN10222-2 | Oxynil tol. | bxn; cry1Ac |
| Cotton | BXN10224 | BXN10224-4 | Oxynil tol. | bxn; cry1Ac |
| Cotton | COT102 | SYN-IR102-7 | Insect res. | vip3A(a) |
| Cotton | COT67B | SYN-IR67B-1 | Insect res. | cry1Ab |
| Cotton | COT202 | | Insect res. | vip3A |
| Cotton | Event 1 | NA | Insect res. | cry1Ac |
| Cotton | GMF CrylA | GTL-GMF311-7 | Insect res. | cry1Ab-Ac |
| Cotton | GHB119 | BCS-GH005-B | Insect res. | cry2Ae |
| Cotton | GHB614 | BCS-GH002-5 | Glyphosate tol. | 2mepsps |
| Cotton | GK12 | NA | Insect res. | cry1Ab-Ac |
| Cotton | LLCotton25 | ACS-GH001-3 | Glufosinate tol. | bar |
| Cotton | MLS 9124 | NA | Insect res. | cry1C |
| Cotton | MON1076 | MON-89924-2 | Insect res. | cry1Ac |
| Cotton | MON1445 | MON-01445-2 | Glyphosate tol. | cp4 epsps (aroA:CP4) |
| Cotton | MON15985 | MON-15985-7 | Insect res. | cry1Ac; cry2Ab2 |
| Cotton | MON1698 | MON-89383-1 | Glyphosate tol. | cp4 epsps (aroA:CP4) |
| Cotton | MON531 | MON-00531-6 | Insect res. | cry1Ac |
| Cotton | MON757 | MON-00757-7 | Insect res. | cry1Ac |
| Cotton | MON88913 | MON-88913-8 | Glyphosate tol. | cp4 epsps (aroA:CP4) |
| Cotton | Nqwe Chi 6 Bt | NA | Insect res. | NA? |
| Cotton | SKG321 | NA | Insect res. | cry1A; CpTI |
| Cotton | T303-3 | BCS-GH003-6 | Insect res.; glufosinate tol. | crylAb; bar |
| Cotton | T304-40 | BCS-GH004-7 | Insect res.; glufosinate tol. | crylAb; bar |
| Cotton | CE43-67B | | Insect res. | cry1Ab |
| Cotton | CE46-02A | | Insect res. | cry1Ab |
| Cotton | CE44-69D | | Insect res. | cry1Ab |
| Cotton | 1143-14A | | Insect res. | cry1Ab |
| Cotton | 1143-51B | | Insect res. | cry1Ab |
| Cotton | T342-142 | | Insect res. | cry1Ab |
| Cotton | PV-GHGT07 (1445) | | Glyphosate tol. | cp4 epsps (aroA:CP4) |
| Cotton | EE-GH3 | | Glyphosate tol. | mepsps |
| Cotton | EE-GH5 | | Insect res. | cry1Ab |
| Cotton | MON88701 | MON-88701-3 | Dicamba & glufosinate tol. | Modified dmo; bar |
| Cotton | OsCr11 | | Anti-allergy | Modified Cry j |
| Creeping Bentgrass | ASR368 | SMG-368∅∅-2 | Glyphosate tol. | cp4 epsps (aroA:CP4) |
| Eucalyptus | 20-C | | Salt tol. | codA |
| Eucalyptus | 12-5C | | Salt tol. | codA |
| Eucalyptus | 12-5B | | Salt tol. | codA |
| Eucalyptus | 107-1 | | Salt tol. | codA |
| Eucalyptus | 1/9/2001 | | Salt tol. | codA |
| Eucalyptus | 2/1/2001 | | Salt tol. | codA |
| Eucalyptus | | | Cold tol. | des9 |
| Flax | FP967 | CDC-FL001-2 | ALS herbicide tol. | als |
| Lentil | RH44 | | Imidazolinone tol. | als |
| Maize | 3272 | SYN-E3272-5 | Modified alpha-amylase | amy797E |
| Maize | 5307 | SYN-05307-1 | Insect res. | ecry3.1Ab |
| Maize | 59122 | DAS-.59122-7 | Insect res.; glufosinate tol. | cry34Abl; cry35Abl; pat |
| Maize | 676 | PH--000676-7 | Glufosinate tol.; pollination control | pat; dam |
| Maize | 678 | PH-0G0678÷9 | Glufosinate tol.; pollination control | pat; dam |
| Maize | 680 | PH-000680-2 | Glufosinate tol.; pollination control | pat; dam |
| Maize | 98140 | DP-098140-6 | Glyphosate toll; ALS herbicide tol. | gat4621; zm-hra |
| Maize | Bt10 | NA | Insect res.; glufosinate tol. | cry1Ab; pat |
| Maize | Bt176 (176) | SYN-EV176-9 | Insect res.; glufosinate tol. | cry1Ab; bar |
| Maize | BVLA430101 | NA | Phytate breakdown | phyA2 |
| Maize | CBH-351 | ACS-ZM004-3 | Insect res.; glufosinate tol. | cry9C; bar |
| Maize | DAS40278-9 | DAS40278-9 | 2,4-D tol. | aad-1 |
| Maize | DBT418 | DKB-89614-9 | Insect res.; glufosinate tol. | cry1Ac; pinII; bar |
| Maize | DLL25 (B16) | DKB-89790-5 | Glufosinate tol. | bar |
| Maize | GA21 | MON-00021-9 | Glyphosate tol. | mepsps |
| Maize | GG25 | | Glyphosate tol. | mepsps |
| Maize | GJ11 | | Glyphosate tol. | mepsps |
| Maize | F1117 | | Glyphosate tol. | mepsps |
| Maize | GAT-ZM1 | | Glufosinate tol. | pat |
| Maize | LY038 | REN-.00038-3 | Increased lysine | cordapA |
| Maize | MIR162 | SYN-IR162-4 | Insect res. | vip3Aa20 |
| Maize | MIR604 | SYN-IR604-5 | Insect res. | mcry3A |
| Maize | MON801 (MON80100) | MON801 | Insect res.; glyphosate tol. | cry1Ab; cp4 epsps (aroA:CP4); goxv247 |
| Maize | MON802 | MON-80200-7 | Insect res.; glyphosate tol. | cry1Ab; cp4 epsps (aroA:CP4); goxv247 |
| Maize | MON809 | PH-MON-809-2 | Insect res.; glyphosate tol. | cry1Ab; cp4 epsps (aroA:CP4); goxv247 |
| Maize | MON810 | MON-0081 0-6 | Insect res.; glyphosate tol. | cry1Ab; cp4 epsps (aroA:CP4); goxv247 |
| Maize | MON832 | NA | Glyphosate tol. | cp4 epsps (aroA:CP4); goxv247 |
| Maize | MON863 | MON-00863-5 | Insect res. | cry3Bb1 |
| Maize | MON87427 | MON-87427-7 | Glyphosate tol. | cp4 epsps (aroA:CP4) |
| Maize | MON87460 | MON-87460-4 | Drought tol. | cspB |
| Maize | MON88017 | MON-88017-3 | Insect res.; glyphosate tol. | cry3Bb1; cp4 epsps (aroA:CP4) |
| Maize | MON89034 | MON-89034-3 | Insect res. | cry2Ab2; cry1A.105 |
| Maize | MS3 | ACS-ZM001-9 | Glufosinate tol.; pollination control | bar; barnase |
| Maize | MS6 | ACS-ZM005-4 | Glufosinate tol.; pollination control | bar; barnase |
| Maize | NK603 | MON-00603-6 | Glyphosate tol. | cp4 epsps (aroA:CP4) |
| Maize | T14 | ACS-ZM002-1 | Glufosinate tol. | pat (syn) |
| Maize | T25 | ACS--ZM003-2 | Glufosinate tol. | pat (syn) |
| Maize | TC1507 | DAS-01507-1 | Insect res.; glufosinate tol. | crylFa2; pat |
| Maize | TC6275 | DAS-06275-8 | Insect res.; glufosinate tol. | mocry1F; bar |
| Maize | VIP1034 | | Insect res.; glufosinate tol. | vip3A; pat |
| Maize | 43A47 | DP--043A47-3 | Insect res.; glufosinate tol. | cry1F; cry34Ab1; cry35Ab1; pat |
| Maize | 40416 | DP--040416-8 | Insect res.; glufosinate tol. | cry1F; cry34Ab1; cry35Ab1; pat |
| Maize | 32316 | DP-032316-8 | Insect res.; glufosinate tol. | cry1F; cry34Ab1; cry35Ab1; pat |
| Maize | 4114 | DP-004114-3 | Insect res.; glufosinate tol. | cry1F; cry34Abl; cry35Abl; pat |
| Melon | Melon A | NA | Delayed ripening/senescence | sam-k |
| Melon | Melon B | NA | Delayed ripening/senescence | sam-k |
| Papaya | 55-1 | CUH-CP551-8 | Disease res. | prsv cp |
| Papaya | 63-1 | CUH-CP631-7 | Disease res. | prsv cp |
| Papaya | Huanong No. 1 | NA | Disease res. | prsv rep |
| Papaya | X17-2 | UFL-X17CP-6 | Disease res. | prsv cp |
| Petunia | Petunia-CHS | NA | Modified product quality | CHS suppres.sion |
| Plum | C-5 | ARS-PLMC5-6 | Disease res. | ppv cp |
| Canola** | ZSR500 | NA | Glyphosate tol. | cp4 epsps (aroA:CP4); goxv247 |
| Canola** | ZSR502 | NA | Glyphosate tol. | cp4 epsps (aroA:CP4); goxv247 |
| Canola** | ZSR503 | NA | Glyphosate tol. | cp4 epsps (aroA:CP4); goxv247 |
| Poplar | Bt poplar | NA | Insect res. | crylAc; API |
| Poplar | Hybrid poplar clone 741 | NA | Insect res. | crylAc; API |
| Poplar | trg300-1 | | High cellulose | AaXEG2 |
| Poplar | trg300-2 | | High cellulose | AaXEG2 |
| Potato | 1210 amk | NA | Insect res. | cry3A |
| Potato | 2904/1 kgs | NA | Insect res. | cry3A |
| Canola** | ZSR500 | NA | Glyphosate tol. | cp4 epsps (aroA:CP4); goxv247 |
| Canola** | ZSR502 | NA | Glyphosate tol. | cp4 epsps (aroA:CP4); goxv247 |
| Potato | ATBT04-27 | NMK-89367-8 | Insect res. | cry3A |
| Potato | ATBT04-30 | NMK-89613-2 | Insect res. | cry3A |
| Potato | ATBT04-31 | NMK-89170-9 | Insect res. | cry3A |
| Potato | ATBT04-36 | NMK-89279-1 | Insect res. | cry3A |
| Potato | ATBT04-6 | NMK-89761-6 | Insect res. | cry3A |
| Potato | BT06 | NMK-89812-3 | Insect res. | cry3A |
| Potato | BT10 | NMK-89175-5 | Insect res. | cry3A |
| Potato | BT12 | NMK-89601-8 | Insect res. | cry3A |
| Potato | BT16 | NMK-89167-6 | Insect res. | cry3A |
| Potato | BT17 | NMK-89593-9 | Insect res. | cry3A |
| Potato | BT18 | NMK-89906-7 | Insect res. | cry3A |
| Potato | BT23 | NMK-89675-1 | Insect res. | cry3A |
| Potato | EH92-527-1 | BPS-25271-9 | Modified starch/carbohydrate | gbss (antisense) |
| Potato | HLMT15-15 | NA | Insect & disease res. | cry3A; pvy cp |
| Potato | HLMT15-3 | NA | Insect & disease res. | cry3A; pvy cp |
| Potato | HLMT15-46 | NA | Insect & disease res. | cry3A; pvy cp |
| Potato | RBMT15-101 | NMK-89653-6 | Insect & disease res. | cry3A; pvy cp |
| Potato | RBMT21-129 | NMK-89684-1 | Insect & disease res. | cry3A; plrv orf1; plrv orf2 |
| Potato | RBMT21-152 | NA | Insect & disease res. | cry3A; plrv orf1; plrv orf2 |
| Potato | RBMT21-350 | NMK-89185-6 | Insect & disease res. | cry3A; plrv orf1; plrv orf2 |
| Potato | RBMT22-082 | NMK-89896-6 | Insect & disease res.; Glyphosate tol. | cry3A; plrv orf1; plrv orf2; cp4 epsps (aroA:CP4) |
| Potato | RBMT22-186 | NA | Insect & disease res.; Glyphosate tol. | cry3A; plrv orf1; plrv orf2; cp4 epsps (aroA:CP4) |
| Potato | RBMT22-238 | NA | Insect & disease res.; Glyphosate tol. | cry3A; plrv orf1; plrv orf2; cp4 epsps (aroA:CP4) |
| Potato | RBMT22-262 | NA | Insect & disease res.; Glyphosate tol. | cry3A; plrv orf1; plrv orf2; cp4 epsps (aroA:CP4) |
| Potato | SEMT15-02 | NMK-89935-9 | Insect & disease res. | cry3A; pvy cp |
| Potato | SEMT15-07 | NA | Insect & disease res. | cry3A; pvy cp |
| Potato | SEMT15-15 | NMK-89930-4 | Insect & disease res. | cry3A; pvy cp |
| Potato | SPBT02-5 | NMK-89576-1 | Insect res. | cry3A |
| Potato | SPBT02-7 | NMK-89724-5 | Insect res. | cry3A |
| Rice | 7Crp#242-95-7 | | Anti-allergy | 7crp |
| Rice | 7Crp#10 | NA | Anti-allergy | 7crp |
| Rice | GM Shanyou 63 | NA | Insect res. | cry1Ab; cry1Ac |
| Rice | Huahui-1/TT51-1 | NA | Insect res. | cry1Ab; cry1Ac |
| Rice | LLRICE06 | ACS-OS001-4 | Glufosinate tol. | bar |
| Rice | LLRICE601 | BCS-OS003-7 | Glufosinate tol. | bar |
| Rice | LLRICE62 | ACS-OS002-5 | Glufosinate tol. | bar |
| Rice | Tarom molaii + cry1Ab | NA | Insect res. | cry1Ab (truncated) |
| Rice | GAT-OS2 | | Glufosinate tol. | bar |
| Rice | GAT-OS3 | | Glufosinate tol. | bar |
| Rice | PE-7 | | Insect res. | Cry1Ac |
| Rice | 7Crp#10 | NA | Anti-allergy | 7crp |
| Rice | KPD627-8 | | High tryptophan | OASA1D |
| Rice | KPD722-4 | | High tryptophan | OASA1D |
| Rice | KA317 | | High tryptophan | OASA1D |
| Rice | HW5 | | High tryptophan | OASA1D |
| Rice | HW1 | | High tryptophan | OASA1D |
| Rice | B-4-1-18 | | Erect leaves semidwarf | Δ OsBRI1 |
| Rice | G-3-3-22 | | Semidwarf | OSGA2oxl |
| Rice | AD77 | | Disease res. | DEF |
| Rice | AD51 | | Disease res. | DEF |
| Rice | AD48 | | Disease res. | DEF |
| Rice | AD41 | | Disease res. | DEF |
| Rice | 13pNasNaatAprtl | | Low iron tol. | HvNAS1; HvNAAT-A; APRT |
| Rice | 13pAprtl | | Low iron tol. | APRT |
| Rice | gHvNAS1-gHvNAAT-1 | | Low iron tol. | HvNAS1; HvNAAT-A; HvNAAT-B |
| Rice | gHvIDS3-1 | | Low iron tol. | HvIDS3 |
| Rice | gHvNAAT1 | | Low iron tol. | HvNAAT-A; HvNAAT-B |
| Rice | gHvNAS1-1 | | Low iron tol. | HvNAS1 |
| Rice | NIA-OS006-4 | | Disease res. | WRKY45 |
| Rice | NIA-OS005-3 | | Disease res. | WRKY45 |
| Rice | NIA-OS004-2 | | Disease res. | WRKY45 |
| Rice | NIA-OS003-1 | | Disease res. | WRKY45 |
| Rice | NIA-OS002-9 | | Disease res. | WRKY45 |
| Rice | NIA-OS001-8 | | Disease res. | WRKY45 |
| Rice | OsCr11 | | Anti-allergy | Modified Cry j |
| Rice | 17053 | | Glyphosate tol. | cp4 epsps (aroA:CP4) |
| Rice | 17314 | | Glyphosate tol. | cp4 epsps (aroA:CP4) |
| Rose | WKS82/130-4-1 | IFD-52401-4 | Modified flower color | 5AT; bp40 (f3'5'h) |
| Rose | WKS92/130-9-1 | IFD-52901-9 | Modified flower color | 5AT; bp40 (f3'5'h) |
| Soybean | 260-05 (G94-1, G94-19, G168) | NA | Modified oil/fatty acid | gm-fad2-1 (silencing locus) |
| Soybean | A2704-12 | ACS-GM005-3 | Glufosinate tol. | pat |
| Soybean | A2704-21 | ACS-GM004-2 | Glufosinate tol. | pat |
| Soybean | A5547-127 | ACS-GM006-4 | Glufosinate tol. | pat |
| Soybean | A5547-35 | ACS-GM008-6 | Glufosinate tol. | pat |
| Soybean | CV127 | BPS-CV127-9 | Imidazolinone tol. | csr1-2 |
| Soybean | DAS68416-4 | DAS68416-4 | Glufosinate tol. | pat |
| Soybean | DP305423 | DP-305423-1 | Modified oil/fatty acid; ALS herbicide tol. | gm-fad2-1 (silencing locus); gm-hra |
| Soybean | DP356043 | DP-356043-5 | Modified oil/fatty acid; glyphosate tol. | gm-fad2-1 (silencing locus); gat4601 |
| Soybean | FG72 | MST-FG072-3 | Glyphosate & HPPD tol. | 2mepsps; hppdPF W336 |
| Soybean | GTS 40-3-2 (40-3-2) | MON-04032-6 | Glyphosate tol. | cp4 epsps (aroA:CP4) |
| Soybean | GU262 | ACS-GM003-1 | Glufosinate tol. | pat |
| Soybean | MON87701 | MON-87701-2 | Insect res. | cry1Ac |
| Soybean | MON87705 | MON-87705-6 | Modified oil/fatty acid; glyphosate tol. | fatb1-A (sense & antisense); fad2-1A (sense & antisense); cp4 epsps (aroA:CP4) |
| Soybean | MON87708 | MON-87708-9 | Dicamba & glyphosate tol. | dmo; cp4 epsps (aroA:CP4) |
| Soybean | MON87769 | MON-87769-7 | Modified oil/fatty acid; glyphosate tol. | Pj.D6D; Nc.Fad3; cp4 epsps (aroA:CP4) |
| Soybean | MON89788 | MON-89788-1 | Glyphosate tol. | cp4 epsps (aroA:CP4) |
| Soybean | W62 | ACS-GM002-9 | Glufosinate tol. | bar |
| Soybean | W98 | ACS-GM001-8 | Glufosinate tol. | bar |
| Soybean | MON87754 | MON-87754-1 | High oil | dgat2A |
| Soybean | DAS21606 | DAS-21606 | Aryloxyalkanoate & glufosinate tol. | Modified aad-12; pat |
| Soybean | DAS44406 | DAS-44406-6 | Aryloxyalkanoate, glyphosate & glufosinate tol. | Modified aad-12; 2mepsps; pat |
| Soybean | SYHT04R | SYN-0004R-8 | Mesotrione tol. | Modified avhppd |
| Soybean | 9582.814.19.1 | | Insect res. & glufosinate tol. | cry1Ac, cry1F, PAT |
| Squash | CZW3 | SEM-ØCZW3-2 | Disease res. | cmv cp, zymv cp, wmv cp |
| Squash | ZW20 | SEM-0ZW20-7 | Disease res. | zymv cp, wmv cp |
| Sugar Beet | GTSB77 (T9100152) | SY-GTSB77-8 | Glyphosate tol. | cp4 epsps (aroA:CP4); goxv247 |
| Sugar Beet | H7-1 | KM-000H71-4 | Glyphosate tol. | cp4 epsps (aroA:CP4) |
| Sugar Beet | T120-7 | ACS-BV001-3 | Glufosinate tol. | pat |
| Sugar Beet | T227-1 | | Glyphosate tol. | cp4 epsps (aroA:CP4) |
| Sugarcane | NXI-1T | | Drought tol. | EcbetA |
| Sunflower | X81359 | | Imidazolinone tol. | als |
| Sweet Pepper | PK-SP01 | NA | Disease res. | cmv cp |
| Tobacco | C/F/93/08-02 | NA | Oxynil tol. | bxn |
| Tobacco | Vector 21-41 | NA | Reduced nicotine | NtQPT1 (antisense) |
| Tomato | 1345-4 | NA | Delayed ripening/senescense | acc (truncated) |
| Tomato | 35-1-N | NA | Delayed ripening/senescense | sam-k |
| Tomato | 5345 | NA | Insect res. | cry1Ac |
| Tomato | 8338 | CGN-89322-3 | Delayed ripening/senescense | accd |
| Tomato | B | SYN-0000B-6 | Delayed ripening/senescense | pg (sense or antisense) |
| Tomato | Da | SYN-0000DA-9 | Delayed ripening/senescense | pg (sense or antisense) |
| Sunflower | X81359 | | Imidazolinone tol. | als |
| Tomato | Da Dong No 9 | NA | Modified product | NA |
| Tomato | F (1401F, h38F, 11013F,7913F) | SYN-0000F-1 | Delayed ripening/senescense | pg (sense or antisense) |
| Tomato | FLAVR SAVR™ | CGN-89564-2 | Delayed ripening/senescense | pg (sense or antisense) |
| Tomato | Huafan No 1 | NA | Delayed ripening/senescense | anti-efe |
| Tomato | PK-TM8805R (8805R) | NA | Disease res. | cmv cp |
| Wheat | MON71800 | MON-718∅∅-3 | Glyphosate tol. | cp4 epsps (aroA:CP4) |

| | | | | |
|---|---|---|---|---|
| * Argentine (Brassica napus) ** Polish (B. rapa) | | | | |

Treatment of genetically modified plants and seeds with compounds of Formula **1**, *N* oxides and salts thereof, or compositions comprising said compounds may result in superadditive or synergistic effects. For example, reduction in application rates, broadening of the activity spectrum, increased tolerance to biotic/abiotic stresses or enhanced storage stability may be greater than expected from just simple additive effects of the application of compounds of Formula 1 or their compositions with one or more additional biologically active compounds or agents on genetically modified plants and seeds.

Pathogens controlled by compounds and compositions of the present invention include: Oomycetes, including *Phytophthora* diseases such as *Phytophthora infestans, Phytophthora megasperma, Phytophthora parasitica, Phytophthora cinnamomi* and *Phytophthora capsici, Pythium* diseases such as *Pythium aphanidermatum,* and diseases in the Peronosporaceae family such as *Plasmopara viticola, Peronospora* spp. (including *Peronospora tabacina* and *Peronospora parasitica), Pseudoperonospora* spp. (including *Pseudoperonospora cubensis)* and *Bremia lactucae;* Ascomycetes, including *Alternaria* diseases such as *Alternaria solani* and *Alternaria brassicae, Guignardia* diseases such as *Guignardia bidwell, Venturia* diseases such as *Venturia inaequalis, Septoria* diseases such as *Septoria nodorum* and *Septoria tritici,* powdery mildew diseases such as *Erysiphe* spp. (including *Erysiphe graminis* and *Erysiphe polygoni*), *Uncinula necatur, Sphaerotheca fuliginea, Podosphaera leucotricha* and *Pseudocercosporella herpotrichoides, Botrytis* diseases such as *Botrytis cinerea, Monilinia fructicola, Sclerotinia* diseases such as *Sclerotinia sclerotiorum, Sclerotinia minor, Magnaporthe grisea,* and *Phomopsis viticola, Helminthosporium* diseases such as *Helminthosporium, tritici repentis* and *Pyrenophora teres,* anthracnose diseases such as *Glomerella* or *Colletotrichum* spp. (such as *Colletotrichum graminicola* and *Colletotrichum orbiculare*), and *Gaeumannomyces graminis;* Basidiomycetes, including *Puccinia* spp. causing (such as *Puccinia recondita, Puccinia striiformis, Puccinia hordei, Puccinia graminis* and *Puccinia arachidis*), *Hemileia vastatrix* and *Phakopsora pachyrhizi;* other pathogens including *Rutstroemia floccosum* (also known as *Sclerotinia homoeocarpa*); *Rhizoctonia* spp. (such as *Rhizoctonia solani*); *Fusarium* diseases such as *Fusarium roseum, Fusarium graminearum* and *Fusarium oxysporum Verticillium dahliae; Sclerotium rolfsii; Rynchosporium secalis; Cercosporidium personatum, Cercospora arachidicola* and *Cercospora beticola; Rhizopus* spp. (such as *Rhizopus stolonifer*); *Aspergillus* spp. (such as *Aspergillus flavus* and *Aspergillus parasiticus*); and other genera and species closely related to these pathogens. In addition to their fungicidal activity, the compositions or combinations also have activity against bacteria such as *Erwinia amylovora, Xanthomonas campestris, Pseudomonas syringae,* and other related species.

Furthermore, the compounds of this invention are useful in treating postharvest diseases of fruits and vegetables caused by fungi and bacteria. These infections can occur before, during and after harvest. For example, infections can occur before harvest and then remain dormant until some point during ripening (e.g., host begins tissue changes in such a way that infection can progress); also infections can arise from surface wounds created by mechanical or insect injury. In this respect, the compounds of this invention can reduce losses (i.e. losses resulting from quantity and quality) due to postharvest diseases which may occur at any time from harvest to consumption. Treatment of postharvest diseases with compounds of the invention can increase the period of time during which perishable edible plant parts (e.g., fruits, seeds, foliage, stems, bulbs. tubers) can be stored refrigerated or un-refrigerated after harvest, and remain edible and free from noticeable or harmful degradation or contamination by fungi or other microorganisms. Treatment of edible plant parts before or after harvest with compounds of the invention can also decrease the formation of toxic metabolites of fungi or other microorganisms, for example mycotoxins such as aflatoxins.

Plant disease control is ordinarily accomplished by applying an effective amount of a compound of this invention either pre- or post-infection, to the portion of the plant to be protected such as the roots, stems, foliage, fruits, seeds, tubers or bulbs, or to the media (soil or sand) in which the plants to be protected are growing. The compounds can also be applied to seeds to protect the seeds and seedlings developing from the seeds. The compounds can also be applied through irrigation water to treat plants. Control of postharvest pathogens which infect the produce before harvest is typically accomplished by field application of a compound of this invention, and in cases where infection occurs after harvest the compounds can be applied to the harvested crop as dips, sprays, fumigants, treated wraps and box liners.

Compounds of this invention are also useful in seed treatments for protecting seeds from plant diseases. In the context of the present disclosure and claims, treating a seed means contacting the seed with a biologically effective amount of a compound of this invention, which is typically formulated as a composition of the invention. This seed treatment protects the seed from plant diseases and generally can also protect roots and other plant parts in contact with the soil of the seedling developing from the germinating seed. The seed treatment may also provide protection of foliage by translocation of the compound of this invention or a second active ingredient within the developing plant. Seed treatments can be applied to all types of seeds, including those from which plants genetically transformed to express specialized traits will germinate. Representative examples include those expressing proteins toxic to invertebrate pests, such as *Bacillus thuringiensis* toxin or those expressing herbicide resistance such as glyphosate acetyltransferase, which provides resistance to glyphosate. Seed treatments with compounds of this invention can also increase vigor of plants growing from the seed.

One method of seed treatment is by spraying or dusting the seed with a compound of the invention (i.e. as a formulated composition) before sowing the seeds. Compositions formulated for seed treatment generally comprise a film former or adhesive agent. Therefore typically a seed coating composition of the present invention comprises a biologically effective amount of a compound of Formula **1** and a film former or adhesive agent. Seed can be coated by spraying a flowable suspension concentrate directly into a tumbling bed of seeds and then drying the seeds. Alternatively, other formulation types such as wetted powders, solutions, suspoemulsions, emulsifiable concentrates and emulsions in water can be sprayed on the seed. This process is particularly useful for applying film coatings on seeds. Various coating machines and processes are available to one skilled in the art. Suitable processes include those listed in P. Kosters et al., Seed Treatment: Progress and Prospects, 1994 BCPC Mongraph No. 57, and references listed therein.

Compounds of Formula **1** and their compositions, both alone and in combination with other insecticides, nematicides, and fungicides, are particularly useful in seed treatment for crops including, but not limited to, maize or corn, soybeans, cotton, cereal (e.g., wheat, oats, barley, rye and rice), potatoes, vegetables and oilseed rape.

Other insecticides or nematicides with which compounds of Formula **1** can be formulated to provide mixtures useful in seed treatment include but are not limited to abamectin, acephate, acetamiprid, acetoprole, acrinathrin, afidopyropen, aldicarb, amidoflumet, amitraz, avermectin, azadirachtin, azinphos-methyl, bensultap, bifenthrin, bifenazate, bistrifluron, buprofezin, cadusafos, carbofuran, carbaryl, carbofuran, cartap, chinomethionat, chlorantraniliprole, chlorfenapyr, chlorfluazuron, chlorpyrifos, chlorpyrifos-methyl, chlorobenzilate, chromafenozide, clothianidin, cyantraniliprole, cyclaniliprole, cyflumetofen, cyfluthrin, beta-cyfluthrin, cyhalothrin, gamma-cyhalothrin, lambda-cyhalothrin, cyhexatin, cypermethrin, alpha-cypermethrin, zeta-cypermethrin, cyromazine, deltamethrin, diafenthiuron, diazinon, dicofol, dieldrin, dienochlor, diflubenzuron, dimefluthrin, dimethoate, dinotefuran, diofenolan, emamectin, endosulfan, esfenvalerate, ethiprole, etofenprox, etoxazole, fenamiphos, fenazaquin, fenbutatin oxide, fenothiocarb, fenoxycarb, fenpropathrin, fenpyroximate, fenvalerate, fipronil, flonicamid, flubendiamide, flucythrinate, flufenerim, flufenoxuron, flufiprole, flupyradifurone, tau-fluvalinate, fonophos, formetanate, halofenozide, heptafluthrin, hexaflumuron, hexythiazox, hydramethylnon, imicyafos, imidacloprid, indoxacarb, isofenphos, lufenuron, malathion, meperfluthrin, metaflumizone, metaldehyde, methamidophos, methidathion, methiocarb, methomyl, methoprene, methoxychlor, methoxyfenozide, metofluthrin, milbemycin oxime, momfluorothrin, monocrotophos, nicotine, nitenpyram, nithiazine, novaluron, noviflumuron, oxamyl, parathion, parathion-methyl, permethrin, phorate, phosalone, phosmet, phosphamidon, pirimicarb, profenofos, profluthrin, propargite, prothiocarb, protrifenbute, pymetrozine, pyrafluprole, pyrethrin, pyridaben, pyridalyl, pyrifluquinazon, pyriprole, pyriproxyfen, rotenone, ryanodine, spinetoram, spinosad, spiridiclofen, spiromesifen, spirotetramat, sulfoxaflor, sulprofos, tebufenozide, tebufenpyrad, teflubenzuron, tefluthrin, terbufos, tetrachlorvinphos, tetramethrin, tetramethylfluthrin, thiacloprid, thiamethoxam, thiodicarb, thiosultap-sodium, tolfenpyrad, tralomethrin, triazamate, trichlorfon and triflumuron; nematocides such as aldicarb, fenamiphos, fluensulfone, fosthiazate, imicyafos and oxamyl; bactericides such as streptomycin; acaricides such as amitraz, chinomethionat, chlorobenzilate, cyenopyrafen, cyhexatin, dicofol, dienochlor, etoxazole, fenazaquin, fenbutatin oxide, fenpropathrin, fenpyroximate, flufenoxystrobin, hexythiazox, propargite, pyflubumide, pyridaben, pyriminostrobin and tebufenpyrad; and biological agents including entomopathogenic bacteria, such as *Bacillus thuringiensis* subsp. *aizawai, Bacillus thuringiensis* subsp. *kurstaki,* and the encapsulated delta-endotoxins of *Bacillus thuringiensis* (e.g., Cellcap, MPV, MPVII); entomopathogenic fungi, such as green muscardine fungus; and entomopathogenic virus including baculovirus, nucleopolyhedro virus (NPV) such as HzNPV, AfNPV; and granulosis virus (GV) such as CpGV.

Fungicides with which compounds of Formula **1** can be formulated to provide mixtures useful in seed treatment include but are not limited to amisulbrom, azoxystrobin, boscalid, carbendazim, carboxin, cymoxanil, cyproconazole, difenoconazole, dimethomorph, fluazinam, fludioxonil, fluquinconazole, fluopicolide, fluoxastrobin, flutriafol, fluxapyroxad, ipconazole, iprodione, metalaxyl, mefenoxam, metconazole, myclobutanil, paclobutrazole, penflufen, picoxystrobin, prothioconazole, pyraclostrobin, sedaxane, silthiofam, tebuconazole, thiabendazole, thiophanate-methyl, thiram, trifloxystrobin and triticonazole.

Compositions comprising compounds of Formula **1** useful for seed treatment can further comprise bacteria and fungi that have the ability to provide protection from the harmful effects of plant pathogenic fungi or bacteria and/or soil born animals such as nematodes. Bacteria exhibiting nematicidal properties may include but are not limited to *Bacillus firmus, Bacillus cereus, Bacillius subtiliis* and *Pasteuria penetrans.* A suitable *Bacillus firmus* strain is strain CNCM 1-1582 (GB-126) which is commercially available as BioNem™. A suitable *Bacillus cereus* strain is strain NCMM 1-1592. Both *Bacillus* strains are disclosed in US 6,406,690. Other suitable bacteria exhibiting nematicidal activity are *B. amyloliquefaciens* IN937a and *B. subtilis* strain GB03. Bacteria exhibiting fungicidal properties may include but are not limited to *B. pumilus* strain GB34. Fungal species exhibiting nematicidal properties may include but are not limited to *Myrothecium verrucaria, Paecilomyces lilacinus* and *Purpureocillium lilacinum.*

Seed treatments can also include one or more nematicidal agents of natural origin such as the elicitor protein called harpin which is isolated from certain bacterial plant pathogens such as *Erwinia amylovora.* An example is the Harpin-N-Tek seed treatment technology available as N-Hibit™ Gold CST.

Seed treatments can also include one or more species of legume-root nodulating bacteria such as the microsymbiotic nitrogen-fixing bacteria *Bradyrhizobium japonicum.* These inocculants can optionally include one or more lipo-chitooligosaccharides (LCOs), which are nodulation (Nod) factors produced by rhizobia bacteria during the initiation of nodule formation on the roots of legumes. For example, the Optimize® brand seed treatment technology incorporates LCO Promoter Technology™ in combination with an inocculant.

Seed treatments can also include one or more isoflavones which can increase the level of root colonization by mycorrhizal fungi. Mycorrhizal fungi improve plant growth by enhancing the root uptake of nutrients such as water, sulfates, nitrates, phosphates and metals. Examples of isoflavones include, but are not limited to, genistein, biochanin A, formononetin, daidzein, glycitein, hesperetin, naringenin and pratensein. Formononetin is available as an active ingredient in mycorrhizal inocculant products such as PHC Colonize® AG.

Seed treatments can also include one or more plant activators that induce systemic acquired resistance in plants following contact by a pathogen. An example of a plant activator which induces such protective mechanisms is acibenzolar-S-methyl.

Rates of application for these compounds (i.e. a fungicidally effective amount) can be influenced by factors such as the plant diseases to be controlled, the plant species to be protected, ambient moisture and temperature and should be determined under actual use conditions. One skilled in the art can easily determine through simple experimentation the fungicidally effective amount necessary for the desired level of plant disease control. Foliage can normally be protected when treated at a rate of from less than about 1 g/ha to about 5,000 g/ha of active ingredient. Seed and seedlings can normally be protected when seed is treated at a rate of from about 0.1 to about 10 g per kilogram of seed.

Compounds of this invention can also be mixed with one or more other biologically active compounds or agents including fungicides, insecticides, nematocides, bactericides, acaricides, herbicides, herbicide safeners, growth regulators such as insect molting inhibitors and rooting stimulants, chemosterilants, semiochemicals, repellents, attractants, pheromones, feeding stimulants, plant nutrients, other biologically active compounds or entomopathogenic bacteria, virus or fungi to form a multi-component pesticide giving an even broader spectrum of agricultural protection. Thus the present invention also pertains to a composition comprising a compound of Formula **1** (in a fungicidally effective amount) and at least one additional biologically active compound or agent (in a biologically effective amount) and can further comprise at least one of a surfactant, a solid diluent or a liquid diluent. The other biologically active compounds or agents can be formulated in compositions comprising at least one of a surfactant, solid or liquid diluent. For mixtures of the present invention, one or more other biologically active compounds or agents can be formulated together with a compound of Formula **1,** to form a premix, or one or more other biologically active compounds or agents can be formulated separately from the compound of Formula **1,** and the formulations combined together before application (e.g., in a spray tank) or, alternatively, applied in succession.

One aspect of the present invention is a fungicidal composition comprising (i.e. a mixture or combination of) a compound of Formula **1,** an *N*-oxide, or a salt thereof (i.e. component a), and at least one other fungicide (i.e. component b). Of note is such a combination where the other fungicidal active ingredient has different site of action from the compound of Formula **1.** In certain instances, a combination with at least one other fungicidal active ingredient having a similar spectrum of control but a different site of action will be particularly advantageous for resistance management. Thus, a composition of the present invention can further comprise a fungicidally effective amount of at least one additional fungicidal active ingredient having a similar spectrum of control but a different site of action.

Of note is a composition which in addition to the compound of Formula **1** include at least one fungicidal compound selected from the group consisting of the classes (1) methyl benzimidazole carbamate (MBC) fungicides; (2) dicarboximide fungicides; (3) demethylation inhibitor (DMI) fungicides; (4) phenylamide fungicides; (5) amine/morpholine fungicides; (6) phospholipid biosynthesis inhibitor fungicides; (7) carboxamide fungicides; (8) hydroxy(2-amino-)pyrimidine fungicides; (9) anilinopyrimidine fungicides; (10) *N*-phenyl carbamate fungicides; (11) quinone outside inhibitor (QoI) fungicides; (12) phenylpyrrole fungicides; (13) quinoline fungicides; (14) lipid peroxidation inhibitor fungicides; (15) melanin biosynthesis inhibitors-reductase (MBI-R) fungicides; (16) melanin biosynthesis inhibitors-dehydratase (MBI-D) fungicides; (17) hydroxyanilide fungicides; (18) squalene-epoxidase inhibitor fungicides; (19) polyoxin fungicides; (20) phenylurea fungicides; (21) quinone inside inhibitor (QiI) fungicides; (22) benzamide fungicides; (23) enopyranuronic acid antibiotic fungicides; (24) hexopyranosyl antibiotic fungicides; (25) glucopyranosyl antibiotic: protein synthesis fungicides; (26) glucopyranosyl antibiotic: trehalase and inositol biosynthesis fungicides; (27) cyanoacetamideoxime fungicides; (28) carbamate fungicides; (29) oxidative phosphorylation uncoupling fungicides; (30) organo tin fungicides; (31) carboxylic acid fungicides; (32) heteroaromatic fungicides; (33) phosphonate fungicides; (34) phthalamic acid fungicides; (35) benzotriazine fungicides; (36) benzene-sulfonamide fungicides; (37) pyridazinone fungicides; (38) thiophene-carboxamide fungicides; (39) pyrimidinamide fungicides; (40) carboxylic acid amide (CAA) fungicides; (41) tetracycline antibiotic fungicides; (42) thiocarbamate fungicides; (43) benzamide fungicides; (44) host plant defense induction fungicides; (45) multi-site contact activity fungicides; (46) fungicides other than classes (1) through (45); and salts of compounds of classes (1) through (46).

Further descriptions of these classes of fungicidal compounds are provided below.
(1) "Methyl benzimidazole carbamate (MBC) fungicides" (Fungicide Resistance Action Committee (FRAC) code 1) inhibit mitosis by binding to β-tubulin during microtubule assembly. Inhibition of microtubule assembly can disrupt cell division, transport within the cell and cell structure. Methyl benzimidazole carbamate fungicides include benzimidazoles and thiophanates. The benzimidazoles include benomyl, carbendazim, fuberidazole and thiabendazole. The thiophanates include thiophanate and thiophanate-methyl.
(2) "Dicarboximide fungicides" (FRAC code 2) inhibit MAP/histidine kinase in osmotic signal transduction. Examples include chlozolinate, iprodione, procymidone and vinclozolin.
(3) "Demethylation inhibitor (DMI) fungicides" (FRAC code 3) (Sterol Biosynthesis Inhibitors (SBI): Class I) inhibit C14-demethylase, which plays a role in sterol production. Sterols, such as ergosterol, are needed for membrane structure and function, making them essential for the development of functional cell walls. Therefore, exposure to these fungicides results in abnormal growth and eventually death of sensitive fungi. Demethylation fungicides are divided between several chemical classes: azoles (including triazoles and imidazoles), piperazines, pyridines, pyrimidines, and triazolinthiones. The piperazines include triforine. The pyridines include buthiobate, pyrifenox, pyrisoxazole (3-[(3*R*)-5-(4-chlorophenyl)-2,3-dimethyl-3-isoxazolidinyl]pyridine, mixture of 3*R*,5*R-* and 3*R*,5*S*-isomers) and (α*S*)-[3-(4-chloro-2-fluorophenyl)-5-(2,4-difluorophenyl)-4-isoxazolyl]-3-pyridinemethanol. The triazolinthiones include prothioconazole and 2-[2-(1-chlorocyclopropyl)-4-(2,2-dichlorocyclopropyl)-2-hydroxybutyl]-1,2-dihydro-3*H*-1,2,4-triazole-3-thione. The pyrimidines include fenarimol, nuarimol and triarimol. The imidazoles include clotrimazole, imazalil, oxpoconazole, prochloraz, pefurazoate and triflumizole. The triazoles include azaconazole, bitertanol, bromuconazole, cyproconazole, difenoconazole, diniconazole (including diniconazole-M), epoxiconazole, fenbuconazole, fluquinconazole, flusilazole, flutriafol, hexaconazole, imibenconazole, ipconazole, metconazole, myclobutanil, penconazole, propiconazole, quinconazole, simeconazole, tebuconazole, tetraconazole, triadimefon, triadimenol, triticonazole, uniconazole and α-(1-chlorocyclopropyl)-α-[2-(2,2-dichlorocyclopropyl)ethyl]-1*H*-1,2,4-triazole-1-ethanol. Biochemical investigations have shown that all of the above mentioned fungicides are DMI fungicides as described by K. H. Kuck et al. in Modern Selective Fungicides - Properties, Applications and Mechanisms of Action, H. Lyr (Ed.), Gustav Fischer Verlag: New York, 1995, 205-258.
(4) "Phenylamide fungicides" (FRAC code 4) are specific inhibitors of RNA polymerase in Oomycete fungi. Sensitive fungi exposed to these fungicides show a reduced capacity to incorporate uridine into rRNA. Growth and development in sensitive fungi is prevented by exposure to this class of fungicide. Phenylamide fungicides include acylalanines, oxazolidinones and butyrolactones. The acylalanines include benalaxyl, benalaxyl-M, (also known as kiralaxyl), furalaxyl, metalaxyl and metalaxyl-M/mefenoxam. The oxazolidinones include oxadixyl. The butyrolactones include ofurace.
(5) "Amine/morpholine fungicides" (FRAC code 5) (SBI: Class II) inhibit two target sites within the sterol biosynthetic pathway, Δ⁸ → Δ⁷ isomerase and Δ¹⁴ reductase. Sterols, such as ergosterol, are needed for membrane structure and function, making them essential for the development of functional cell walls. Therefore, exposure to these fungicides results in abnormal growth and eventually death of sensitive fungi. Amine/morpholine fungicides (also known as non-DMI sterol biosynthesis inhibitors) include morpholines, piperidines and spiroketal-amines. The morpholines include aldimorph, dodemorph, fenpropimorph, tridemorph and trimorphamide. The piperidines include fenpropidin and piperalin. The spiroketal-amines include spiroxamine.
(6) "Phospholipid biosynthesis inhibitor fungicides" (FRAC code 6) inhibit growth of fungi by affecting phospholipid biosynthesis. Phospholipid biosynthesis fungicides include phophorothiolates and dithiolanes. The phosphorothiolates include edifenphos, iprobenfos and pyrazophos. The dithiolanes include isoprothiolane.
(7) "Carboxamide fungicides" (FRAC code 7) also known as "Succinate dehydrogenase inhibitor (SDHI) fungicides", inhibit Complex II fungal respiration by disrupting a key enzyme in the Krebs Cycle (TCA cycle) named succinate dehydrogenase. Inhibiting respiration prevents the fungus from making ATP, and thus inhibits growth and reproduction. Carboxamide fungicides include phenylbenzamides, pyridinyl ethyl benzamides, furan carboxamides, oxathiin carboxamides, thiazole carboxamides, pyrazole-4-carboxamides, pyridine carboxamides, phenyl oxoethyl thiophene amides and pyridinylethyl benzamides. The phenylbenzamides include benodanil, flutolanil and mepronil. The pyridinyl ethyl benzamides include fluopyram. The furan carboxamides include fenfuram. The oxathiin carboxamides include carboxin and oxycarboxin. The thiazole carboxamides include thifluzamide. The pyrazole-4-carboxamides include benzovindiflupyr (*N*-[9-(dichloromethylene)-1,2,3,4-tetrahydro-1,4-methanonaphthalen-5-yl]-3-(difluoromethyl)-1-methyl-1*H*-pyrazole-4-carboxamide), bixafen, furametpyr, isopyrazam (3-(difluoromethyl)-1-methyl-*N*-[1,2,3,4-tetrahydro-9-(1-methylethyl)-1,4-methanonaphthalen-5-yl]-1*H-*pymzole-4-carboxamide), fluxapyroxad (3-(difluoromethyl)-1-methyl-*N*-(3,4,5-trifluoro[1,1'-biphenyl]-2-yl)-1*H-*pyrazole-4-carboxamide), penthiopyrad, sedaxane (*N*-[2-[1,1'-bicyclopropyl]-2-ylphenyl]-3-(difluoromethyl)-1-methyl-1*H*-pyrazole-4-carboxamide), *N*-[2-(1*S*,2*R*)-[1,1'-bicyclopropyl]-2-ylphenyl]-3-(difluoromethyl)-1 -methyl-1*H*-pyrazole-4-carboxamide, 3-(difluoromethyl)-*N*-(2,3-dihydro-1,1,3-trirnethyl-1*H*-inden-4-yl)-1-methyl-1*H*-pyrazole-4-carboxamide, *N*-[2-(2,4-dichlorophenyl)-2-methoxy-1-methylethyl]-3-(difluoromethyl)-1-methyl-1*H*-pyrazole-4-carboxamide, *N*-cyclopropyl-3-(difluoromethyl)-5-fluoro-1-methyl-*N*-[[2-(1-methylethyl)phenyl]methyl]-1*H*-pyrazole-4-carboxamide and penflufen (*N*-[2-(1,3-dimethylbutyl)phenyl]-5-fluoro-1,3-dimethyl-1*H*-pyrazole-4-carboxamide) (PCT Patent Publication WO 2003/010149). The pyridine carboxamides include boscalid. The phenyl oxoethyl thiophene amides include isofetamid, (*N-*[1,1-dimethyl-2-[2-methyl-4-(1-methylethoxy)phenyl]-2-oxoethyl]-3-methyl-2-thiophene-carboxamide). The pyridinylethyl benzamides include fluopyram.
(8) "Hydroxy(2-amino-)pyrimidine fungicides" (FRAC code 8) inhibit nucleic acid synthesis by interfering with adenosine deaminase. Examples include bupirimate, dimethirimol and ethirimol.
(9) "Anilinopyrimidine fungicides" (FRAC code 9) are proposed to inhibit biosynthesis of the amino acid methionine and to disrupt the secretion of hydrolytic enzymes that lyse plant cells during infection. Examples include cyprodinil, mepanipyrim and pyrimethanil.
(10) "*N*-Phenyl carbamate fungicides" (FRAC code 10) inhibit mitosis by binding to β-tubulin and disrupting microtubule assembly. Inhibition of microtubule assembly can disrupt cell division, transport within the cell and cell structure. Examples include diethofencarb.
(11) "Quinone outside inhibitor (QoI) fungicides" (FRAC code 11) inhibit Complex III mitochondrial respiration in fungi by affecting ubiquinol oxidase. Oxidation of ubiquinol is blocked at the "quinone outside" (Qₒ) site of the cytochrome *bc*₁ complex, which is located in the inner mitochondrial membrane of fungi. Inhibiting mitochondrial respiration prevents normal fungal growth and development. Quinone outside inhibitor fungicides (also known as strobilurin fungicides) include methoxyacrylates, methoxycarbamates, oximinoacetates, oximinoacetamides, oxazolidinediones, dihydrodioxazines, imidazolinones and benzylcarbamates. The methoxyacrylates include azoxystrobin, coumoxystrobin (methyl (α*E*)-2-[[(3-butyl-4-methyl-2-oxo-2*H*-1-benzopyran-7-yl)oxy]methyl]-α-(methoxymethylene)benzeneacetate), enoxastrobin (methyl (α*E*)-2-[[[(*E*)-[(2*E*)-3-(4-chlorophenyl)-1-methyl-2-propen-1-ylidene]amino]oxy]methyl]-α-(methoxymethylene)-benzeneaceate) (also known as enestroburin), flufenoxystrobin (methyl (α*E*)-2-[[2-chloro-4-(trifluoromethyl)phenoxy]methyl]-α-(methoxymethylene)benzeneacetate), picoxystrobin and pyraoxystrobin (methyl (α*E*)-2-[[[3-(4-chlorophenyl)-1-methyl-1*H-*pyrazol-5-yl]oxy]-methyl]-α-(methoxymethylene)benzeneacetate). The methoxycarbamates include pyraclostrobin, pyrametostrobin (methyl *N*-[2-[[(1,4-dimethyl-3-phenyl-1*H*-pyrazol-5-yl)oxy]methyl]phenyl]-*N*-methoxycarbamate) and triclopyricarb (methyl *N*-methoxy-*N*-[2-[[(3,5,6-trichloro-2-pyridinyl)oxy]methyl]phenyl]carbamate). The oximinoacetates include kresoxim-methyl and trifloxystrobin. The oximinoacetamides include dimoxystrobin, fenaminstrobin ((α*E*)-2-[[[(*E*)-[(2*E*)-3-(2,6-dichlorophenyl)-1-methyl-2-propen-1-ylidene]-amino]oxy]methyl]-α-(methoxyimino)-*N*-methylbenzeneacetamide, also known as 2-[[[(3-(2,6-dichlorophenyl)-1-methyl-2-propen-1-ylidene]amino]oxy]methyl]-α-(methoxyimino)-*N*-methylbenzeneacetamide), metominostrobin, orysastrobin and α-(methoxyimino)-*N-*methyl-2-[[[1 -[3-(trifluoromethyl)phenyl]ethoxy]imino]methyl]benzeneacetamide. The dihydrodioxazines include fluoxastrobin. The oxazolidinediones include famoxadone. The imidazolinones include fenamidone. The benzylcarbamates include pyribencarb. Class (c11) also includes mandestrobin (2-[(2,5-dimethylphenoxy)methyl]-α-methoxy-*N-*benzeneacetamide).
(12) "Phenylpyrrole fungicides" (FRAC code 12) inhibit a MAP histidine kinase associated with osmotic signal transduction in fungi. Fenpiclonil and fludioxonil are examples of this fungicide class.
(13) "Azanaphthalene fungicides" (FRAC code 13), also known as "azanaphthalene fungicides, are proposed to inhibit signal transduction by a mechanism which is yet unknown. They have been shown to interfere with germination and/or appressorium formation in fungi that cause powder mildew diseases. Quinoline fungicides include aryloxyquinolines and auinazolinones. The aryloxyquinolines include quinoxyfen and tebufloquin. The quinazolinones include proquinazid.
(14) "Lipid peroxidation inhibitor fungicides" (FRAC code 14) are proposed to inhibit lipid peroxidation which affects membrane synthesis in fungi. Members of this class, such as etridiazole, may also affect other biological processes such as respiration and melanin biosynthesis. Lipid peroxidation fungicides include aromatic hydrocarbons and 1,2,4-thiadiazoles. The aromatic hydrocarbon fungicides include biphenyl, chloroneb, dicloran, quintozene, tecnazene and tolclofos-methyl. The 1,2,4-thiadiazole fungicides include etridiazole.
(15) "Melanin biosynthesis inhibitors-reductase (MBI-R) fungicides" (FRAC code 16.1) inhibit the naphthal reduction step in melanin biosynthesis. Melanin is required for host plant infection by some fungi. Melanin biosynthesis inhibitors-reductase fungicides include isobenzofuranones, pyrroloquinolinones and triazolobenzothiazoles. The isobenzofuranones include phthalide (alternatively spelled fthalide). The pyrroloquinolinones include pyroquilon. The triazolobenzothiazoles include tricyclazole.
(16) "Melanin biosynthesis inhibitors-dehydratase (MBI-D) fungicides" (FRAC code 16.2) inhibit scytalone dehydratase in melanin biosynthesis. Melanin in required for host plant infection by some fungi. Melanin biosynthesis inhibitors-dehydratase fungicides include cyclopropanecarboxamides, carboxamides and propionamides. The cyclopropanecarboxamides include carpropamid. The carboxamides include diclocymet. The propionamides include fenoxanil.
(17) "Hydroxyanilide fungicides (FRAC code 17) Sterol Biosynthesis Inhibitor (SBI): Class III fungicides inhibit 3-ketoreductase during C4-demethylation in sterol production. SBI: Class III inhibitors include hydroxyanilide fungicides and aminopyrazolinone fungicides. Hydroxyanilides include fenhexamid. Aminopyrazolinones include fenpyrazamine (*S*-2-propen-1-yl5-amino-2,3-dihydro-2-(1-methylethyl)-4-(2-methylphenyl)-3-oxo-1*H*-pyrazole-1-carbothioate, also known as 1-[(2-propenylthio)carbonyl]-2-(1-methylethyl)-4-(2-methylphenyl)-5-amino-1*H-*pyrazol-3-one,).
(18) "Squalene-epoxidase inhibitor fungicides" (FRAC code 18) inhibit squalene-epoxidase in the sterol biosynthesis pathway. Sterols such as ergosterol are needed for membrane structure and function, making them essential for the development of functional cell walls. Therefore exposure to these fungicides results in abnormal growth and eventually death of sensitive fungi. Squalene-epoxidase inhibitor fungicides include thiocarbamates and allylaminess. The thiocarbamates include pyributicarb. The allylamines include naftifine and terbinafine.
(19) "Polyoxin fungicides" (FRAC code 19) inhibit chitin synthase. Examples include polyoxin.
(20) "Phenylurea fungicides" (FRAC code 20) are proposed to affect cell division. Examples include pencycuron.
(21) "Quinone inside inhibitor (QiI) fungicides" (FRAC code 21) inhibit Complex III mitochondrial respiration in fungi by affecting ubiquinolonel reductase. Reduction of ubiquinol is blocked at the "quinone inside" (Qᵢ) site of the cytochrome *bc*₁ complex, which is located in the inner mitochondrial membrane of fungi. Inhibiting mitochondrial respiration prevents normal fungal growth and development. Quinone inside inhibitor fungicides include cyanoimidazoles and sulfamoyltriazoles. The cyanoimidazoles include cyazofamid. The sulfamoyltriazoles include amisulbrom.
(22) "Benzamide and thiazolecarboxamide fungicides" (FRAC code 22) (also known simply as "benzamide fungicides") inhibit mitosis by binding to β-tubulin and disrupting microtubule assembly. Inhibition of microtubule assembly can disrupt cell division, transport within the cell and cell structure. The benzamides include zoxamide. The thiazolecarboxamides include ethaboxam.
(23) "Enopyranuronic acid antibiotic fungicides" (FRAC code 23) inhibit growth of fungi by affecting protein biosynthesis. Examples include blasticidin-S.
(24) "Hexopyranosyl antibiotic fungicides" (FRAC code 24) inhibit growth of fungi by affecting protein biosynthesis. Examples include kasugamycin.
(25) "Glucopyranosyl antibiotic: protein synthesis fungicides" (FRAC code 25) inhibit growth of fungi by affecting protein biosynthesis. Examples include streptomycin.
(26) "Glucopyranosyl antibiotic: trehalase and inositol biosynthesis fungicides" (FRAC code 26) inhibit trehalase and inositol biosynthesis. Examples include validamycin.
(27) "Cyanoacetamideoxime fungicides (FRAC code 27) include cymoxanil.
(28) "Carbamate fungicides" (FRAC code 28) are considered multi-site inhibitors of fungal growth. They are proposed to interfere with the synthesis of fatty acids in cell membranes, which then disrupts cell membrane permeability. Propamacarb, propamacarb-hydrochloride, iodocarb, and prothiocarb are examples of this fungicide class.
(29) "Oxidative phosphorylation uncoupling fungicides" (FRAC code 29) inhibit fungal respiration by uncoupling oxidative phosphorylation. Inhibiting respiration prevents normal fungal growth and development. This class includes 2,6-dinitroanilines such as fluazinam, and dinitrophenyl crotonates such as dinocap, meptyldinocap and binapacryl.
(30) "Organo tin fungicides" (FRAC code 30) inhibit adenosine triphosphate (ATP) synthase in oxidative phosphorylation pathway. Examples include fentin acetate, fentin chloride and fentin hydroxide.
(31) "Carboxylic acid fungicides" (FRAC code 31) inhibit growth of fungi by affecting deoxyribonucleic acid (DNA) topoisomerase type II (gyrase). Examples include oxolinic acid.
(32) "Heteroaromatic fungicides" (FRAC code 32) are proposed to affect DNA/ribonucleic acid (RNA) synthesis. Heteroaromatic fungicides include isoxazoles and isothiazolones. The isoxazoles include hymexazole and the isothiazolones include octhilinone.
(33) "Phosphonate fungicides" (FRAC code 33) include phosphorous acid and its various salts, including fosetyl-aluminum.
(34) "Phthalamic acid fungicides" (FRAC code 34) include teclofthalam.
(35) "Benzotriazine fungicides" (FRAC code 35) include triazoxide.
(36) "Benzene-sulfonamide fungicides" (FRAC code 36) include flusulfamide.
(37) "Pyridazinone fungicides" (FRAC code 37) include diclomezine.
(38) "Thiophene-carboxamide fungicides" (FRAC code 38) are proposed to affect ATP production. Examples include silthiofam.
(39) "Pyrimidinamide fungicides" (FRAC code 39), also known as Complex I NADH oxidoreductase inhibitor fungicides inhibit electron transport in mitochondria and include pyrimidinamines such as diflumetorim, and pyrazole-5-carboxamides such as tolfenpyrad.
(40) "Carboxylic acid amide (CAA) fungicides" (FRAC code 40) inhibit cellulose synthase, which prevents growth and leads to death of the target fungus. Carboxylic acid amide fungicides include cinnamic acid amides, valinamide carbamates, carbamates and mandelic acid amides. The cinnamic acid amides include dimethomorph, flumorph and pyrimorph (3-(2-chloro-4-pyridinyl)-3-[4-(1,1-dimethylethyl)phenyl]-1-(4-morpholinyl)-2-propene-1-one). The valinamide carbamates include benthiavalicarb, benthiavalicarb-isopropyl, iprovalicarb, tolprocarb (2,2,2-trifluoroethyl *N*-[(1*S*)-2-methyl-1-[[(4-methylbenzoyl)amino]methyl]propyl]carbamate) valifenalate (methyl *N*-[(1-methylethoxy)carbonyl]-L-valyl-3-(4-chlorophenyl)-β-alaninate) (also known as valiphenal). The mandelic acid amides include mandipropamid, *N*-[2-[4-[[3-(4-chlorophenyl)-2-propyn-1-yl]oxy]-3-methoxyphenyl]ethyl]-3-methyl-2-[(methylsulfonyl)amino]butanamide and *N-*[2-[4-[[3-(4-chlorophenyl)-2-propyn-1-yl]oxy]-3-methoxyphenyl]ethyl]-3-methyl-2-[(ethylsulfonyl)amino]butanamide.
(41) "Tetracycline antibiotic fungicides" (FRAC code 41) inhibit growth of fungi by affecting protein synthesis. Examples include oxytetracycline.
(42) "Thiocarbamate fungicides" (FRAC code 42) include methasulfocarb.
(43) "Benzamide fungicides" (FRAC code 43) inhibit growth of fungi by delocalization of spectrin-like proteins. Examples include pyridinylmethyl benzamide fungicides such as fluopicolide.
(44) "Host plant defense induction fungicides" (FRAC code P) induce host plant defense mechanisms. Host plant defense induction fungicides include benzothiadiazoles, benzisothiazoles and thiadiazolecarboxamides. The benzothiadiazoles include acibenzolar-S-methyl. The benzisothiazoles include probenazole, The thiadiazolecarboxamides include tiadinil and isotianil.
(45) "Multi-site contact fungicides" inhibit fungal growth through multiple sites of action and have contact/preventive activity. This class of fungicides includes: (45.1) "copper fungicides" (FRAC code M1)", (45.2) "sulfur fungicides" (FRAC code M2), (45.3) "dithiocarbamate fungicides" (FRAC code M3), (45.4) "phthalimide fungicides" (FRAC code M4), (45.5) "chloronitrile fungicides" (FRAC code M5), (45.6) "sulfamide fungicides" (FRAC code M6), (45.7) "multi-site contact guanidine fungicides" (FRAC code M7), (45.8) "triazine fungicides" (FRAC code M8), (45.9) "quinone fungicides" (FRAC code M9), quinoxaline fungicides" (FRAC code M10) and "maleimide fungicides" (FRAC code M11).. "Copper fungicides" are inorganic compounds containing copper, typically in the copper(II) oxidation state; examples include copper oxychloride, copper sulfate and copper hydroxide, including compositions such as Bordeaux mixture (tribasic copper sulfate). "Sulfur fungicides" are inorganic chemicals containing rings or chains of sulfur atoms; examples include elemental sulfur. "Dithiocarbamate fungicides" contain a dithiocarbamate molecular moiety; examples include mancozeb, metiram, propineb, ferbam, maneb, thiram, zineb and ziram. "Phthalimide fungicides" contain a phthalimide molecular moiety; examples include folpet, captan and captafol. "Chloronitrile fungicides" contain an aromatic ring substituted with chloro and cyano; examples include chlorothalonil. "Sulfamide fungicides" include dichlofluanid and tolyfluanid. "Multi-site contact guanidine fungicides" include guazatine, iminoctadine albesilate and iminoctadine triacetate. "Triazine fungicides" include anilazine. "Quinone fungicides" include dithianon. "Quinoxaline fungicides" include quinomethionate (also known as chinomethionate). "Maleimide fungicides" include fluoroimide.
(46) "Fungicides other than fungicides of classes (1) through (45)" include certain fungicides whose mode of action may be unknown. These include: (46.1) "phenylacetamide fungicides" (FRAC code U6), (46.2) "arylphenylketone fungicides" (FRAC code U8) also known as "benzophenone fungicides", (46.3) "guanidine fungicides" (FRAC code U12), (46.4) "thiazolidine fungicides" (FRAC code U13), (46.5) "pyrimidinone-hydrazone fungicides" (FRAC code U14) and (46.6) "QₓI fungicides" (also known as "triazolopyrimidylamine fungicides") (FRAC code 45). The phenylacetamides include cyflufenamid and *N*-[[(cyclopropylmethoxy)amino][6-(difluoromethoxy)-2,3-difluorophenyl]-methylene]benzeneacetamide. The arylphenylketones include benzophenones such as metrafenone and benzoylpyridines such as pyriofenone (5-chloro-2-methoxy-4-methyl-3-pyridinyl)(2,3,4-trimethoxy-6-methylphenyl)methanone). The guanidines include dodine. The thiazolidines include flutianil ((2*Z*)-2-[[2-fluoro-5-(trifluoromethyl)phenyl]thio]-2-[3-(2-methoxyphenyl)-2-thiazolidinylidene]acetonitrile). The pyrimidinone-hydrazones include ferimzone.

The QₓI fungicides are now believed to inhibit Complex III mitochondrial respiration in fungi by affecting ubiquinone reductase at an unknown (Qₓ) site of the cytochrome *bc1* complex. Inhibiting mitochondrial respiration prevents normal fungal growth and development. QₓI fungicides include triazolopyrimidinylamines such as ametoctradin (5-ethyl-6-octyl[1,2,4]triazolo[1,5-*a*]pyrimidin-7-amine).

"Fungicides include (46.7) *N'*-[4-[[3-[(4-chlorophenyl)methyl]-1,2,4-thiadiazol-5-yl]oxy]-2,5-dimethylphenyl]-*N*-ethyl-*N*-methylmethanimidamide, which is believed to inhibit C24-methyl transferase involved in biosynthesis of sterols. The (46) class also includes (46.8) compounds that bind to oxysterol-binding protein as described in PCT Patent Publication WO 2013/009971, such as oxathiapiprolin (1-[4-[4-[5-(2,6-difluorophenyl)-4,5-dihydro-3-isoxazolyl]-2-thiazolyl]-1-piperidinyl]-2-[5-methyl-3-(trifluoromethyl)-1*H-*pyrazol-1-yl]ethanone) and its R-enantiomer. The (46) class further includes mitosis- and cell division-inhibiting fungicides besides those of the particular classes described above (e.g., 1, 10 and 22).

Class (46) (i.e. "Fungicides other than classes (1) through (45)") also includes bethoxazin, flumetover, neo-asozin (ferric methanearsonate), pyrrolnitrin, quinomethionate, tebufloquin, tolnifanide, (6-(1,1-dimethylethyl)-8-fluoro-2,3-dimethyl-4-quinolinyl acetate), 2-[[2-fluoro-5-(trifluoromethyl)phenyl]thio]-2-[3-(2-methoxyphenyl)-2-thiazolidinylidene]acetonitrile, 4-fluorophenyl *N*-[1-[[[1-(4-cyanophenyl)ethyl]sulfonyl]-methyl]propyl]carbamate, *N*-(4-chloro-2-nitrophenyl)-*N*-ethyl-4-methylbenzenesulfonamide, *N*-[[(cyclopropylmethoxy)amino][6-(difluoromethoxy)-2,3-difluorophenyl]methylene]-benzeneacetamide, *N'*-[4-[4-chloro-3-(trifluoromethyl)phenoxy]-2,5-dimethylphenyl]-*N-*ethyl-*N*-methyl-methanimidamide, 1,1-dimethylethyl *N*-[6-[[[(1 -methyl- 1*H*-tetrazol-5-yl)phenylmethylene]amino]oxy]methyl]-2-pyridinyl]carbamate, 3-butyn-1-yl *N*-[6-[[[[(1-methyl-1*H*-tetrazol-5-yl)phenylmethylene]amino]oxy]methyl]-2-pyridinyl]carbamate, 2,6-dimethyl-1*H*,5*H*-[1,4]dithiino[2,3-*c*:5,6-*c*']dipyrrole-1,3,5,7(2*H*,6*H*)-tetrone, 5-fluoro-2-[(4-methylphenyl)methoxy]-4-pyrimidinamine and 5 -fluoro-2-[(4-fluorophenyl)methoxy] -4-pyrimidinamine.

Additional fungicides other than fungicides listed above also include: 2-[(3-bromo-6-quinolinyl)oxy]-*N*-(1,1-dimethyl-2-butyn-1 -yl)-2-(methylthio)acetamide, 2-[(3-ethynyl-6-quinolinyl)oxy]-*N*-[1 -(hydroxymethyl)-1 -methyl-2-propyn-1 -yl]-2-(methylthio)acetamide, *N*-(1,1-dimethyl-2-butyn-1 -yl)-2- [(3 -ethynyl-6-quinolinyl)oxy] -2-(methylthio)acetamide, 2-[(3-bromo-8-methyl-6-quinolinyl)oxy]-*N*-(1,1-dimethyl-2-propyn-1-yl)-2-(methylthio)acetamide, 2-[(3-bromo-6-quinolinyl)oxy]-*N*-(1,1-dimethylethyl)butanamide, (2-chloro-6-fluorophenyl)methyl 2-[1-[2-[3,5-bis(difluoromethyl)-1*H*-pyrazol-1-yl]acetyl]-4-piperidinyl]-4-thiazolecarboxylate, (1*R*)-1,2,3,4-tetrahydro-1-naphthalenyl 2-[1-[2-[3,5-bis(difluoromethyl)-1*H-*pyrazol-1-yl]acetyl]-4-piperidinyl]-4-thiazolecarboxylate, 1-[4-[4-[5-[(2,6-difluorophenoxy)methyl]-4,5-dihydro-3-isoxazolyl]-2-thiazolyl]-1-piperdinyl]-2-[5-methyl-3-(trifluoromethyl)-1*H*-pyrazol-1-yl]ethanone, [[4-methoxy-2-[[[(3*S*,7*R*,8*R*,9*S*)-9-methyl-8-(2-methyl-1-oxopropoxy)-2,6-dioxo-7-(phenylmethyl)-1,5-dioxonan-3-yl]-amino] carbonyl] -3 -pyridinyl]oxy]methyl 2-methylpropanoate, (3*S*,6*S*,7*R*,8*R*)-3-[[[3 - (acetyloxy)-4-methoxy-2-pyridinyl]carbonyl]amino]-6-methyl-4,9-dioxo-8-(phenylmethyl)-1,5-dioxonan-7-yl 2-methylpropanoate, (3*S*,6*S*,7*R*,8*R*)-3-[[[3-[(acetyloxy)methoxy]-4-methoxy-2-pyridinyl]carbonyl]amino]-6-methyl-4,9-dioxo-8-(phenylmethyl)-1,5-dioxonan-7-yl 2-methylpropanoate, (3*S*,6*S*,7*R*,8*R*)-3-[[[4-methoxy-3-[[(2-methylpropoxy)carbonyl]-oxy]-2-pyridinyl]carbonyl]amino]-6-methyl-4,9-dioxo-8-(phenylmethyl)-1,5-dioxonan-7-yl 2-methylpropanoate, *N*-[[3-(1,3-benzodioxol-5-ylmethoxy)-4-methoxy-2-pyridinyl]-carbonyl]-*O*-[2,5-dideoxy-3-*O*-(2-methyl-1-oxopropyl)-2-(phenylmethyl)-L-arabinonoyl]-L-serine, (1→4')-lactone, 3-(difluoromethyl)-*N*-[4-fluoro-2-(1,1,2,3,3,3-hexafluoropropoxy)-phenyl]-1-methyl-1*H*-pyrazole-4-carboxamide, 3-(difluoromethyl)-1-methyl-*N*-[2-(1,1,2,2-tetrafluoroethoxy)phenyl]-1*H*-pyrazole-4-carboxamide, *rel*-1-[[(2*R*,3*S*)-3-(2-chlorophenyl)-2-(2,4-difluorophenyl)-2-oxiranyl]methyl]-1*H*-1,2,4-triazole, *rel*-2-[[(2*R*,3*S*)-3-(2-chlorophenyl)-2-(2,4-difluorophenyl)-2-oxiranyl]methyl]-1,2-dihydro-3*H*-1,2,4-triazole-3-thione, *rel*-1-[[(2*R*,3*S*)-3-(2-chlorophenyl)-2-(2,4-difluorophenyl)-2-oxiranyl]methyl]-5-(2-propen-1-ylthio)-1*H*-1,2,4-triazole, *rel*-1-[[(2*R*,3*S*)-3-(2-chlorophenyl)-2-(2,4-difluorophenyl)-2-oxiranyl]methyl]-1*H*-1,2,4-triazol-5-yl thiocyanate, α-[3-(4-chloro-2-fluorophenyl)-5-(2,4-difluorophenyl)-4-isoxazolyl]-3-pyridinemethanol, (α*S*)-[3-(4-chloro-2-fluorophenyl)-5-(2,4-difluorophenyl)-4-isoxazolyl]-3-pyridinemethanol, (α*R*)-[3-(4-chloro-2-fluorophenyl)-5-(2,4-difluorophenyl)-4-isoxazolyl]-3-pyridinemethanol, 3-[2-[3-(4-chloro-2-fluorophenyl)-5-(2,4-difluorophenyl)-4-isoxazolyl]-2-oxiranyl]pyridine and 2-ethyl-3,7-dimethyl-6-[4-(trifluoromethoxy)phenoxy]-4-quinolinyl methyl carbonate (flometoquin).

In addition to or as an alternative to the fungicidal compounds disclosed as components (1) through (46), component (b) can comprise one or more "Microbial fungicides" (FRAC code 44). "Microbial fungicides" typically disrupt fungal pathogen cell membranes. Microbial fungicides include Bacillus species such as *Bacillus* species such as *Bacillus amyloliquefaciens* strains QST 713, FZB24, MB1600, D747 and the fungicidal lipopeptides which they produce.

Therefore of note is a mixture (i.e. composition) comprising a compound of Formula 1 and at least one fungicidal compound selected from the group consisting of the aforedescribed classes (1) through (46). Also of note is a composition comprising said mixture (in fungicidally effective amount) and further comprising at least one additional component selected from the group consisting of surfactants, solid diluents and liquid diluents. A composition comprising a compound of Formula 1 and one or more of the fungicidal compounds described above can provide improved control (i.e. prevention and/or cure) of plant disease from synergic contributions of the components. The improved plant disease control may be manifest by a broader spectrum or longer duration of plant disease control, or retardation of resistance development. The contributions of components may be complementarily additive or even greater than additive through synergistic interaction.

Examples of component (b) fungicides include acibenzolar-S-methyl, aldimorph, ametoctradin, amisulbrom, anilazine, azaconazole, azoxystrobin, benalaxyl (including benalaxyl-M), benodanil, benomyl, benthiavalicarb (including benthiavalicarb-isopropyl), benzovindiflupyr, bethoxazin, binapacryl, biphenyl, bitertanol, bixafen, blasticidin-S, boscalid, bromuconazole, bupirimate, buthiobate, captafol, captan, carbendazim, carboxin, carpropamid, chloroneb, chlorothalonil, chlozolinate, clotrimazole, copper hydroxide, copper oxychloride, copper sulfate, cyazofamid, cyflufenamid, cymoxanil., cyproconazole, cyprodinil, dichlofluanid, diclocymet, diclomezine, dicloran, diethofencarb, difenoconazole, diflumetorim, dimethirimol, dimethomorph, dimoxystrobin, diniconazole (including diniconazole-M), dinocap, dithianon, dithiolanes, dodemorph, dodine, econazole, edifenphos, enoxastrobin, epoxiconazole, etaconazole, ethaboxam, ethirimol, etridiazole, famoxadone, fenarimol, fenamidone, fenbuconazole, fenfuram, fenhexamid, fenoxanil, fenpiclonil, fenpropidin, fenpropimorph, fenpyrazamine, fentin acetate, fentin chloride, fentin hydroxide, ferbam, ferimzone, fluazinam, fludioxonil, flumetover, flumorph, fluopicolide, fluopyram, fluoromide, fluoxastrobin, fluquinconazole, flusilazole, flusulfamide, flutianil, flutolanil, flutriafol, fluxapyroxad, folpet, fosetyl-aluminum, fuberidazole, furalaxyl, furametpyr, guazatine, hexaconazole, hymexazole, imazalil, imibenconazole, iminoctadine albesilate, iminoctadine triacetate, iodocarb, ipconazole, iprobenfos, iprodione, iprovalicarb, isoconazole, isofetamid, isoprothiolane, isopyrazam, isotianil, kasugamycin, kresoxim-methyl, mancozeb, mandipropamid, maneb, mefenoxam, mepanipyrim, mepronil, meptyldinocap, metalaxyl (including metalaxyl-M/mefenoxam), metconazole, methasulfocarb, metiram, metominostrobin, metrafenone, miconazole, myclobutanil, naftifine, neo-asozin, nuarimol, octhilinone, ofurace, orysastrobin, oxadixyl, oxolinic acid, oxpoconazole, oxycarboxin, oxytetracycline, pefurazoate, penconazole, pencycuron, penflufen, penthiopyrad, phosphorous acid (including salts thereof), phthalide, picobenzamid, picoxystrobin, piperalin, polyoxin, probenazole, prochloraz, procymidone, propamacarb, propiconazole, propineb, proquinazid, prothiocarb, prothioconazole, pyraclostrobin, pyrametostrobin, pyraoxystrobin, pyrazophos, pyribencarb, pyributicarb, pyrifenox, pyrimethanil, pyriofenone, pyroquilon, pyrrolnitrin, quinconazole, quinomethionate, quinoxyfen, quintozene, sedaxane, silthiofam, simeconazole, spiroxamine, streptomycin, sulfur, tebuconazole, tebufloquin, tebufloquin, teclofthalam, tecnazene, terbinafine, tetraconazole, thiabendazole, thifluzamide, thiophanate, thiophanate-methyl, thiram, tiadinil, tolclofos-methyl, tolprocarb, tolyfluanid, triadimefon, triadimenol, triarimol, triticonazole, triazoxide, tribasic copper sulfate, tricyclazole, tridemorph, trifloxystrobin, triflumizole, triforine, trimorphamide, uniconazole, validamycin, valifenalate, valiphenal, vinclozolin, zineb, ziram, zoxamide, 3-[5-(4-chlorophenyl)-2,3-dimethyl-3-isoxazolidinyl]pyridine, 4-fluorophenyl *N*-[1-[[[1 -(4-cyanophenyl)ethyl]sulfonyl]methyl]-propyl]carbamate, 5-chloro-6-(2,4,6-trifluorophenyl)-7-(4-methylpiperidin-1-yl)[1 ,2,4]-triazolo[1,5-a]pyrimidine, *N*-(4-chloro-2-nitrophenyl)-*N*-ethyl-4-methylbenzenesulfonamide, *N*-[[(cyclopropylmethoxy)amino][6-(difluoromethoxy)-2,3-difluorophenyl]methylene]-benzeneacetamide, *N*-[2-(1*S*,2*R*)-[1,1'-bicyclopropyl]-2-ylphenyl]-3-(difluoromethyl)-1-methyl-1*H*-pyrazole-4-carboxamide, *N*-[2-[4-[[3-(4-chlorophenyl)-2-propyn-1-yl]oxy]-3-methoxyphenyl]ethyl]-3-methyl-2-[(methylsulfonyl)amino]butanamide, *N*-[2-[4-[[3-(4-chlorophenyl)-2-propyn-1-yl]oxy] -3-methoxyphenyl]ethyl]-3-methyl-2-[(ethylsulfonyl)-amino]butanamide, *N*-[2-[4-[[3-(4-chlorophenyl)-2-propyn-1-yl]oxy]-3-methoxyphenyl]-ethyl]-3-methyl-2-[(methylsulfonyl)amino]butanamide, *N*-[2-[4-[[3-(4-chlorophenyl)-2-propyn-1-yl]oxy]-3-methoxyphenyl]ethyl]-3-methyl-2-[(ethylsulfonyl)amino]butanamide, *N*-[4-[[3-[(4-chlorophenyl)methyl]-1,2,4-thiadiazol-5-yl]oxy]-2,5-dimethylphenyl]-*N-*ethyl-*N*-methyl-methanimidamide, *N'*-[4-[4-chloro-3-(trifluoromethyl)-phenoxy]-2,5-dimethylphenyl]-*N*-ethyl-*N*-methylmethanimidamide, α-[methoxyimino]-*N*-methyl-2-[[[1-[3-(trifluoromethyl)phenyl]ethoxy]imino]methyl]benzeneacetamide, 2-butoxy-6-iodo-3-propyl-4*H*-1-benzopyran-4-one, 2-[[[3-(2,6-dichlorophenyl)-1-methyl-2-propen-1-ylidene]amino]-oxy]methyl]-α-(methoxyimino)-*N*-methylbenzeneacetamide (fenaminstrobin), pentyl *N*-[4-[[[[(1-methyl-1*H*-tetrazol-5-yl)phenylmethylene]amino]oxy]methyl]-2-thiazolyl]carbamate, 2-[(3-bromo-6-quinolinyl)oxy]-*N*-(1,1-dimethyl-2-butyn-1-yl)-2-(methylthio)acetamide, 2-[(3-ethynyl-6-quinolinyl)oxy]-*N*-[1-(hydroxymethyl)-1-methyl-2-propyn-1-yl]-2-(methylthio)acetamide and *N*-(1,1-dimethyl-2-butyn-1-yl)-2-[(3-ethynyl-6-quinolinyl)oxy]-2-(methylthio)acetamide.

Additional examples of component (b) fungicides include 1-[4-[4-[5-(2,6-difluorophenyl)-4,5-dihydro-3-isoxazolyl]-2-thiazolyl]-1-piperidinyl]-2-[5-methyl-3-(trifluoromethyl)-1*H*-pyrazol-1-yl]ethanone (oxathiapiprolin), 1-[4-[4-[5*R*-(2,6-difluorophenyl)-4,5-dihydro-3-isoxazolyl]-2-thiazolyl]-1-piperidinyl]-2-[5-methyl-3-(trifluoromethyl)-1*H-*pyrazol-1-yl]ethanone, 1-[4-[4-[5-[(2,6-difluorophenoxy)methyl]-4,5-dihydro-3-isoxazolyl]-2-thiazolyl]-1-piperdinyl-2-[5-methyl-3-(trifluoromethyl)-1*H-*pyrazol-1-yl]ethanone, (2-chloro-6-fluorophenyl)methyl 2-[1-[2-[3,5-bis(difluoromethyl)-1*H*-pyrazol-1-yl]acetyl]-4-piperidinyl]-4-thiazolecarboxylate, (1*R*)-1,2,3,4-tetrahydro-1-naphthalenyl 2-[1-[2-[3,5-bis(difluoromethyl)-1*H*-pyrazol-1-yl]acetyl]-4-piperidinyl]-4-thiazolecarboxylate, [[4-methoxy-2-[[[(3*S*,7*R*,8*R*,9*S*)-9-methyl-8-(2-methyl-1-oxopropoxy)-2,6-dioxo-7-(phenylmethyl)-1,5-dioxonan-3-yl]amino]carbonyl]-3-pyridinyl]oxy]methyl 2-methylpropanoate, (3*S*,6*S*,7*R*,8*R*)-3-[[[3-(acetyloxy)-4-methoxy-2-pyridinyl]carbonyl]-amino]-6-methyl-4,9-dioxo-8-(phenylmethyl)-1,5-dioxonan-7-yl 2-methylpropanoate, (3*S*,6*S*,7*R*,8*R*)-3-[[[3-[(acetyloxy)methoxy]-4-methoxy-2-pyridinyl]carbonyl]amino]-6-methyl-4,9-dioxo-8-(phenylmethyl)-1,5-dioxonan-7-yl 2-methylpropanoate, (3*S*,6*S*,7*R*,8*R*)-3-[[[4-methoxy-3-[[(2-methylpropoxy)carbonyl]oxy]-2-pyridinyl]carbonyl]amino]-6-methyl-4,9-dioxo-8-(phenylmethyl)-1,5-dioxonan-7-yl 2-methylpropanoate, *N*-[[3-(1,3-benzodioxol-5-ylmethoxy)-4-methoxy-2-pyridinyl]carbonyl]-*O*-[2,5-dideoxy-3-*O*-(2-methyl-1-oxopropyl)-2-(phenylmethyl)-L-arabinonoyl]-L-serine, (1→4')-lactone, 5-fluoro-2-[(4-methylphenyl)methoxy]-4-pyrimidinamine, 5-fluoro-2-[(4-fluorophenyl)methoxy]-4-pyrimidinamine, 5,8-difluoro-*N*-[2-[3-methoxy-4-[[4-(trifluoromethyl)-2-pyridinyl]oxy]-phenyl]ethyl]-4-quinazolinamine, pentyl [6-[[[(Z)-[(1-methyl-1*H*-tetrazol-5-yl)phenylmethylene]amino]oxy]methyl]-2-pyridinyl]carbamate, 1,1-dimethylethyl *N*-[6-[[[(Z)-[(1-methyl-1*H*-tetrazol-5-yl)phenylmethylene]amino]oxy]methyl]-2-pyridinyl]carbamate, 3-butyn-1-yl *N*-[6-[[[(Z)-[(1-methyl-1*H*-tetrazol-5-yl)phenylmethylene]amino]oxy]methyl]-2-pyridinyl]carbamate, *N*-(3',4'-difluoro[1,1'-biphenyl]-2-yl)-3-(trifluoromethyl)-2-pyrazinecarboxamide, *N*-[2-(2,4-dichlorophenyl)-2-methoxy-1 -methylethyl] -3 -(difluoromethyl)-1 -methyl-1*H*-pyrazole-4-carboxamide, 3-(difluoromethyl)-*N*-[4-fluoro-2-(1,1,2,3,3,3-hexafluoropropoxy)phenyl]-1-methyl-1*H-*pyrazole-4-carboxamide, 3-(difluoromethyl)-1-methyl*-N*-[2-(1,1,2,2-tetrafluoroethoxy)-phenyl]-1*H*-pyrazole-4-carboxamide, (α*R*)-2-[(2,5-dimethylphenoxy)methyl]-α-methoxy-*N-*methylbenzeneacetamide, 2,6-dimethyl-1*H*,5*H*-[1,4]dithiino[2,3-*c*:5,6-*c*']dipyrrole-1,3,5,7(2*H*,6*H*)-tetrone, isofetamid, tolprocarb, 1-[[(2*S*,3*R*)-3-(2-chlorophenyl)-2-(2,4-difluorophenyl)-2-oxiranyl]methyl]-1*H*-1,2,4-triazole, 2-[[(2*S*,3*R*)-3-(2-chlorophenyl)-2-(2,4-difluorophenyl)-2-oxiranyl]methyl]-1,2-dihydro-3*H*-1,2,4-triazole-3-thione, 1-[[(2*S*,3*R*)-3-(2-chlorophenyl)-2-(2,4-difluorophenyl)-2-oxiranyl]methyl]-5-(2-propen-1-ylthio)-1*H*-1 ,2,4-triazole, α-[3-(4-chloro-2-fluorophenyl)-5-(2,4-difluorophenyl)-4-isoxazolyl]-3-pyridinemethanol, (α*S*)-[3-(4-chloro-2-fluorophenyl)-5-(2,4-difluorophenyl)-4-isoxazolyl]-3-pyridinemethanol, (α*R*)-[3-(4-chloro-2-fluorophenyl)-5-(2,4-difluorophenyl)-4-isoxazolyl]-3-pyridinemethanol, 3-[2-[3-(4-chloro-2-fluorophenyl)-5-(2,4-difluorophenyl)-4-isoxazolyl]-2-oxiranyl]pyridine and 2-ethyl-3,7-dimethyl-6-[4-(trifluoromethoxy)-phenoxy]-4-quinolinyl methyl carbonate.

Examples of other biologically active compounds or agents with which compounds of this invention can be formulated are: insecticides such as abamectin, acephate, acetamiprid, acrinathrin, amidoflumet (S-1955), avermectin, azadirachtin, azinphos-methyl, bifenthrin, bifenazate, buprofezin, carbofuran, cartap, chlorantraniliprole, chlorfenapyr, chlorfluazuron, chlorpyrifos, chlorpyrifos-methyl, chromafenozide, clothianidin, cyantraniliprole (3-bromo-1-(3-chloro-2-pyridinyl)-*N*-[4-cyano-2-methyl-6-[(methylamino)carbonyl]phenyl]-1*H-*pyrazole-5-carboxamide), cyflumetofen, cyfluthrin, beta-cyfluthrin, cyhalothrin, lambda-cyhalothrin, cypermethrin, cyromazine, deltamethrin, diafenthiuron, diazinon, dieldrin, diflubenzuron, dimefluthrin, dimethoate, dinotefuran, diofenolan, emamectin, endosulfan, esfenvalerate, ethiprole, fenothiocarb, fenoxycarb, fenpropathrin, fenvalerate, fipronil, flonicamid, flubendiamide, flucythrinate, tau-fluvalinate, flufenerim (UR-50701), flufenoxuron, fonophos, halofenozide, hexaflumuron, hydramethylnon, imidacloprid, indoxacarb, isofenphos, lufenuron, malathion, meperfluthrin, metaflumizone, metaldehyde, methamidophos, methidathion, methomyl, methoprene, methoxychlor, methoxyfenozide, metofluthrin, milbemycin oxime, monocrotophos, nicotine, nitenpyram, nithiazine, novaluron, noviflumuron (XDE-007), oxamyl, parathion, parathion-methyl, permethrin, phorate, phosalone, phosmet, phosphamidon, pirimicarb, profenofos, profluthrin, pymetrozine, pyrafluprole, pyrethrin, pyridalyl, pyrifluquinazon, pyriprole, pyriproxyfen, rotenone, ryanodine, spinetoram, spinosad, spirodiclofen, spiromesifen (BSN 2060), spirotetramat, sulfoxaflor, sulprofos, tebufenozide, teflubenzuron, tefluthrin, terbufos, tetrachlorvinphos, tetramethylfluthrin, thiacloprid, thiamethoxam, thiodicarb, thiosultap-sodium, tolfenpyrad, tralomethrin, triazamate, trichlorfon and triflumuron; and biological agents including entomopathogenic bacteria, such as *Bacillus thuringiensis* subsp. *aizawai, Bacillus thuringiensis* subsp. *kurstaki,* and the encapsulated delta-endotoxins of *Bacillus thuringiensis* (e.g., Cellcap, MPV, MPVII); entomopathogenic fungi, such as green muscardine fungus; and entomopathogenic virus including baculovirus, nucleopolyhedro virus (NPV) such as HzNPV, AfNPV; and granulosis virus (GV) such as CpGV.

Compounds of this invention and compositions thereof can be applied to plants genetically transformed to express proteins toxic to invertebrate pests (such as *Bacillus* *thuringiensis* delta-endotoxins). The effect of the exogenously applied fungicidal compounds of this invention may be synergistic with the expressed toxin proteins.

General references for agricultural protectants (i.e. insecticides, fungicides, nematocides, acaricides, herbicides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Farnham, Surrey, U.K., 2001.

In certain instances, combinations of a mixture of components (a) and (b) fungicidal compounds with invertebrate pest control compounds or agents (i.e. as component (c) biologically active ingredients) can result in a greater-than-additive (i.e. synergistic) effect. Reducing the quantity of active ingredients released in the environment while ensuring effective pest control is always desirable. When synergism of invertebrate pest control active ingredients occurs at application rates giving agronomically satisfactory levels of invertebrate pest control, such combinations can be advantageous for reducing crop production cost and decreasing environmental load. Synergism can also result in increased plant disease control or protection.

In the fungicidal compositions of the present invention, component (a) (i.e. at least one compound selected from compounds of Formula **1,** *N*-oxides, and salts thereof) and component (b) are present in fungicidally effective amounts. The weight ratio of component (a) to component (b) (i.e. one or more additional fungicidal compounds) is generally between about 1:3000 to about 3000: 1, more typically between about 1:500 and about 500:1. Of note are compositions where in the weight ratio of component (a) to component (b) is from about 125:1 to about 1:125. With many fungicidal compounds of component (b), these compositions are particularly effective for controlling plant diseases caused by fungal plant pathogens. Of particular note are compositions wherein the weight ratio of component (a) to component (b) is from about 25:1 to about 1:25, or from about 5:1 to about 1:5. One skilled in the art can easily determine through simple experimentation the weight ratios and application rates of fungicidal compounds necessary for the desired spectrum of fungicidal protection and control.

Table A1 lists specific combinations of a Component (b) compound with Compound 18 as Component (a) illustrative of the mixtures, compositions and methods of the present invention. (Compound numbers refer to compounds in Index Tables A-G). The second column of Table A1 lists the specific Component (b) compound (e.g., "1-[4-[4-[5-(2,6-difluorophenyl)-4,5-dihydro-3-isoxazolyl]-2-thiazolyl]-1-piperidinyl]-2-[5-methyl-3-(trifluoromethyl)-1*H*-pyrazol-1-yl]ethanone " in the first line). The third, fourth and fifth columns of Table A1 lists ranges of weight ratios for rates at which the Component (a) compound is typically applied to a field-grown crop relative to Component (b). Thus, for example, the first line of Table A1 specifically discloses the combination of Compound 1 with Component (b) is typically applied in a weight ratio of Compound 1 to Component (b) of between 400:1 and 1:1. The remaining lines of Table A1 are to be construed similarly.

**Table A1**

| Component (a) | Component (b) | Typical Weight Ratio | More Typical Weight Ratio | Most Typical Weight Ratio |
|---|---|---|---|---|
| Compound 18 | oxathiapiprolin (1-[4-[4-[5-(2,6-difluorophenyl)-4,5-dihydro-3-isoxazolyl]-2-thiazolyl]-1-piperidinyl]-2-[5-methyl-3-(trifluoromethyl)-1*H*-pyrazol-1-yl]ethanone) | 1:1 to 1:90 | 1:2 to 1:30 | 1:2 to 1:18 |
| Compound 18 | 1-[4-[4-[5*R*-(2,6-difluorophenyl)-4,5-dihydro-3-isoxazolyl]-2-thiazolyl]-1-piperidinyl]-2-[5-methyl-3-(trifluoromethyl)-1*H*-pyrazol-1-yl]-ethanone | 1:1 to 1:90 | 1:2 to 1:30 | 1:2 to 1:18 |
| Compound 18 | 1-[4-[4-[5-[(2,6-difluorophenoxy)methyl]-4,5-dihydro-3-isoxazolyl]-2-thiazolyl]-1-piperdinyl-2-[5-methyl-3-(trifluoromethyl)-1*H*-pyrazol-1-yl]ethanone | 1:1 to 1:90 | 1:2 to 1:30 | 1:2 to 1:18 |
| Compound 18 | (2-chloro-6-fluorophenyl)methyl 2-[1-[2-[3,5-bis(difluoromethyl)-1*H*-pyrazol-1-yl]acetyl]-4-piperidinyl]-4-thiazolecarboxylate | 1:1 to 1:90 | 1:2 to 1:30 | 1:2 to 1:18 |
| Compound 18 | (1*R*)-1,2,3,4-tetrahydro-1-naphthalenyl 2-[1-[2-[3,5-bis(difluoromethyl)-1*H*-pyrazol-1-yl]acetyl]-4-piperidinyl]-4-thiazolecarboxylate | 1:1 to 1:90 | 1:2 to 1:30 | 1:2 to 1:18 |
| Compound 18 | [[4-methoxy-2-[[[(3*S*,7*R*,8*R*,9*S*)-9-methyl-8-(2-methyl-1-oxopropoxy)-2,6-dioxo-7-(phenylmethyl)-1,5-dioxonan-3-yl]amino]-carbonyl]-3-pyridinyl]oxy]methyl 2-methylpropanoate ( | 90:1 to 1:4 | 30:1 to 1:2 | 15:1 to 3:1 |
| Compound 18 | (3*S*,6*S*,7*R*,8*R*)-3-[[[3-(acetyloxy)-4-methoxy-2-pyridinyl]carbonyl]amino]-6-methyl-4,9-dioxo-8-(phenylmethyl)-1,5-dioxonan-7-yl 2-methylpropanoate | 90:1 to 1:4 | 30:1 to 1:2 | 15:1 to 3:1 |
| Compound 18 | (3*S*,6*S*,7*R*,8*R*)-3-[[[3-[(acetyloxy)methoxy]-4-methoxy-2-pyridinyl]carbonyl]amino]-6-methyl-4,9-dioxo-8-(phenylmethyl)-1,5-dioxonan-7-yl 2-methylpropanoate | 90:1 to 1:4 | 30:1 to 1:2 | 15:1 to 3:1 |
| Compound 18 | (3*S*,6*S*,7*R*,8*R*)-3-[[[4-methoxy-3-[[(2-methyl-propoxy)carbonyl]oxy]-2-pyridinyl]-carbonyl]amino]-6-methyl-4,9-dioxo-8-(phenylmethyl)-1,5-dioxonan-7-yl 2-methylpropanoate | 90:1 to 1:4 | 30:1 to 1:2 | 15:1 to 3:1 |
| Compound 18 | *N*-[[3-(1,3-benzodioxol-5-ylmethoxy)-4-methoxy-2-pyridinyl]carbonyl]-*O*-[2,5-dideoxy-3-*O*-(2-methyl-1-oxopropyl)-2-(phenylmethyl)-L-arabinonoyl]-L-serine, (1→4')-lactone | 90:1 to 1:4 | 30:1 to 1:2 | 15:1 to 3:1 |
| Compound 18 | 5-fluoro-2-[(4-methylphenyl)methoxy]-4-pyrimidinamine | 20:1 to 1:20 | 5:1 to 1:5 | 3:1 to 1:3 |
| Compound 18 | 5-fluoro-2-[(4-fluorophenyl)methoxy]-4-pyrimidinamine | 20:1 to 1:20 | 5:1 to 1:5 | 3:1 to 1:3 |
| Compound 18 | 5,8-difluoro-*N*-[2-[3-methoxy-4-[[4-(trifluoromethyl)-2-pyridinyl]oxy]phenyl]-ethyl]-4-quinazolinamine | 40:1 to 1:10 | 10:1 to 1:3 | 5:1 to 1:2 |
| Compound 18 | pentyl [6-[[[(*Z*)-[(1-methyl-1*H*-tetrazol-5-yl)phenylmethylene]amino]oxy]methyl]-2-pyridinyl] carbamate | 40:1 to 1:10 | 10:1 to 1:3 | 5:1 to 1:2 |
| Compound 18 | 1,1-dimethylethyl *N*-[6-[[[(*Z*)-[(1-methyl-1*H-*tetrazol-5-yl)phenylmethylene]amino]oxy]-methyl]-2-pyridinyl] carbamate | 40:1 to 1:10 | 10:1 to 1:3 | 5:1 to 1:2 |
| Compound 18 | 3-butyn-1-yl *N*-[6-[[[(*Z*)-[(1-methyl-1*H-*tetrazol-5-yl)phenylmethylene]amino]oxy]-methyl]-2-pyridinyl]carbamate | 40:1 to 1:10 | 10:1 to 1:3 | 5:1 to 1:2 |
| Compound 18 | *N*-(3',4'-difluoro[1,1'-biphenyl]-2-yl)-3-(trifluoromethyl)-2-pyrazinecarboxamide | 20:1 to 1:20 | 5:1 to 1:5 | 3:1 to 1:3 |
| Compound 18 | *N*-[2-(2,4-dichlorophenyl)-2-methoxy-1-methylethyl]-3-(difluoromethyl)-1-methyl-1*H*-pyrazole-4-carboxamide | 20:1 to 1:20 | 5:1 to 1:5 | 3:1 to 1:3 |
| Compound 18 | 3-(difluoromethyl)-*N*-[4-fluoro-2-(1,1,2,3,3,3-hexafluoropropoxy)phenyl]-1-methyl-1*H-*pyrazole-4-carboxamide | 20:1 to 1:20 | 5:1 to 1:5 | 3:1 to 1:3 |
| Compound 18 | 3-(difluoromethyl)-1-methyl-*N*-[2-(1,1,2,2-tetrafluoroethoxy)phenyl]-1*H*-pyrazole-4-carboxamide | 20:1 to 1:20 | 5:1 to 1:5 | 3:1 to 1:3 |
| Compound 18 | isofetamid | 20:1 to 1:20 | 5:1 to 1:5 | 3:1 to 1:3 |
| Compound 18 | tolprocarb | 20:1 to 1:20 | 5:1 to 1:5 | 3:1 to 1:3 |
| Compound 18 | (α*R*)-2-[(2,5-dimethylphenoxy)methyl]-α-methoxy-*N*-methylbenzeneacetamide | 20:1 to 1:20 | 5:1 to 1:5 | 3:1 to 1:3 |
| Compound 18 | 2,6-dimethyl-1*H*,5*H-*[1,4]dithiino[2,3-c:5,6-c']dipyrrole-1,3,5,7(2*H*,6*H*)-tetrone | 1:1 to 1:400 | 1:4 to 1:100 | 1:8 to 1:50 |
| Compound 18 | 1-[[(2*S*,3*R*)-3-(2-chlorophenyl)-2-(2,4-difluorophenyl)-2-oxiranyl]methyl]-1*H*-1,2,4-triazole | 36:1 to 1:30 | 12:1 to 1:10 | 6:1 to 1:4 |
| Compound 18 | 2-[[(2*S*,3*R*)-3-(2-chlorophenyl)-2-(2,4-difluorophenyl)-2-oxiranyl]methyl]-1,2-dihydro-3*H*-1,2,4-triazole-3-thione | 36:1 to 1:30 | 12:1 to 1:10 | 6:1 to 1:4 |
| Compound 18 | 1-[[(2*S*,3*R*)-3-(2-chlorophenyl)-2-(2,4-difluorophenyl)-2-oxiranyl]methyl]-5-(2-propen-1-ylthio)-1*H-*1,2,4-triazole | 36:1 to 1:30 | 12:1 to 1:10 | 6:1 to 1:4 |
| Compound 18 | α-[3-(4-chloro-2-fluorophenyl)-5-(2,4-difluorophenyl)-4-isoxazolyl]-3-pyridinemethanol | 36:1 to 1:30 | 12:1 to 1:10 | 6:1 to 1:4 |
| Compound 18 | (α*S*)-[3-(4-chloro-2-fluorophenyl)-5-(2,4-difluorophenyl)-4-isoxazolyl]-3-pyridinemethanol | 36:1 to 1:30 | 12:1 to 1:10 | 6:1 to 1:4 |
| Compound 18 | (α*R*)-[3-(4-chloro-2-fluorophenyl)-5-(2,4-difluorophenyl)-4-isoxazolyl]-3-pyridinemethanol | 36:1 to 1:30 | 12:1 to 1:10 | 6:1 to 1:4 |
| Compound 18 | 3-[2-[3-(4-chloro-2-fluorophenyl)-5-(2,4-difluorophenyl)-4-isoxazolyl]-2-oxiranyl]pyridine | 36:1 to 1:30 | 12:1 to 1:10 | 6:1 to 1:4 |
| Compound 18 | 2-ethyl-3,7-dimethyl-6-[4-(trifluoromethoxy)phenoxy]-4-quinolinyl methyl carbonate | 36:1 to 1:30 | 12:1 to 1:10 | 6:1 to 1:4 |

Tables A2 through A29 are each constructed the same as Table A1 above except that entries below the "Component (a)" column heading are replaced with the respective Component (a) Column Entry shown below. Thus, for example, in Table A2 the entries below the "Component (a)" column heading all recite "Compound 10", and the first line below the column headings in Table A2 specifically discloses combination of Compound 10 with 1-[4-[4-[5-(2,6-difluorophenyl)-4,5-dihydro-3-isoxazolyl]-2-thiazolyl]-1-piperidinyl]-2-[5-methyl-3-(trifluoromethyl)-1*H*-pyrazol-1-yl]ethanone. Tables A3 through A29 are constructed similarly.

| Table Number | Component (a) Column Entry | Table Number | Component (a) Column Entry |
|---|---|---|---|
| A2 | Compound 10 | A16 | Compound 97 |
| A3 | Compound 20 | A17 | Compound 136 |
| A4 | Compound 11 | A18 | Compound 151 |
| A5 | Compound 17 | A19 | Compound 118 |
| A6 | Compound 23 | A20 | Compound 152 |
| A7 | Compound 28 | A21 | Compound 153 |
| A8 | Compound 8 | A22 | Compound 123 |
| A9 | Compound 31 | A23 | Compound 144 |
| A10 | Compound 66 | A24 | Compound 145 |
| A11 | Compound 38 | A25 | Compound 146 |
| A12 | Compound 91 | A26 | Compound 147 |
| A13 | Compound 46 | A27 | Compound 148 |
| A14 | Compound 115 | A28 | Compound 149 |
| A15 | Compound 116 | A29 | Compound 150 |

Table B1 also lists specific combinations of a Component (b) compound with Compound 18 as Component (a) illustrative of the mixtures, compositions and methods of the present invention. (Compound numbers refer to compounds in Index Tables A-G). The second column of Table B1 lists the specific Component (b) compound (e.g., "acibenzolar-S-methyl" in the first line). The third, fourth and fifth columns of Table B1 lists ranges of weight ratios for rates at which the Component (a) compound is typically applied to a field-grown crop relative to Component (b). Thus, for example, the first line of Table B1 specifically discloses the combination of Compound 18 with acibenzolar-S-methyl is typically applied in a weight ratio of between 2:1 and 1:180. The remaining lines of Table B1 are to be construed similarly.

**Table B1**

| Component (a) | Component (b) | Typical Weight Ratio | More Typical Weight Ratio | Most Typical Weight Ratio |
|---|---|---|---|---|
| Compound 18 | acibenzolar-*S*-methyl | 2:1 to 1:180 | 1:1 to 1:60 | 1:1 to 1:18 |
| Compound 18 | aldimorph | 30:1 to 1:3 | 10:1 to 1:1 | 7:1 to 1:1 |
| Compound 18 | ametoctradin | 9:1 to 1:18 | 3:1 to 1:6 | 3:1 to 1:3 |
| Compound 18 | amisulbrom | 6:1 to 1:18 | 2:1 to 1:6 | 1:1 to 1:6 |
| Compound 18 | anilazine | 90:1 to 2:1 | 30:1 to 4:1 | 22:1 to 4:1 |
| Compound 18 | azaconazole | 7:1 to 1:18 | 2:1 to 1:6 | 2:1 to 1:4 |
| Compound 18 | azoxystrobin | 9:1 to 1:12 | 3:1 to 1:4 | 3:1 to 1:3 |
| Compound 18 | benalaxyl | 4:1 to 1:18 | 1:1 to 1:6 | 1:1 to 1:6 |
| Compound 18 | benalaxyl-M | 4:1 to 1:36 | 1:1 to 1:12 | 1:1 to 1:8 |
| Compound 18 | benodanil | 18:1 to 1:6 | 6:1 to 1:2 | 4:1 to 1:2 |
| Compound 18 | benomyl | 45:1 to 1:4 | 15:1 to 1:1 | 11:1 to 1:1 |
| Compound 18 | benthiavalicarb or benthiavalicarb-isopropyl | 2:1 to 1:36 | 1:1 to 1:12 | 1:1 to 1:12 |
| Compound 18 | bethoxazin | 150:1 to 1:36 | 50:1 to 1:12 | 15:1 to 2:1 |
| Compound 18 | binapacryl | 150:1 to 1:36 | 50:1 to 1:12 | 15:1 to 2:1 |
| Compound 18 | biphenyl | 150:1 to 1:36 | 50:1 to 1:12 | 15:1 to 2:1 |
| Compound 18 | bitertanol | 15:1 to 1:5 | 5:1 to 1:2 | 3:1 to 1:2 |
| Compound 18 | bixafen | 12:1 to 1:9 | 4:1 to 1:3 | 2:1 to 1:3 |
| Compound 18 | blasticidin-S | 3:1 to 1:90 | 1:1 to 1:30 | 1:4 to 1:30 |
| Compound 18 | boscalid | 18:1 to 1:6 | 6:1 to 1:2 | 4:1 to 1:2 |
| Compound 18 | bromuconazole | 15:1 to 1:9 | 5:1 to 1:3 | 3:1 to 1:3 |
| Compound 18 | bupirimate | 3:1 to 1:90 | 1:1 to 1:30 | 1:3 to 1:30 |
| Compound 18 | captafol | 90:1 to 1:4 | 30:1 to 1:2 | 15:1 to 2:1 |
| Compound 18 | captan | 90:1 to 1:4 | 30:1 to 1:2 | 15:1 to 2:1 |
| Compound 18 | carbendazim | 45:1 to 1:4 | 15:1 to 1:2 | 11:1 to 2:1 |
| Compound 18 | carboxin | 18:1 to 1:6 | 6:1 to 1:2 | 4:1 to 1:2 |
| Compound 18 | carpropamid | 15:1 to 1:9 | 5:1 to 1:3 | 3:1 to 1:3 |
| Compound 18 | chloroneb | 300:1 to 2:1 | 100:1 to 4:1 | 100:1 to 14:1 |
| Compound 18 | chlorothalonil | 90:1 to 1:4 | 30:1 to 1:2 | 15:1 to 2:1 |
| Compound 18 | chlozolinate | 45:1 to 1:2 | 15:1 to 2:1 | 11:1 to 2:1 |
| Compound 18 | clotrimazole | 15:1 to 1:9 | 5:1 to 1:3 | 3:1 to 1:3 |
| Compound 18 | copper salts such as Bordeaux mixture (tribasic copper sulfate), copper oxychloride, copper sulfate and copper hydroxide | 450:1 to 1:1 | 150:1 to 4:1 | 45:1 to 5:1 |
| Compound 18 | cyazofamid | 4:1 to 1:18 | 1:1 to 1:6 | 1:1 to 1:6 |
| Compound 18 | cyflufenamid | 1:1 to 1:90 | 1:2 to 1:30 | 1:2 to 1:24 |
| Compound 18 | cymoxanil | 6:1 to 1:18 | 2:1 to 1:6 | 1:1 to 1:5 |
| Compound 18 | cyproconazole | 4:1 to 1:18 | 1:1 to 1:6 | 1:1 to 1:6 |
| Compound 18 | cyprodinil | 22:1 to 1:9 | 7:1 to 1:3 | 4:1 to 1:2 |
| Compound 18 | dichlofluanid | 150:1 to 1:36 | 50:1 to 1:12 | 15:1 to 2:1 |
| Compound 18 | diclocymet | 150:1 to 1:36 | 50:1 to 1:12 | 15:1 to 2:1 |
| Compound 18 | diclomezine | 15:1 to 1:9 | 5:1 to 1:3 | 3:1 to 1:3 |
| Compound 18 | dicloran | 150:1 to 1:36 | 50:1 to 1:12 | 15:1 to 2:1 |
| Compound 18 | diethofencarb | 22:1 to 1:9 | 7:1 to 1:3 | 7:1 to 1:2 |
| Compound 18 | difenoconazole | 4:1 to 1:36 | 1:1 to 1:12 | 1:1 to 1:12 |
| Compound 18 | diflumetorim | 150:1 to 1:36 | 50:1 to 1:12 | 15:1 to 2:1 |
| Compound 18 | dimethirimol | 3:1 to 1:90 | 1:1 to 1:30 | 1:3 to 1:30 |
| Compound 18 | dimethomorph | 9:1 to 1:6 | 3:1 to 1:2 | 3:1 to 1:2 |
| Compound 18 | dimoxystrobin | 9:1 to 1:18 | 3:1 to 1:6 | 2:1 to 1:4 |
| Compound 18 | diniconazole | 3:1 to 1:36 | 1:1 to 1:12 | 1:1 to 1:8 |
| Compound 18 | diniconazole M | 3:1 to 1:90 | 1:1 to 1:30 | 1:1 to 1:12 |
| Compound 18 | dinocap | 7:1 to 1:9 | 2:1 to 1:3 | 2:1 to 1:3 |
| Compound 18 | dithianon | 15:1 to 1:4 | 5:1 to 1:2 | 5:1 to 1:2 |
| Compound 18 | dodemorph | 30:1 to 1:3 | 10:1 to 1:1 | 7:1 to 1:1 |
| Compound 18 | dodine | 30:1 to 1:2 | 10:1 to 2:1 | 10:1 to 2:1 |
| Compound 18 | edifenphos | 30:1 to 1:9 | 10:1 to 1:3 | 3:1 to 1:3 |
| Compound 18 | enoxastrobin | 9:1 to 1:18 | 3:1 to 1:6 | 2:1 to 1:4 |
| Compound 18 | epoxiconazole | 3:1 to 1:36 | 1:1 to 1:12 | 1:1 to 1:7 |
| Compound 18 | etaconazole | 3:1 to 1:36 | 1:1 to 1:12 | 1:1 to 1:7 |
| Compound 18 | ethaboxam | 7:1 to 1:9 | 2:1 to 1:3 | 2:1 to 1:3 |
| Compound 18 | ethirimol | 30:1 to 1:3 | 10:1 to 1:1 | 7:1 to 1:1 |
| Compound 18 | etridiazole | 30:1 to 1:9 | 10:1 to 1:3 | 7:1 to 1:2 |
| Compound 18 | famoxadone | 9:1 to 1:18 | 3:1 to 1:6 | 2:1 to 1:4 |
| Compound 18 | fenamidone | 6:1 to 1:18 | 2:1 to 1:6 | 2:1 to 1:4 |
| Compound 18 | fenarimol | 3:1 to 1:90 | 1:1 to 1:30 | 1:2 to 1:24 |
| Compound 18 | fenbuconazole | 3:1 to 1:30 | 1:1 to 1:10 | 1:1 to 1:10 |
| Compound 18 | fenfuram | 18:1 to 1:6 | 6:1 to 1:2 | 4:1 to 1:2 |
| Compound 18 | fenhexamid | 30:1 to 1:2 | 10:1 to 2:1 | 10:1 to 2:1 |
| Compound 18 | fenoxanil | 150:1 to 1:36 | 50:1 to 1:12 | 15:1 to 1:1 |
| Compound 18 | fenpiclonil | 75:1 to 1:9 | 25:1 to 1:3 | 15:1 to 2:1 |
| Compound 18 | fenpropidin | 30:1 to 1:3 | 10:1 to 1:1 | 7:1 to 1:1 |
| Compound 18 | fenpropimorph | 30:1 to 1:3 | 10:1 to 1:1 | 7:1 to 1:1 |
| Compound 18 | fenpyrazamine | 100:1 to 1:100 | 10:1 to 1:10 | 3:1 to 1:3 |
| Compound 18 | fentin salt such as the acetate, chloride or hydroxide | 15:1 to 1:9 | 5:1 to 1:3 | 3:1 to 1:3 |
| Compound 18 | ferbam | 300:1 to 1:2 | 100:1 to 2:1 | 30:1 to 4:1 |
| Compound 18 | ferimzone | 30:1 to 1:5 | 10:1 to 1:2 | 7:1 to 1:2 |
| Compound 18 | fluazinam | 22:1 to 1:5 | 7:1 to 1:2 | 3:1 to 1:2 |
| Compound 18 | fludioxonil | 7:1 to 1:12 | 2:1 to 1:4 | 2:1 to 1:4 |
| Compound 18 | flumetover | 9:1 to 1:6 | 3:1 to 1:2 | 3:1 to 1:2 |
| Compound 18 | flumorph | 9:1 to 1:18 | 3:1 to 1:6 | 3:1 to 1:3 |
| Compound 18 | fluopicolide | 3:1 to 1:18 | 1:1 to 1:6 | 1:1 to 1:6 |
| Compound 18 | fluopyram | 15:1 to 1:9 | 5:1 to 1:3 | 3:1 to 1:3 |
| Compound 18 | fluoromide | 150:1 to 2:1 | 50:1 to 4:1 | 37:1 to 5:1 |
| Compound 18 | fluoxastrobin | 4:1 to 1:18 | 1:1 to 1:6 | 1:1 to 1:6 |
| Compound 18 | fluquinconazole | 4:1 to 1:12 | 1:1 to 1:4 | 1:1 to 1:4 |
| Compound 18 | flusilazole | 15:1 to 1:9 | 5:1 to 1:3 | 3:1 to 1:3 |
| Compound 18 | flusulfamide | 90:1 to 1:2 | 30:1 to 2:1 | 15:1 to 2:1 |
| Compound 18 | flutianil | 7:1 to 1:36 | 2:1 to 1:12 | 1:1 to 1:6 |
| Compound 18 | flutolanil | 18:1 to 1:6 | 6:1 to 1:2 | 4:1 to 1:2 |
| Compound 18 | flutriafol | 4:1 to 1:12 | 1:1 to 1:4 | 1:1 to 1:4 |
| Compound 18 | fluxapyroxad | 12:1 to 1:9 | 4:1 to 1:3 | 2:1 to 1:3 |
| Compound 18 | folpet | 90:1 to 1:4 | 30:1 to 1:2 | 15:1 to 2:1 |
| Compound 18 | fosetyl-aluminum | 225:1 to 2:1 | 75:1 to 5:1 | 30:1 to 5:1 |
| Compound 18 | fuberidazole | 45:1 to 1:4 | 15:1 to 1:2 | 11:1 to 2:1 |
| Compound 18 | furalaxyl | 15:1 to 1:45 | 5:1 to 1:15 | 1:1 to 1:6 |
| Compound 18 | furametpyr | 150:1 to 1:36 | 50:1 to 1:12 | 15:1 to 2:1 |
| Compound 18 | guazatine or iminoctadine | 150:1 to 1:36 | 50:1 to 1:12 | 15:1 to 2:1 |
| Compound 18 | hexaconazole | 15:1 to 1:36 | 5:1 to 1:12 | 1:1 to 1:5 |
| Compound 18 | hymexazol | 225:1 to 2:1 | 75:1 to 4:1 | 75:1 to 9:1 |
| Compound 18 | imazalil | 7:1 to 1:18 | 2:1 to 1:6 | 1:1 to 1:5 |
| Compound 18 | imibenconazole | 15:1 to 1:36 | 5:1 to 1:12 | 1:1 to 1:5 |
| Compound 18 | iodocarb | 150:1 to 1:36 | 50:1 to 1:12 | 15:1 to 2:1 |
| Compound 18 | ipconazole | 15:1 to 1:36 | 5:1 to 1:12 | 1:1 to 1:5 |
| Compound 18 | iprobenfos | 150:1 to 1:36 | 50:1 to 1:12 | 15:1 to 2:1 |
| Compound 18 | iprodione | 120:1 to 1:2 | 40:1 to 2:1 | 15:1 to 2:1 |
| Compound 18 | iprovalicarb | 9:1 to 1:9 | 3:1 to 1:3 | 2:1 to 1:3 |
| Compound 18 | isoprothiolane | 150:1 to 2:1 | 50:1 to 4:1 | 45:1 to 5:1 |
| Compound 18 | isopyrazam | 12:1 to 1:9 | 4:1 to 1:3 | 2:1 to 1:3 |
| Compound 18 | isotianil | 12:1 to 1:9 | 4:1 to 1:3 | 2:1 to 1:3 |
| Compound 18 | kasugamycin | 7:1 to 1:90 | 2:1 to 1:30 | 1:2 to 1:24 |
| Compound 18 | kresoxim-methyl | 7:1 to 1:18 | 2:1 to 1:6 | 2:1 to 1:4 |
| Compound 18 | mancozeb | 180:1 to 1:3 | 60:1 to 2:1 | 22:1 to 3:1 |
| Compound 18 | mandipropamid | 6:1 to 1:18 | 2:1 to 1:6 | 2:1 to 1:4 |
| Compound 18 | maneb | 180:1 to 1:3 | 60:1 to 2:1 | 22:1 to 3:1 |
| Compound 18 | mepanipyrim | 18:1 to 1:3 | 6:1 to 1:1 | 6:1 to 1:1 |
| Compound 18 | mepronil | 7:1 to 1:36 | 2:1 to 1:12 | 1:1 to 1:6 |
| Compound 18 | meptyldinocap | 7:1 to 1:9 | 2:1 to 1:3 | 2:1 to 1:3 |
| Compound 18 | metalaxyl | 15:1 to 1:45 | 5:1 to 1:15 | 1:1 to 1:6 |
| Compound 18 | metalaxyl-M | 7:1 to 1:90 | 2:1 to 1:30 | 1:1 to 1:12 |
| Compound 18 | metconazole | 3:1 to 1:18 | 1:1 to 1:6 | 1:1 to 1:6 |
| Compound 18 | methasulfocarb | 150:1 to 1:36 | 50:1 to 1:12 | 15:1 to 1:1 |
| Compound 18 | metiram | 150:1 to 1:36 | 50:1 to 1:12 | 15:1 to 1:1 |
| Compound 18 | metominostrobin | 9:1 to 1:12 | 3:1 to 1:4 | 3:1 to 1:3 |
| Compound 18 | metrafenone | 6:1 to 1:12 | 2:1 to 1:4 | 2:1 to 1:4 |
| Compound 18 | myclobutanil | 5:1 to 1:26 | 1:1 to 1:9 | 1:1 to 1:8 |
| Compound 18 | naftifine | 150:1 to 1:36 | 50:1 to 1:12 | 15:1 to 2:1 |
| Compound 18 | neo-asozin (ferric methanearsonate) | 150:1 to 1:36 | 50:1 to 1:12 | 15:1 to 2:1 |
| Compound 18 | nuarimol | 15:1 to 1:9 | 5:1 to 1:3 | 3:1 to 1:3 |
| Compound 18 | octhilinone | 150:1 to 1:36 | 50:1 to 1:12 | 15:1 to 1:1 |
| Compound 18 | ofurace | 15:1 to 1:45 | 5:1 to 1:15 | 1:1 to 1:6 |
| Compound 18 | orysastrobin | 9:1 to 1:12 | 3:1 to 1:4 | 3:1 to 1:3 |
| Compound 18 | oxadixyl | 15:1 to 1:45 | 5:1 to 1:15 | 1:1 to 1:6 |
| Compound 18 | oxolinic acid | 30:1 to 1:9 | 10:1 to 1:3 | 7:1 to 1:2 |
| Compound 18 | oxpoconazole | 15:1 to 1:36 | 5:1 to 1:12 | 1:1 to 1:5 |
| Compound 18 | oxycarboxin | 18:1 to 1:6 | 6:1 to 1:2 | 4:1 to 1:2 |
| Compound 18 | oxytetracycline | 15:1 to 1:9 | 5:1 to 1:3 | 3:1 to 1:3 |
| Compound 18 | pefurazoate | 150:1 to 1:36 | 50:1 to 1:12 | 15:1 to 2:1 |
| Compound 18 | penconazole | 1:1 to 1:45 | 1:2 to 1:15 | 1:2 to 1:15 |
| Compound 18 | pencycuron | 150:1 to 1:2 | 50:1 to 2:1 | 11:1 to 2:1 |
| Compound 18 | penflufen | 12:1 to 1:9 | 4:1 to 1:3 | 2:1 to 1:3 |
| Compound 18 | penthiopyrad | 12:1 to 1:9 | 4:1 to 1:3 | 2:1 to 1:3 |
| Compound 18 | phosphorous acid and salts thereof | 150:1 to 1:36 | 50:1 to 1:12 | 15:1 to 2:1 |
| Compound 18 | phthalide | 150:1 to 1:36 | 50:1 to 1:12 | 15:1 to 2:1 |
| Compound 18 | picoxystrobin | 7:1 to 1:18 | 2:1 to 1:6 | 1:1 to 1:5 |
| Compound 18 | piperalin | 15:1 to 1:9 | 5:1 to 1:3 | 3:1 to 1:3 |
| Compound 18 | polyoxin | 15:1 to 1:9 | 5:1 to 1:3 | 3:1 to 1:3 |
| Compound 18 | probenazole | 15:1 to 1:9 | 5:1 to 1:3 | 3:1 to 1:3 |
| Compound 18 | prochloraz | 22:1 to 1:4 | 7:1 to 1:1 | 7:1 to 1:2 |
| Compound 18 | procymidone | 45:1 to 1:3 | 15:1 to 1:1 | 11:1 to 2:1 |
| Compound 18 | propamocarb or propamocarb-hydrochloride | 30:1 to 1:2 | 10:1 to 2:1 | 10: 1 to 2:1 |
| Compound 18 | propiconazole | 4:1 to 1:18 | 1:1 to 1:6 | 1:1 to 1:5 |
| Compound 18 | propineb | 45:1 to 1:2 | 15:1 to 2:1 | 11:1 to 2:1 |
| Compound 18 | proquinazid | 3:1 to 1:36 | 1:1 to 1:12 | 1:1 to 1:12 |
| Compound 18 | prothiocarb | 9:1 to 1:18 | 3:1 to 1:6 | 3:1 to 1:3 |
| Compound 18 | prothioconazole | 6:1 to 1:18 | 2:1 to 1:6 | 1:1 to 1:5 |
| Compound 18 | pyraclostrobin | 9:1 to 1:18 | 3:1 to 1:6 | 2:1 to 1:4 |
| Compound 18 | pyrametostrobin | 9:1 to 1:18 | 3:1 to 1:6 | 2:1 to 1:4 |
| Compound 18 | pyraoxystrobin | 9:1 to 1:18 | 3:1 to 1:6 | 2:1 to 1:4 |
| Compound 18 | pyrazophos | 150:1 to 1:36 | 50:1 to 1:12 | 15:1 to 1:1 |
| Compound 18 | pyribencarb | 15:1 to 1:6 | 5:1 to 1:2 | 4:1 to 1:2 |
| Compound 18 | pyrifenox | 15:1 to 1:9 | 5:1 to 1:3 | 3:1 to 1:3 |
| Compound 18 | pyrimethanil | 30:1 to 1:6 | 10:1 to 1:2 | 3:1 to 1:2 |
| Compound 18 | pyriofenone | 6:1 to 1:12 | 2:1 to 1:4 | 2:1 to 1:4 |
| Compound 18 | pyroquilon | 15:1 to 1:9 | 5:1 to 1:3 | 3:1 to 1:3 |
| Compound 18 | pyrrolnitrin | 150:1 to 1:36 | 50:1 to 1:12 | 15:1 to 2:1 |
| Compound 18 | quinconazole | 4:1 to 1:12 | 1:1 to 1:4 | 1:1 to 1:4 |
| Compound 18 | quinomethionate | 150:1 to 1:36 | 50:1 to 1:12 | 15:1 to 2:1 |
| Compound 18 | quinoxyfen | 4:1 to 1:18 | 1:1 to 1:6 | 1:1 to 1:6 |
| Compound 18 | quintozene | 150:1 to 1:36 | 50:1 to 1:12 | 15:1 to 2:1 |
| Compound 18 | silthiofam | 7:1 to 1:18 | 2:1 to 1:6 | 2:1 to 1:4 |
| Compound 18 | simeconazole | 15:1 to 1:36 | 5:1 to 1:12 | 1:1 to 1:5 |
| Compound 18 | spiroxamine | 22:1 to 1:4 | 7:1 to 1:2 | 5:1 to 1:2 |
| Compound 18 | streptomycin | 15:1 to 1:9 | 5:1 to 1:3 | 3:1 to 1:3 |
| Compound 18 | sulfur | 300:1 to 3:1 | 100:1 to 9:1 | 75:1 to 9:1 |
| Compound 18 | tebuconazole | 7:1 to 1:18 | 2:1 to 1:6 | 1:1 to 1:5 |
| Compound 18 | tebufloquin | 100:1 to 1:100 | 10:1 to 1:10 | 3:1 to 1:3 |
| Compound 18 | tecloftalam | 150:1 to 1:36 | 50:1 to 1:12 | 15:1 to 2:1 |
| Compound 18 | tecnazene | 150:1 to 1:36 | 50:1 to 1:12 | 15:1 to 2:1 |
| Compound 18 | terbinafine | 150:1 to 1:36 | 50:1 to 1:12 | 15:1 to 2:1 |
| Compound 18 | tetraconazole | 15:1 to 1:36 | 5:1 to 1:12 | 1:1 to 1:5 |
| Compound 18 | thiabendazole | 45:1 to 1:4 | 15:1 to 1:2 | 11:1 to 2:1 |
| Compound 18 | thifluzamide | 15:1 to 1:9 | 5:1 to 1:3 | 3:1 to 1:3 |
| Compound 18 | thiophanate | 45:1 to 1:3 | 15:1 to 2:1 | 11:1 to 2:1 |
| Compound 18 | thiophanate- methyl | 45:1 to 1:3 | 15:1 to 2:1 | 11:1 to 2:1 |
| Compound 18 | thiram | 150:1 to 1:2 | 50:1 to 2:1 | 37:1 to 5:1 |
| Compound 18 | tiadinil | 12:1 to 1:9 | 4:1 to 1:3 | 2:1 to 1:3 |
| Compound 18 | tolclofos-methyl | 150:1 to 1:2 | 50:1 to 2:1 | 37:1 to 5:1 |
| Compound 18 | tolylfluanid | 150:1 to 1:36 | 50:1 to 1:12 | 15:1 to 2:1 |
| Compound 18 | triadimefon | 15:1 to 1:36 | 5:1 to 1:12 | 1:1 to 1:5 |
| Compound 18 | triadimenol | 15:1 to 1:36 | 5:1 to 1:12 | 1:1 to 1:5 |
| Compound 18 | triarimol | 3:1 to 1:90 | 1:1 to 1:30 | 1:2 to 1:24 |
| Compound 18 | triazoxide | 150:1 to 1:36 | 50:1 to 1:12 | 15:1 to 2:1 |
| Compound 18 | tricyclazole | 15:1 to 1:9 | 5:1 to 1:3 | 3:1 to 1:3 |
| Compound 18 | tridemorph | 30:1 to 1:3 | 10:1 to 1:1 | 7:1 to 1:1 |
| Compound 18 | trifloxystrobin | 6:1 to 1:18 | 2:1 to 1:6 | 2:1 to 1:4 |
| Compound 18 | triflumizole | 15:1 to 1:9 | 5:1 to 1:3 | 3:1 to 1:3 |
| Compound 18 | triforine | 15:1 to 1:9 | 5:1 to 1:3 | 3:1 to 1:3 |
| Compound 18 | trimorphamide | 45:1 to 1:9 | 15:1 to 1:3 | 7:1 to 1:2 |
| Compound 18 | triticonazole | 15:1 to 1:36 | 5:1 to 1:12 | 1:1 to 1:5 |
| Compound 18 | uniconazole | 15:1 to 1:36 | 5:1 to 1:12 | 1:1 to 1:5 |
| Compound 18 | validamycin | 150:1 to 1:36 | 50:1 to 1:12 | 3:1 to 1:3 |
| Compound 18 | valifenalate | 6:1 to 1:18 | 2:1 to 1:6 | 2:1 to 1:4 |
| Compound 18 | vinclozolin | 120:1 to 1:2 | 40:1 to 2:1 | 15:1 to 2:1 |
| Compound 18 | zineb | 150:1 to 1:2 | 50:1 to 2:1 | 37:1 to 5:1 |
| Compound 18 | ziram | 150:1 to 1:2 | 50:1 to 2:1 | 37:1 to 5:1 |
| Compound 18 | zoxamide | 6:1 to 1:18 | 2:1 to 1:6 | 2:1 to 1:4 |
| Compound 18 | *N*-[2-[4-[[3-(4-chlorophenyl)-2-propyn-1-yl]oxy]-3-methoxyphenyl]ethyl]-3-methyl-2-[(methylsulfonyl)amino]butanamide | 6:1 to 1:18 | 2:1 to 1:6 | 2:1 to 1:4 |
| Compound 18 | *N*-[2-[4-[[3-(4-chlorophenyl)-2-propyn-1-yl]oxy]-3-methoxyphenyl]ethyl]-3-methyl-2-[(ethylsulfonyl)amino]butanamide | 6:1 to 1:18 | 2:1 to 1:6 | 2:1 to 1:4 |
| Compound 18 | 2-butoxy-6-iodo-3-propyl-4*H*-1-benzopyran-4-one | 3:1 to 1:36 | 1:1 to 1:12 | 1:1 to 1:12 |
| Compound 18 | 3-[5-(4-chlorophenyl)-2,3-dimethyl-3-isoxazolidinyl]pyridine | 15:1 to 1:9 | 5:1 to 1:3 | 3:1 to 1:3 |
| Compound 18 | *N*'-[4-[[3-[(4-chlorophenyl)methyl]-1,2,4-thiadiazol-5-yl]oxy]-2,5-dimethylphenyl]-*N*-ethyl-*N*-methylmethanimidamide | 20:1 to 1:20 | 8:1 to 1:8 | 3:1 to 1:3 |
| Compound 18 | 4-fluorophenyl *N*-[1-[[[1-(4-cyanophenyl)-ethyl]sulfonyl]methyl]propyl]carbamate | 6:1 to 1:18 | 2:1 to 1:6 | 2:1 to 1:4 |
| Compound 18 | *N*-[[(cyclopropylmethoxy)amino][6-(difluoromethoxy)-2,3-difluorophenyl]-methylene]benzeneacetamide | 1:1 to 1:90 | 1:2 to 1:30 | 1:2 to 1:24 |
| Compound 18 | α-[methoxyimino]-*N*-methyl-2-[[[1-[3-(trifluoromethyl)phenyl]ethoxy]imino]-methyl]benzeneacetamide | 9:1 to 1:18 | 3:1 to 1:6 | 3:1 to 1:3 |
| Compound 18 | *N'*-[4-[4-chloro-3-(trifluoromethyl)-phenoxy]-2,5-dimethylphenyl]-*N*-ethyl-*N*-methylmethanimidamide | 15:1 to 1:18 | 5:1 to 1:6 | 3:1 to 1:3 |
| Compound 18 | *N*-(4-chloro-2-nitrophenyl)-*N*-ethyl-4-methylbenzenesulfonamide | 15:1 to 1:18 | 5:1 to 1:6 | 3:1 to 1:3 |
| Compound 18 | fenaminstrobin (2-[[[3-(2,6-dichlorophenyl)-1-methyl-2-propen-1-ylidene]amino]oxy]methyl]-α-(methoxyimino)-*N*-methylbenzeneacetamide) | 9:1 to 1:18 | 3:1 to 1:6 | 3:1 to 1:3 |
| Compound 18 | pentyl *N*-[4-[[[[(1-methyl-1*H-*tetrazol-5-yl)-phenylmethylene]amino]oxy]methyl]-2-thiazolyl]carbamate | 9:1 to 1:18 | 3:1 to 1:6 | 3:1 to 1:3 |
| Compound 18 | 2-[(3-bromo-6-quinolinyl)oxy]-*N*-(1,1-dimethyl-2-butyn-1-yl)-2-(methylthio)acetamide | 5:1 to 1:22 | 2:1 to 1:8 | 2:1 to 1:4 |
| Compound 18 | 2-[(3-ethynyl-6-quinolinyl)oxy]-*N-*[1-(hydroxymethyl)-1-methyl-2-propyn-1-yl]-2-(methylthio)acetamide | 5:1 to 1:22 | 2:1 to 1:8 | 2:1 to 1:4 |
| Compound 18 | *N*-(1,1-dimethyl-2-butyn-1-yl)-2-[(3 - ethynyl-6-quinolinyl)oxy]-2-(methylthio)acetamide | 5:1 to 1:22 | 2:1 to 1:8 | 2:1 to 1:4 |

Tables B2 through B29 are each constructed the same as Table B1 above except that entries below the "Component (a)" column heading are replaced with the respective Component (a) Column Entry shown below. Thus, for example, in Table B2 the entries below the "Component (a)" column heading all recite "Compound 10", and the first line below the column headings in Table B2 specifically discloses combination of Compound 10 with acibenzolar-S-methyl. Tables B3 through B29 are constructed similarly.

| Table Number | Component (a) Column Entry | Table Number | Component (a) Column Entry |
|---|---|---|---|
| B2 | Compound 10 | B16 | Compound 97 |
| B3 | Compound 20 | B17 | Compound 136 |
| B4 | Compound 11 | B18 | Compound 151 |
| B5 | Compound 17 | B19 | Compound 118 |
| B6 | Compound 23 | B20 | Compound 152 |
| B7 | Compound 28 | B21 | Compound 153 |
| B8 | Compound 8 | B22 | Compound 123 |
| B9 | Compound 31 | B23 | Compound 144 |
| B10 | Compound 66 | B24 | Compound 145 |
| B11 | Compound 38 | B25 | Compound 146 |
| B12 | Compound 91 | B26 | Compound 147 |
| B13 | Compound 46 | B27 | Compound 148 |
| B14 | Compound 115 | B28 | Compound 149 |
| B15 | Compound 116 | B29 | Compound 150 |

The compounds of the present invention are particularly photolytically stable relative to similar compounds of the same chemical sub-structure. The enhanced photostability results in longer half-life under field conditions where ultraviolet irradiation from sunlight can degrade susceptible compounds. The longer field half-life results in control of plant pathogens for an extended period of time. Data is provided for compounds of the present invention and comparative compounds outside the present scope in the Photostability Example. The Photostability Example data demonstrates that the compounds of the present invention with methyl substitution in the ring between the carbamate functional group and the A group (tolyl substitution or R^{P} is methyl) are more stable to simulated sunlight than the corresponding halogen substituted compounds (R^{P} is halogen).

Representative compounds of this invention prepared by the methods described herein are shown in Index Tables A-G. See Index Table H for ¹H NMR data. For mass spectral data (AP⁺ (M+1)), the numerical value reported is the molecular weight of the parent molecular ion (M) formed by addition of H⁺ (molecular weight of 1) to the molecule to give a M+1 peak observed by mass spectrometry using atmospheric pressure chemical ionization (AP⁺). The alternate molecular ion peaks (e.g., M+2 or M+4) that occur with compounds containing multiple halogens are not reported. The reported M+1 peaks were observed by mass spectrometry using atmospheric pressure chemical ionization (AP⁺) or electrospray ionization (ESI).

The following abbreviations are used in the Index Tables which follow: n is normal, *i* is iso, *c* is cyclo, Me is methyl, Et is ethyl, Pr is propyl, OMe is methoxy, OEt is ethoxy, SMe is methylthio, SEt is ethylthio, -CN is cyano and -NO₂ is nitro.

**INDEX TABLE A**

| | | | | |
|---|---|---|---|---|
| 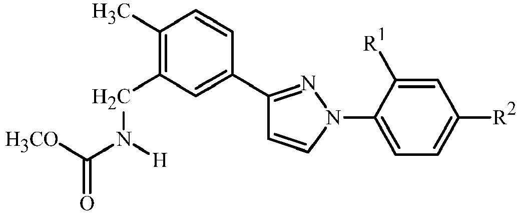 | | | | |

| Cmpd. | R¹ | R² | AP+ (M+1) | m.p. (°C) |
|---|---|---|---|---|
| 1 | OCH₂CH₃ | OCH₃ | 396 | |
| 2 | OH | OCH₃ | 368 | |
| 5 | OCH₂C≡CH | OCH₃ | 406 | |
| 15 | OCH₃ | SCH₃ | 398 | |
| 16 | SCH₃ | OCH₃ | 398 | |
| 18 | CH₃ | OCH₃ | | 117-120 |
| 19 | F | OCH₃ | | 112-114 |
| 20 | Cl | OCH₃ | 386 | |
| 21 | CN | OCH₃ | 399* | |
| 22 | NO₂ | OCH₃ | 397 | |
| 24 | OCH₃ | OCH₃ | | ** |
| 26 | CH₂CH₃ | OCH₃ | 380 | |
| 27 | OCH₃ | F | 370 | |
| 28 | OCH₃ | Cl | 386 | |
| 29 | OCH₃ | CH₃ | 366 | |
| 32 | OCH₂CN | OCH₃ | 407 | |
| 33 | OH | Cl | 372 | |
| 34 | OCH₂C≡CH | Cl | 410 | |
| 35 | OCH₂CN | Cl | 411 | |
| 48 | NO₂ | Br | 445 | |
| 49 | NO₂ | Cl | 401 | |
| 52 | CN | Cl | 381 | |
| 60 | Cl | F | 374 | 105-107 |
| 61 | Cl | Br | 433 | 127-130 |
| 62 | Br | Br | 478 | 144-147 |
| 63 | Br | Cl | 434 | 140-143 |
| 64 | F | F | 358 | 133-135 |
| 65 | F | Cl | 374 | 140-142 |
| 66 | CH₃ | CH₃ | 374 | 140-142 |
| 84 | Cl | Cl | 390 | 125-126 |
| 89 | Cl | NO₂ | 375 | |
| 90 | Cl | NH₂ | 371 | |
| 93 | CN | CN | ** | |
| 111 | CN | Br | ** | |
| 120 | CH₃ | OCH₂CH₃ | 380 | |
| 121 | CH₃ | OCH(CH₃)₂ | 394 | |
| 122 | CH₃ | O(*c*-Hex) | 434 | |
| 123 | CH₃ | OCF₃ | 420 | |
| 124 | CH₃ | *t*-Bu | 392 | |
| 125 | CH₃ | OCH₂CH₂CH₃ | 394 | |
| 126 | CH₃ | OCH₂CH₂OCH₃ | 410 | |
| 133 | CH₃ | CH(CH₃)₂ | 378 | |
| 134 | CH₃ | OCH₂CH(CH₃)₂ | 408 | |
| 139 | CF₃ | CF₃ | ** | |
| 144 | CH₃ | OCHF₂ | | |

| | | | | |
|---|---|---|---|---|
| * sodium adduct of molecular ion ** see Index Table H for ¹H NMR data. | | | | |

**INDEX TABLE B**

| | | | | |
|---|---|---|---|---|
| 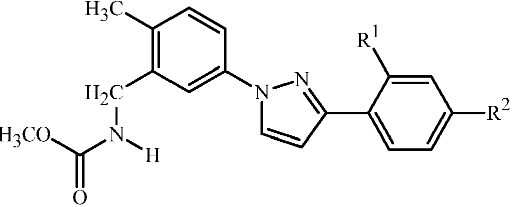 | | | | |

| Cmpd. | R¹ | R² | AP+ (M+1) | m.p. (°C) |
|---|---|---|---|---|
| 9 | F | OCH₃ | 370 | 110-112 |
| 10 | CH₃ | OCH₃ | 366 | 97-99 |
| 11 | OCH₃ | Cl | 386 | 126-127 |
| 12 | OCH₃ | CH₃ | 366 | 141-143 |
| 13 | Cl | OCH₃ | 386 | 84-86 |
| 14 | OCH₃ | F | 370 | 126-127 |
| 23 | OCH₃ | OCH₃ | 382 | 127 |
| 30 | F | F | 358 | 118-120 |
| 31 | Cl | Cl | 390 | 101-103 |
| 36 | CH₃ | Cl | 370 | |
| 37 | CH₃ | F | 354 | |
| 38 | CH₃ | CH₃ | 350 | |
| 39 | F | Cl | 374 | |
| 40 | Cl | F | 374 | |
| 41 | Cl | CH₃ | 370 | |
| 42 | F | CH₃ | 354 | |
| 51 | OCH₂C≡CH | OCH₃ | | 132-135 |
| 53 | OCH₂CN | OCH₃ | | 130-132 |
| 55 | Cl | Br | 433 | |
| 56 | F | Br | 417 | |
| 57 | Br | F | 417 | |
| 58 | OCH₃ | Br | 429 | |
| 59 | Br | OCH₃ | 431 | |
| 69 | CH₃ | CN | 361 | |
| 71 | OH | Cl | | 159-162 |
| 72 | OCH₂CN | Cl | | 163-166 |
| 76 | Cl | OCH₂CH₃ | 400 | |
| 77 | OCH₂CH₃ | Cl | 400 | |
| 80 | Br | Cl | 433 | |
| 82 | *c*-Pr | OCH₃ | 392 | |
| 83 | CH₂CH₃ | OCH₃ | 380 | |
| 92 | Cl | I | 481 | |
| 100 | CH₃ | CF₃ | 404 | |
| 101 | OCH₃ | CF₃ | 420 | |
| 102 | CH₃ | Br | 414 | |
| 103 | OH | OCH₃ | | 139-142 |
| 108 | OCH₂C≡CH | Cl | | 126-129 |
| 109 | Br | Br | 477 | |
| 110 | Cl | NO₂ | 400 | |
| 112 | CH₃ | C≡CH | 360 | |
| 113 | CH₃ | CH=CH₂ | 362 | |
| 127 | Br | CH₃ | 415 | |
| 128 | CH₃ | OCH₂CH₃ | 380 | |
| 129 | OCH₂CH₃ | F | 384 | |
| 137 | F | OCH₂CH₃ | 384 | |
| 138 | OCH₂CH₃ | CH₃ | 380 | |
| 140 | CH₃ | (C=O)CH₃ | 378 | |
| 145 | CH₃ | OCF₃ | | |
| 146 | CH₃ | OCHF₂ | | |

**INDEX TABLE C**

| | | | | |
|---|---|---|---|---|
| 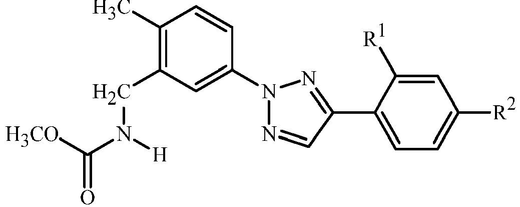 | | | | |

| Cmpd. | R¹ | R² | AP+ (M+1) | m.p. (°C) |
|---|---|---|---|---|
| 6 | OCH₃ | Cl | | 165-167 |
| 7 | OCH₃ | CH₃ | | 144-146 |
| 8 | CH₃ | OCH₃ | 367 | |
| 17 | OCH₃ | OCH₃ | | 132-135 |
| 47 | C≡CH | OCH₃ | 377 | |
| 50 | F | Cl | | 139-143 |
| 67 | F | F | 359 | |
| 68 | Cl | OCH₃ | 387 | |
| 73 | OCH₂CN | OCH₃ | 408 | |
| 74 | OCH₂C≡CH | OCH₃ | 407 | |
| 75 | OCH₂CH=CH₂ | OCH₃ | 409 | |
| 85 | NO₂ | OCH₃ | 398 | |
| 86 | Cl | Cl | 391 | |
| 87 | Cl | F | 375 | |
| 88 | OCH₂CH₃ | OCH₃ | 397 | |
| 95 | OCH₃ | I | 479 | |
| 96 | I | OCH₃ | 479 | |
| 97 | CH₃ | CH₃ | 351 | |
| 98 | OCH₂CN | CH₃ | ** | |
| 99 | OCH₃ | C≡CH | 377 | |
| 104 | CF₃ | OCH₃ | | 112-115 |
| 105 | CH₃ | OCH₂CN | 392 | |
| 106 | CH₃ | OCH₂C≡CH | 391 | |
| 107 | CH₃ | OCH₂CH₃ | 381 | |
| 132 | CF₃ | CF₃ | 459 | |
| 141 | OCH₃ | Br | 431 | |
| 143 | Br | OCH₃ | 431 | |
| 149 | CH₃ | OCF₃ | | |
| 150 | CH₃ | OCHF₂ | | |

**INDEX TABLE D**

| | | | | |
|---|---|---|---|---|
| 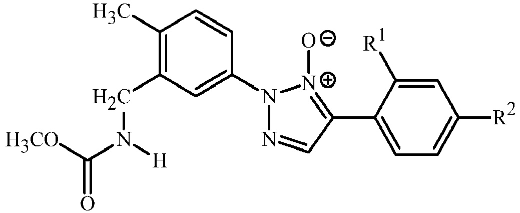 | | | | |

| Cmpd. | R¹ | R² | AP+ (M+1) | m.p. (°C) |
|---|---|---|---|---|
| 4 | OCH₃ | OCH₃ | 399 | |
| 81 | OCH₃ | I | ** | |
| 94 | I | OCH₃ | ** | |

**INDEX TABLE E**

| | | | | |
|---|---|---|---|---|
| | 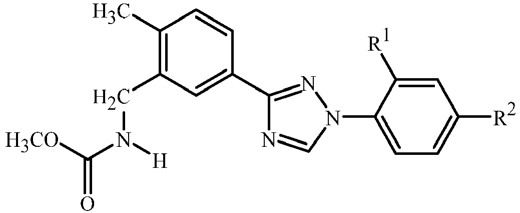 | | | |

| Cmpd. | R¹ | R² | AP+ (M+1) | m.p. (°C) |
|---|---|---|---|---|
| 54 | OCH₃ | OCH₃ | | 174-177 |
| 70 | CH₃ | OCH₃ | | 110-114 |
| 78 | Cl | OCH₃ | | 145-148 |
| 79 | F | OCH₃ | | 164-166 |
| 117 | F | F | | 156-159 |
| 118 | CH₃ | CH₃ | | 148-151 |
| 119 | Cl | Cl | | 152-155 |
| 147 | CH₃ | OCF₃ | | |
| 148 | CH₃ | OCHF₂ | | |

**TABLE F**

| | | | | | |
|---|---|---|---|---|---|
| 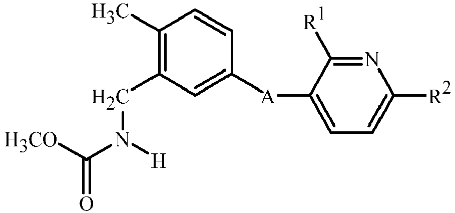 | | | | | |

| Cmpd. | A | R¹ | R² | AP+ (M+1) | m.p. (°C) |
|---|---|---|---|---|---|
| 43 | A-2 | Cl | CH₃ | 371 | |
| 44 | A-2 | CH₃ | Cl | 371 | |
| 45 | A-2 | OCH₃ | Cl | 386 | |
| 46 | A-2 | CH₃ | OCH₃ | 367 | |
| 91 | A-2 | OCH₃ | OCH₃ | 383 | |
| 114 | A-1 | CH₃ | CH₃ | 351 | |
| 115 | A-1 | OCH₃ | OCH₃ | 383 | |
| 116 | A-1 | CH₃ | OCH₃ | 367 | |
| 130 | A-2 | CH₃ | CH₃ | 351 | |
| 131 | A-2 | OCH₃ | CH₃ | 367 | |
| 136 | A-3 | OCH₃ | OCH₃ | 384 | |
| 142 | A-2 | CH₃ | OCH₂CH₃ | 382 | |
| 151 | A-3 | CH₃ | OCH₃ | | |
| 152 | A-4 | CH₃ | OCH₃ | | |
| 153 | A-4 | OCH₃ | OCH₃ | | |

**INDEX TABLE G**

| Cmpd. | Structure | AP+ (M+1) | m.p. (°C) |
|---|---|---|---|
| 135 | 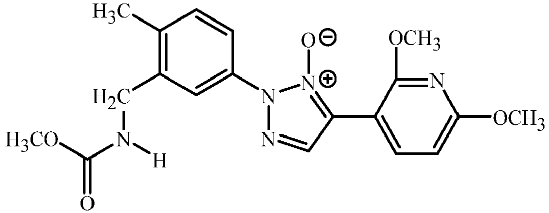 | 400 | |

**INDEX TABLE H**

| Cmpd No. | ¹H NMR Data^{a} |
|---|---|
| 24 | δ (CDCl₃) 7.89 (d, 1H), 7.78 (s, 1H), 7.68 (m, 2H), 7.21 (d, 1H), 6.68 (d, 1H), 6.59 (m, 2H), 4.85 (bs, 1H), 4.42 (d, 2H), 3.85 (s, 6H), 3.70 (s, 3H), 2.36 (s, 3H). |
| 81 | δ (CDCl₃) 7.97 (br s, 1H), 7.84 (br s, 1H), 7.82 (d, 1H), 7.78 (br d, 1H), 7.43 (dd, 1H), 7.36-7.32 (m, 2H), 4.98 (br s, 1H), 4.45 (br d, 2H), 3.94 (s, 3H), 3.71 (br, s, 3H), 2.40 (s, 3H). |
| 93 | δ (CDCl₃) 8.36 (d, 1H, J=5 Hz), 8.11 (d, 1H, J=10 Hz), 8.06 (s, 1H), 7.93 (d, 1H, J=10 Hz), 7.80 (s, 1H), 7.72 (d, 1H, J=10 Hz), 7.26 (d, 1H, J=5 Hz), 6.90 (s, 1H), 4.93 (s, 1H), 4.44 (d, 2H, J=5 Hz), 3.72 (s, 3H), 2.39 (s, 3H). |
| 94 | δ (CDCl₃) 7.93 (s, 1H), 7.87 (br s, 1H), 7.80 (br d, 1H), 7.59 (d, 1H), 7.51 (d, 1H), 7.35 (d, 1H), 7.01 (dd, 1H), 4.99 (br s, 1H), 4.44 (br d, 2H), 3.85 (s, 3H), 3.70 (br s, 3H), 2.41 (s, 3H). |
| 98 | δ (CDCl₃) 8.14 (s, 1H), 8.02 (m, 2H), 7.94 (dd, 1H), 7.29 (d, 1H), 7.04 (d, 1H), 6.90 (s, 1H), 4.96 (br s, 1H), 4.89 (s, 3H), 4.46 (br d, 2H), 3.73 (br s, 3H), 2.44 (s, 3H), 2.39 (s, 3H). |
| 111 | δ (CDCl₃) 8.17 (d, 1H, J=5 Hz), 7.88 (s, 1H), 7.79 (m, 3H), 7.70 (d, 1H, J=5 Hz), 7.23 (d, 1H, J=10 Hz), 6.82 (s, 1H), 4.95 (s, 1H), 4.42 (d, 2H, J=5 Hz), 2.93 (s, 3H), 3.26 (s, 3H). |
| 139 | δ (CDCl₃) 8.08 (s, 1H), 7.95 (d, 1H), 7.85 (d, 1H), 7.77 (m, 2H), 7.69 (m, 1H), 7.24 (m, 1H), 6.80 (d, 1H), 4.87 (br. s, 1H), 4.43 (d, 2H), 3.70 (s, 3H), 2.37 (s, 3H). |

| | |
|---|---|
| ^{a 1}H NMR data are in ppm downfield from tetramethylsilane. CDCl₃ solution unless indicated otherwise. DMSO-d₆ is CD₃S(O)CD₃. Couplings are designated by (s)-singlet, (d)-doublet, (t)-triplet, (m)-multiplet, (dd)-doublet of doublets, (br s)-broad singlet. | |

Aqueous photolysis data is a simple proxy for photostability under field conditions. The following aqueous photolysis data supports the enhanced photostability of the tolyl fungicides of the present invention.

### PHOTOSTABILITY EXAMPLE

Test compound (0.670 µg/mL) dissolved in 80/20 10 mM pH 7 phosphate buffer/acetonitrile is added to 2-mL borosilicate glass HPLC vials. The addition of 20% acetonitrile is intended to ensure solubility of compounds with low aqueous solubility. Samples are exposed to light in an Atlas Ci65 Burner photolysis chamber fitted with a xenon arc lamp (6500 W) to simulate natural sunlight for 24 hours and compared with samples wrapped in aluminum foil to protect them from light under the same conditions. The 24-hour irradiation is equivalent to ∼2.6 days of average Delaware summer sunlight exposure. Samples are analyzed by HPLC and results are reported as percent remaining based on peak area relative to the dark controls.

**TABLE P**

| | | | | | |
|---|---|---|---|---|---|
| 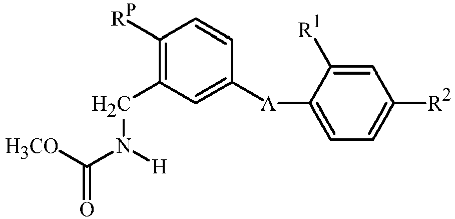 | | | | | |

| Cmpd. | A | R^{P} | R¹ | R² | % Cmpd Remaining |
|---|---|---|---|---|---|
| 18 | A-1 | CH₃ | CH₃ | OCH₃ | 98 |
| 18P | A-1 | Cl | CH₃ | OCH₃ | 0 |
| 24 | A-1 | CH₃ | OCH₃ | OCH₃ | 78 |
| 24P | A-1 | Cl | OCH₃ | OCH₃ | 0 |
| 23 | A-2 | CH₃ | OCH₃ | OCH₃ | 54 |
| 23P | A-2 | Cl | OCH₃ | OCH₃ | 0 |
| 23PP | A-2 | F | OCH₃ | OCH₃ | 5 |
| 17 | A-3 | CH₃ | OCH₃ | OCH₃ | 30 |
| 17P | A-3 | Cl | OCH₃ | OCH₃ | 0 |
| 10 | A-2 | CH₃ | CH₃ | OCH₃ | 97 |
| 10P | A-2 | Cl | CH₃ | OCH₃ | 0 |
| 10PP | A-2 | F | CH₃ | OCH₃ | 64 |

The following Tests demonstrate the control efficacy of compounds of this invention on specific pathogens. The pathogen control protection afforded by the compounds is not limited, however, to these species. See Index Tables A-G for compound descriptions.

### BIOLOGICAL EXAMPLES OF THE INVENTION

General protocol for preparing test suspensions for Tests A-E: the test compounds were first dissolved in acetone in an amount equal to 11% of the final volume and then suspended at the desired concentration (in ppm) in acetone and purified water (50/50 mix by volume) containing 250 ppm of the surfactant Trem® 014 (polyhydric alcohol esters). The resulting test suspensions were then used in Tests A-E. Spraying a 200 ppm test suspension to the point of run-off on the test plants was the equivalent of a rate of 800 g/ha.

### TEST A

The test suspension was sprayed to the point of run-off on wheat seedlings. The following day the seedlings were inoculated with a spore dust of *Erysiphe graminis* f. sp. *tritici,* (the causal agent of wheat powdery mildew) and incubated in a growth chamber at 20 °C for 8 days, after which time disease ratings were visually made.

### TEST B

The test suspension was sprayed to the point of run-off on wheat seedlings. The following day the seedlings were inoculated with a spore suspension of *Puccinia recondita* f. sp. *tritici* (the causal agent of wheat leaf rust) and incubated in a saturated atmosphere at 20 °C for 24 h, and then moved to a growth chamber at 20 °C for 7 days, after which time disease ratings were visually made.

### TEST C

The test suspension was sprayed to the point of run-off on wheat seedlings. The following day the seedlings were inoculated with a spore suspension of *Septoria tritici* (the causal agent of wheat leaf blotch) and incubated in a saturated atmosphere at 24 °C for 48 h, and then moved to a growth chamber at 20 °C for 19 days, after which time disease ratings were visually made.

### TEST D

The test suspension was sprayed to the point of run-off on wheat seedlings. The following day the seedlings were inoculated with a spore suspension of *Septoria nodorum* (the causal agent of wheat glume blotch) and incubated in a saturated atmosphere at 20 °C for 48 h, and then moved to a growth chamber at 20 °C for 7 days, after which time disease ratings were visually made.

### TEST E

The test suspension was sprayed to the point of run-off on tomato seedlings. The following day the seedlings were inoculated with a spore suspension of *Botrytis cinerea* (the causal agent of tomato botrytis) and incubated in saturated atmosphere at 20 °C for 48 h, and then moved to a growth chamber at 27 °C for 3 additional days, after which time disease ratings were visually made.

Results for Tests A-E are given in Table A. In the Table, a rating of 100 indicates 100% disease control and a rating of 0 indicates no disease control (relative to the controls). A dash (-) indicates no test results. All results are for 200 ppm except where followed by an "*", which indicates 40 ppm or "**", which indicates 50 ppm.

**TABLE A**

| Cmpd No. | Test A | Test B | Test C | Test D | Test E |
|---|---|---|---|---|---|
| 1 | 99 | 100 | 100 | 100 | 56 |
| 2 | - | - | - | - | - |
| 4 | 99 | 100 | 99 | 100 | 69 |
| 5 | - | - | - | - | - |
| 6 | 100 | 100 | 100 | 100 | 0 |
| 7 | 100 | 100 | 100 | 100 | 26 |
| 8 | 100 | 100 | 100 | 100 | 90 |
| 9 | 100 | 100 | 99 | 100 | 87 |
| 10 | 100 | 100 | 100 | 100 | 97 |
| 11 | 100 | 100 | 100 | 100 | 56 |
| 12 | 100 | 100 | 97 | 100 | 95 |
| 13 | 100 | 100 | 100 | 100 | 99 |
| 14 | 100 | 100 | 100 | 100 | 97 |
| 15 | 96 | 100 | 77 | 100 | 64 |
| 16 | 97 | 100 | 85 | 100 | 97 |
| 17 | 100 | 100 | 100 | 100 | 33 |
| 18 | 100 | 100 | 100 | - | 44 |
| 19 | 100 | 100 | 100 | - | 37 |
| 20 | 100 | 100 | 100 | 100 | 0 |
| 21 | 96 | 100 | 98 | 100 | 66 |
| 22 | 0 | 100 | 100 | 100 | 85 |
| 23 | 100 | 100 | 100 | 100 | 66 |
| 24 | 100 | 100 | 100 | - | 55 |
| 26 | 99 | 100 | 100 | 100 | 31 |
| 27 | 100 | 100 | 100 | 100 | 54 |
| 28 | 100 | 100 | 100 | 100 | 57 |
| 29 | 99 | 100 | 100 | 100 | 63 |
| 30 | 100 | 100 | 100 | 100 | 0 |
| 31 | 100 | 100 | 100 | 100 | 16 |
| 32 | 94 | 100 | 100 | 100 | 66 |
| 33 | 0 | 94 | 2 | 100 | 0 |
| 34 | 100 | 100 | 100 | 100 | 91 |
| 35 | 92 | 100 | 100 | 100 | 41 |
| 36 | 100* | 100* | 100* | 100* | 74* |
| 37 | 95* | 100* | 100* | 100* | 77* |
| 38 | 100* | 100* | 100* | 100* | 98* |
| 39 | 99* | 100* | 100* | 100* | 53* |
| 40 | 100* | 100* | 100* | 100* | 80* |
| 41 | 100* | 100* | 100* | 100* | 36* |
| 42 | 100* | 100* | 99* | 100* | 65* |
| 43 | 98* | 100* | 87* | 100* | 62* |
| 44 | 98* | 100* | 92* | 100* | 0* |
| 45 | 98* | 100* | 95* | 100* | 39* |
| 46 | 98* | 100* | 98* | 100* | 92* |
| 47 | 99* | 100* | - | 100* | 0* |
| 48 | 0* | 100* | 100* | 100* | 37* |
| 49 | 26* | 100* | 95* | 100* | 8* |
| 50 | 100* | 100* | 99* | 100* | 77* |
| 51 | 0 | 100 | 88 | 100 | 0 |
| 52 | 97* | 100* | 88* | 100* | 0* |
| 53 | 100 | 100 | 97 | 100 | 0 |
| 54 | 99* | 100* | 49* | 100* | 49* |
| 55 | 100* | 100* | 91* | 100* | 38* |
| 56 | 100* | 100* | 98* | 100* | 56* |
| 57 | 100* | 100* | 99* | 100* | 63* |
| 58 | 100* | 100* | 96* | 100* | 26* |
| 59 | 99* | 100* | 97* | 100* | 52* |
| 60 | 100* | 100* | 91* | 100* | 99* |
| 61 | 100* | 100* | 94* | 100* | 91* |
| 62 | 100* | 100* | 97* | 100* | 100* |
| 63 | 100* | 100* | 96* | 100* | 99* |
| 64 | 100* | 100* | 97* | 100* | 74* |
| 65 | 100* | 100* | 98* | 100* | 94* |
| 66 | 100* | 100* | 96* | 100* | 99* |
| 67 | 100* | 100* | 98* | 100* | 33* |
| 68 | 100* | 100* | 99* | 100* | 77* |
| 69 | 100* | 100* | 66* | 100* | 0* |
| 70 | 99* | 100* | 96* | 100* | 84* |
| 71 | 90 | 99 | 51 | 100 | 8 |
| 72 | 93* | 100* | 90* | 100* | 0* |
| 73 | 99* | 100* | - | 100* | 0* |
| 74 | 98* | 100* | - | 100* | 8* |
| 75 | 90* | 100* | - | 100* | 8* |
| 76 | 100* | 100* | 99* | 100* | 0* |
| 77 | 100* | 100* | 99* | 100* | 0* |
| 78 | 99* | 100* | 93* | 100* | 68* |
| 79 | 100* | 100* | 95* | 100* | 76* |
| 80 | 97* | 100* | 58* | 100* | 81* |
| 81 | 99 | 100 | 98 | 100 | 67 |
| 82 | 99* | 100* | - | 100* | 0* |
| 83 | 99* | 100* | - | 100* | 0* |
| 84 | - | - | - | - | - |
| 85 | 99* | 100* | - | 100* | 0* |
| 86 | 99* | 100* | 93* | 100* | 54* |
| 87 | 98* | 100* | 88* | 100* | 61* |
| 88 | 100* | 100* | - | 100* | 0* |
| 89 | 83 | 100 | 100 | 100 | 94 |
| 90 | 0 | 98 | 100 | 100 | 97 |
| 91 | 100* | 100* | 92* | 100* | 33* |
| 92 | 99* | 100* | - | 100* | 33* |
| 93 | 0 | 0 | - | 64 | 0 |
| 94 | 98* | 100* | - | 100* | 11* |
| 95 | 98* | 100* | - | 100* | 0* |
| 96 | 99* | 100* | - | 100* | 70* |
| 97 | 99* | 100* | - | 100* | 43* |
| 98 | 95* | 100* | - | 100* | 6* |
| 99 | 99* | 100* | - | 100* | 14* |
| 100 | 99* | 100* | 97* | 100* | - |
| 101 | 99* | 100* | 94* | 100* | - |
| 102 | 99* | 100* | 98* | 100* | - |
| 103 | 48 | 100 | 74 | 100 | 0 |
| 104 | 98* | 100* | 91* | 100* | 0* |
| 105 | 90* | 99* | 86* | 100* | - |
| 106 | 99* | 100* | 98* | 100* | - |
| 107 | 99* | 100* | 99* | 100* | - |
| 108 | 99 | 100 | 95 | 100 | 8 |
| 109 | 99* | 100* | 92* | 100* | 0* |
| 110 | 72* | 100* | 93* | 100* | 0* |
| 111 | 94 | 100 | 93 | 100 | 0 |
| 112 | 100* | 100* | 97* | 100* | - |
| 113 | 40* | 99* | 74* | 100* | - |
| 114 | 90* | 100* | 86* | 100* | - |
| 115 | 100* | 100* | 99* | 100* | - |
| 116 | 100** | 100** | 99** | - | - |
| 117 | 100 | 100 | - | - | - |
| 118 | 99 | 100 | - | - | - |
| 119 | 99 | 100 | - | - | - |
| 120 | 100** | 100** | 99** | - | - |
| 121 | 100** | 100** | 98** | - | - |
| 122 | 87* | 91* | - | - | - |
| 123 | 99* | 100* | - | - | - |
| 124 | 99* | 100* | - | - | - |
| 125 | 98* | 100* | - | - | - |
| 126 | 87* | 100* | - | - | - |
| 127 | 100* | 100* | 93* | 100* | - |
| 128 | 99* | 100* | 98* | 100* | - |
| 129 | 98* | 100* | 68* | 100* | - |
| 130 | 96* | 99* | 86* | 100* | - |
| 131 | 99* | 100* | 97* | 100* | - |
| 132 | 100** | 99** | - | 100** | - |
| 133 | 100** | 100** | - | 100** | - |
| 134 | 99** | 100** | - | 100** | - |
| 135 | 99** | 100** | - | 100** | - |
| 136 | 99** | 100** | - | 100** | - |
| 137 | 100* | 100* | 97* | 100* | - |
| 138 | 99* | 100* | 95* | 100* | - |
| 139 | - | - | - | - | - |
| 140 | 91* | 100* | 94* | 100* | - |
| 141 | 99* | 100* | 97* | 100* | - |
| 142 | 99* | 100* | 99* | 100* | - |
| 143 | 99* | 100* | 99* | 100* | - |

## Claims

1. A compound selected from Formula 1, *N*-oxides and salts thereof, 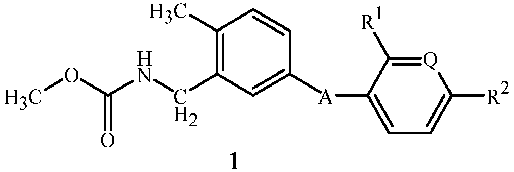 wherein
A is a radical selected from the group consisting of 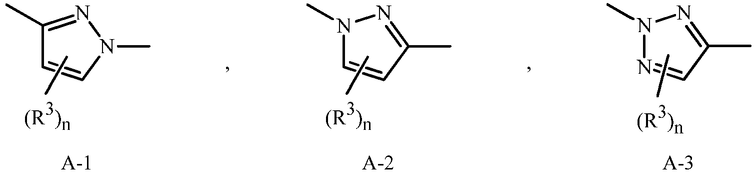 and 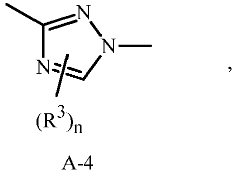 wherein the bond extending to the left of the A group is attached to the phenyl group having the CH₃O(C=O)NHCH₂ and the bond extending to the right of the A group is attached to the phenyl group having R¹ and R² substituents in Formula **1;**
Q is CH or N;
R¹ is halogen, cyano, hydroxy, nitro, amino, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
R² is halogen, cyano, hydroxy, nitro, amino, C₁-C₆ alkyl, C₃-C₆ cycloalkyl, C₄-C₁₀ cycloalkylalkyl, C₂-C₆ alkenyl, C₂-C₆ alkynyl, C₁-C₆ haloalkyl, C₁-C₆ alkoxy, C₁-C₆ haloalkoxy, C₃-C₆ cycloalkoxy, C₃-C₆ halocycloalkoxy, C₄-C₇ cycloalkylalkoxy, C₂-C₆ alkenyloxy, C₂-C₆ haloalkenyloxy, C₂-C₆ alkynyloxy, C₃-C₆ haloalkynyloxy, C₂-C₈ alkoxyalkoxy, C₂-C₈ alkoxyalkyl, C₁-C₆ cyanoalkyl, C₁-C₆ cyanoalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl or C₁-C₆ haloalkylsulfonyl;
each R³ is independently halogen, cyano, C₁-C₃ alkyl, C₁-C₃ haloalkyl, C₁-C₃ alkoxy or C₁-C₃ haloalkoxy; and
n is 0, 1 or 2.

2. A compound of Claim 1 wherein
A is a radical selected from the group consisting of A¹, A² and A³;
Q is CH;
R¹ is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy;
R² is halogen, cyano, nitro, C₁-C₆ alkyl, C₃-C₆ cycloalkyl, C₁-C₆ haloalkyl, C₁-C₆ alkoxy, C₁-C₆ haloalkoxy, C₃-C₆ cycloalkoxy, C₂-C₆ alkenyloxy or C₂-C₆ alkynyloxy;
R³ is halogen or C₁-C₃ alkyl; and
n is 0 or 1.

3. The compound of Claim 2 wherein
R¹ is C₁-C₆ alkyl, C₁-C₆ alkoxy or C₁-C₆ haloalkoxy;
R² is C₁-C₆ alkyl, C₁-C₆ alkoxy or C₁-C₆ haloalkoxy; and
n is 0.

4. The compound of Claim 3 wherein
R¹ is C₁-C₃ alkyl or C₁-C₃ alkoxy; and
R² is C₁-C₃ alkyl or C₁-C₃ alkoxy.

5. A compound of Claim 1 that is selected from the group consisting of:
methyl *N*-[[5-[1-(4-methoxy-2-methylphenyl)-1*H*-pyrazol-3-yl]-2-methylphenyl]-methyl] carbamate;
methyl *N*-[[5-[3-(4-methoxy-2-methylphenyl)-1*H*-pyrazol-1-yl]-2-methylphenyl]-methyl] carbamate;
methyl *N*-[[5-[1-(2-chloro-4-methoxyphenyl)-1*H*-pyrazol-3-yl]-2-methylphenyl]-methyl]carbamate;
methyl *N*-[[5-[3-(4-chloro-2-methoxyphenyl)-1*H*-pyrazol-1-yl]-2-methylphenyl]-methyl] carbamate;
methyl *N*-[[5-[4-(2,4-dimethoxyphenyl)-2*H*-1,2,3-triazol-2-yl]-2-methylphenyl]-methyl] carbamate;
methyl *N*-[[5-[3-(2,4-dimethoxyphenyl)-1*H*-pyrazol-1-yl]-2-methylphenyl]-methyl] carbamate;
methyl *N*-[[5-[1-(4-chloro-2-methoxyphenyl)-1*H-*pyrazol-3-yl]-2-methylphenyl]-methyl]carbamate;
methyl *N*-[[5-[4-(4-methoxy-2-methylphenyl)-2*H*-1,2,3-triazol-2-yl]-2-methylphenyl]methyl] carbamate;
methyl *N*-[[5-[3-(2,4-dichlorophenyl)-1*H*-pyrazol-1-yl]-2-methylphenyl]-methyl]carbamate;
methyl N-[[5-[1-(2,4-dimethylphenyl)-1H-pyrazol-3-yl]-2-methylphenyl]-methyl]carbamate;
methyl N-[[5-[3-(2,4-dimethylphenyl)-1H-pyrazol-1 -yl] -2-methylphenyl]-methyl] carbamate;
methyl N-[[5-[4-(2,4-dimethylphenyl)-2H-1,2,3-triazol-2-yl]-2-methylphenyl]-methyl] carbamate;
methyl N-[[5-[3-(2,6-dimethoxy-3-pyridinyl)-1H-pyrazol-1-yl]-2-methylphenyl]-methyl] carbamate;
methyl N-[[5-[3-(6-methoxy-2-methyl-3-pyridinyl)-1H-pyrazol-1-yl]-2-methylphenyl]methyl] carbamate;
methyl N-[[5-[1-(2,6-dimethoxy-3-pyridinyl)-1H-pyrazol-3-yl]-2-methylphenyl]-methyl] carbamate;
methyl N-[[5-[1-(6-methoxy-2-methyl-3-pyridinyl)-1H-pyrazol-3-yl]-2-methylphenyl] -methyl] carbamate;
methyl N-[[5-[4-(2,6-dimethoxy-3-pyridinyl)-2H-1,2,3-triazol-2-yl]-2-methylphenyl]methyl] carbamate;
methyl N-[[5-[4-(6-methoxy-2-methyl-3-pyridinyl)-2H-1,2,3-triazol-2-yl]-2-methylphenyl]methyl] carbamate;
methyl N-[[5-[1-(2,4-dimethylphenyl)-1H-1,2,4-triazol-3-yl]-2-methylphenyl]methyl] carbamate;
methyl N-[[5-[1-(6-methoxy-2-methyl-3-pyridinyl)-1H-1,2,4-triazol-3-yl]-2-methylphenyl]methyl] carbamate;
methyl N-[[5-[1-(2,6-dimethoxy-3-pyridinyl)-1H-1,2,4-triazol-3-yl]-2-methylphenyl]methyl]carbamate;
methyl N-[[2-methyl-5-[1-[2-methyl-4-(trifluoromethoxy)phenyl]-1H-pyrazol-3-yl]phenyl]methyl] carbamate;
methyl N-[[5-[1-[4-(difluoromethoxy)-2-methylphenyl]-1H-pyrazol-3-yl]-2-methylphenyl]methyl]carbamate;
methyl N-[[2-methyl-5-[3-[2-methyl-4-(trifluoromethoxy)phenyl]-1H-pyrazol-1-yl]phenyl]methyl] carbamate;
methyl N-[[5-[3-[4-(difluoromethoxy)-2-methylphenyl]-1H-pyrazol-1-yl]-2-methylphenyl]methyl]carbamate;
methyl N-[[2-methyl-5-[1-[2-methyl-4-(trifluoromethoxy)phenyl]-1H-1,2,4-triazol-3-yl]phenyl]methyl]carbamate;
methyl N-[[5-[1-[4-(difluoromethoxy)-2-methylphenyl]-1H-1,2,4-triazol-3-yl]-2-methylphenyl]methyl]carbamate;
methyl N-[[2-methyl-5-[4-[2-methyl-4-(trifluoromethoxy)phenyl]-2H-1,2,3-triazol-2-yl]phenyl]methyl]carbamate; and
methyl N-[[5-[4-[4-(difluoromethoxy)-2-methylphenyl]-2H-1,2,3-triazol-2-yl]-2-methylphenyl]methyl] carbamate.

6. A fungicidal composition comprising (a) a compound of any one of Claims 1 to 5; and (b) at least one other fungicide.

7. A fungicidal composition comprising (a) a compound of any one of Claims 1 to 5; and (b) at least one additional component selected from the group consisting of surfactants, solid diluents and liquid diluents.

8. A method for controlling plant diseases caused by fungal plant pathogens comprising applying to the plant or portion thereof, or to the plant seed, a fungicidally effective amount of a compound of any one of Claims 1 to 5.

## Patentansprüche

1. Verbindung, ausgewählt aus Formel **1**, *N*-Oxiden und Salzen davon, 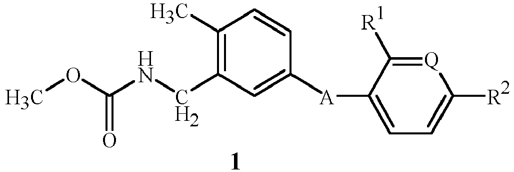 wobei
A ein Radikal ist, ausgewählt aus der Gruppe, bestehend aus 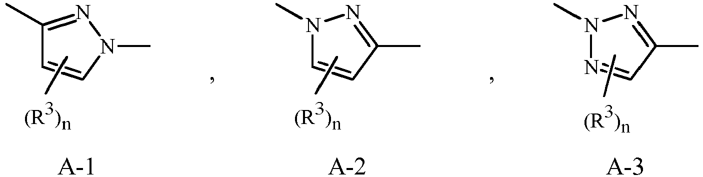 und 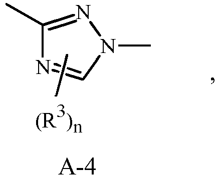 wobei die sich links der Gruppe A erstreckende Bindung an die das CH₃O(C=O)NHCH₂ aufweisende Phenylgruppe angefügt ist und die sich rechts der Gruppe A erstreckende Bindung an die Substituenten R¹ und R² aufweisende Phenylgruppe angefügt ist, in Formel **1;**
Q CH oder N ist;
R¹ Halogen, Cyano, Hydroxy, Nitro, Amino, C₁-C₆-Alkyl, C₃-C₆-Cycloalkyl, C₄-C₁₀-Cycloalkylalkyl, C₂-C₆-Alkenyl, C₂-C₆-Alkinyl, C₁-C₆-Halogenalkyl, C₁-C₆-Alkoxy, C₁-C₆-Halogenalkoxy, C₃-C₆-Cycloalkoxy, C₃-C₆-Halogencycloalkoxy, C₄-C₇-Cycloalkylalkoxy, C₂-C₆-Alkenyloxy, C₂-C₆-Halogenalkenyloxy, C₂-C₆-Alkinyloxy, C₃-C₆-Halogenalkinyloxy, C₂-C₈-Alkoxyalkoxy, C₂-C₈-Alkoxyalkyl, C₁-C₆-Cyanoalkyl, C₁-C₆-Cyanoalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl ist;
R² Halogen, Cyano, Hydroxy, Nitro, Amino, C₁-C₆-Alkyl, C₃-C₆-Cycloalkyl, C₄-C₁₀-Cycloalkylalkyl, C₂-C₆-Alkenyl, C₂-C₆-Alkinyl, C₁-C₆-Halogenalkyl, C₁-C₆-Alkoxy, C₁-C₆-Halogenalkoxy, C₃-C₆-Cycloalkoxy, C₃-C₆-Halogencycloalkoxy, C₄-C₇-Cycloalkylalkoxy, C₂-C₆-Alkenyloxy, C₂-C₆-Halogenalkenyloxy, C₂-C₆-Alkinyloxy, C₃-C₆-Halogenalkinyloxy, C₂-C₈-Alkoxyalkoxy, C₂-C₈-Alkoxyalkyl, C₁-C₆-Cyanoalkyl, C₁-C₆-Cyanoalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl ist;
jedes R³ unabhängig Halogen, Cyano, C₁-C₃-Alkyl, C₁-C₃-Halogenalkyl, C₁-C₃-Alkoxy oder C₁-C₃-Halogenalkoxy ist; und
n 0, 1 oder 2 ist.

2. Verbindung nach Anspruch 1, wobei
A ein Radikal ist, ausgewählt aus der Gruppe, bestehend aus A¹, A² und A³;
Q CH ist;
R¹ Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Cycloalkoxy, C₂-C₆-Alkenyloxy oder C₂-C₆-Alkinyloxy ist;
R² Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Cycloalkoxy, C₂-C₆-Alkenyloxy oder C₂-C₆-Alkinyloxy ist;
R³ Halogen oder C₁-C₃-Alkyl ist; und
n 0 oder 1 ist.

3. Verbindung nach Anspruch 2, wobei
R¹ C₁-C₆-Alkyl, C₁-C₆-Alkoxy oder C₁-C₆-Halogenalkoxy ist;
R² C₁-C₆-Alkyl, C₁-C₆-Alkoxy oder C₁-C₆-Halogenalkoxy ist; und
n 0 ist.

4. Verbindung nach Anspruch 3, wobei
R¹ C₁-C₃-Alkyl oder C₁-C₃-Alkoxy ist; und
R² C₁-C₃-Alkyl oder C₁-C₃-Alkoxy ist.

5. Verbindung nach Anspruch 1, die ausgewählt ist aus der Gruppe, bestehend aus:
Methyl-*N*-[[5-[1-(4-methoxy-2-methylphenyl)-1*H*-pyrazol-3-yl]-2-methylphenyl]-methyl] carbamat;
Methyl-*N*-[[5-[3-(4-methoxy-2-methylphenyl)-1*H*-pyrazol-1-yl]-2-methylphenyl]-methyl] carbamat;
Methyl-*N*-[[5-[1-(2-chlor-4-methoxyphenyl)-1*H*-pyrazol-3-yl]-2-methylphenyl]-methyl]carbamat;
Methyl-*N*-[[5-[3-(4-chlor-2-methoxyphenyl)-1*H*-pyrazol-1-yl]-2-methylphenyl]-methyl]carbamat;
Methyl-*N*-[[5-[4-(2,4-dimethoxyphenyl)-2*H*-1,2,3-triazol-2-yl]-2-methylphenyl]-methyl]carbamat;
Methyl-*N*-[[5-[3-(2,4-dimethoxyphenyl)-1*H*-pyrazol-1-yl]-2-methylphenyl]-methyl]carbamat;
Methyl-*N*-[[5-[1-(4-chlor-2-methoxyphenyl)-1*H*-pyrazol-3-yl]-2-methylphenyl]-methyl]carbamat;
Methyl-*N*-[[5-[4-(4-methoxy-2-methylphenyl)-2*H-*1,2,3-triazol-2-yl]-2-methylphenyl]methyl]carbamat;
Methyl-*N-*[[5-[3-(2,4-dichlorphenyl)-1*H*-pyrazol-1-yl]-2-methylphenyl]-methyl]carbamat;
Methyl-N-[[5-[1H-pyrazol-3-yl]-2-methylphenyl]-methyl]carbamat;
Methyl-N-[[5-[3-(2,4-dimethylphenyl)-1H-pyrazol-1-yl]-2-methylphenyl]-methyl]carbamat;
Methyl-N-[[5-[4-(2,4-dimethylphenyl)-2H-1,2,3-triazo1-2-yl]-2-methylphenyl]-methyl]carbamat;
Methyl-N-[[5-[3-(2,6-dimethoxy-3-pyridinyl)-1H-pyrazo1-1-yl]-2-methylphenyl]-methyl]carbamat;
Methyl-N-[[5-[3-(6-methoxy-2-methyl-3-pyridinyl)-1H-pyrazol-1-yl]-2-methylphenyl]methyl]carbamat;
Methyl-N-[[5-[1H-pyrazol-3-yl]-2-methylphenyl]-methyl]carbamat;
Methyl-N-[[5-[1-(6-methoxy-2-1H-pyrazol-3-yl]-2-methylphenyl] -methyl] carbamat;
Methyl-N-[[5-[4-(2,6-dimethoxy-3-pyridinyl)-2H-1,2,3-triazol-2-yl]-2-methylphenyl]methyl]carbamat;
Methyl-N-[[5-[4-(6-methoxy-2-methyl-3-pyridinyl)-2H-1,2,3-triazol-2-yl]-2-methylphenyl]methyl] carbamat;
Methyl-N-[[5-[1-(2,4-dimethylphenyl)-1H-1 ,2,4-triazol-3-yl]-2-methylphenyl]methyl]carbamat;
Methyl-N-[[5-[1-(6-methoxy-2-methyl-3-pyridinyl)-1H-1,2,4-triazol-3-yl]-2-methylphenyl]methyl]carbamat;
Methyl-N-[[5-[1-(2,6-dimethoxy-3-pyridinyl)-1H-1 ,2,4-triazol-3-yl]-2-methylphenyl]methyl]carbamat;
Methyl-N-[[2-methyl-5-[1-[2-methyl-4-(trifluormethoxy)phenyl]-1H-pyrazol-3-yl]phenyl]methyl]carbamat;
Methyl-N-[[5-[1-[4-(difluormethoxy)-2-methylphenyl]-1H-pyrazol-3-yl]-2-methylphenyl]methyl] carbamat;
Methyl-N-[[2-methyl-5-[3-[2-methyl-4-(trifluormethoxy)phenyl]-1H-pyrazol-1-yl]phenyl]methyl] carbamat;
Methyl-N-[[5-[3-[4-(difluormethoxy)-2-methylphenyl]-1H-pyrazol-1-yl]-2-methylphenyl]methyl] carbamat;
Methyl-N-[[2-methyl-5-[1-[2-methyl-4-(trifluormethoxy)phenyl]-1H-1,2,4-triazol-3-yl]phenyl]methyl]carbamat;
Methyl-N-[[5-[1-[4-(difluormethoxy)-2-methylphenyl]-1H-1,2,4-triazol-3-yl]-2-methylphenyl]methyl] carbamat;
Methyl-N-[[2-methyl-5-[4-[2-methyl-4-(trifluormethoxy)phenyl]-2H-1,2,3-triazol-2-yl]phenyl]methyl]carbamat; und
Methyl-N-[[5-[4-[4-(difluormethoxy)-2-methylphenyl]-2H-1,2,3-triazol-2-yl]-2-methylphenyl]methyl]carbamat.

6. Fungizide Zusammensetzung, umfassend (a) eine Verbindung nach einem der Ansprüche 1 bis 5; und (b) zumindest ein weiteres Fungizid.

7. Fungizide Zusammensetzung, umfassend (a) eine Verbindung nach einem der Ansprüche 1 bis 5; und (b) zumindest eine zusätzliche Komponente, ausgewählt aus der Gruppe, bestehend aus Tensiden, festen Verdünnungsmitteln und flüssigen Verdünnungsmitteln.

8. Verfahren zur Kontrolle von Pflanzenkrankheiten, verursacht durch pflanzenpathogene Pilze, umfassend Anwenden, auf die Pflanze oder einen Teil davon, oder auf den Pflanzensamen, einer fungizid wirksamen Menge einer Verbindung nach einem der Ansprüche 1 bis 5.

## Revendications

1. Composé choisi parmi la Formule 1, des N-oxydes et des sels de celui-ci, 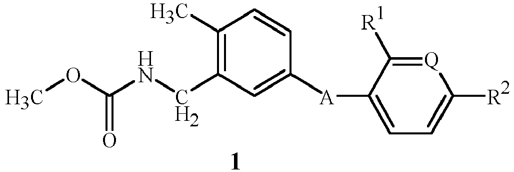 dans lequel:
A est un radical choisi dans le groupe constitué de: 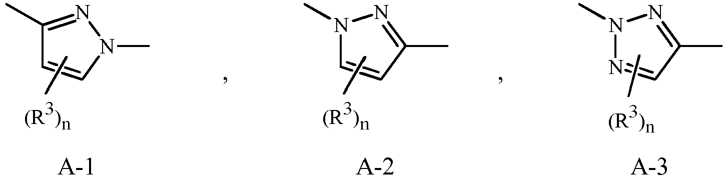 et 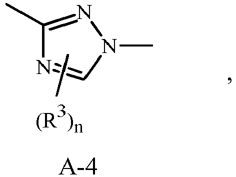 où la liaison s'étendant vers la gauche du groupe A est attachée au groupe phényle possédant CH₃O(C=O)NHCH₂ et la liaison s'étendant vers la droite du groupe A est attachée au groupe phényle possédant les substituants R¹ et R² dans la Formule 1 ;
Q est CH ou N;
R¹ est un halogène, un cyano, un hydroxy, un nitro, un amino, un C₁-C₆ alkyle, un C₃-C₆ cycloalkyle, un C₄-C₁₀ cycloalkylalkyle, un C₂-C₆ alcényle, un C₂-C₆ alcynyle, un C₁-C₆ haloalkyle, un C₁-C₆ alcoxy, un C₁-C₆ haloalcoxy, un C₃-C₆ cycloalcoxy, un C₃-C₆ halocycloalcoxy, un C₄-C₇ cycloalkylalcoxy, un C₂-C₆ alcényloxy, un C₂-C₆ haloalcényloxy, un C₂-C₆ alcynyloxy, un C₃-C₆ haloalcynyloxy, un C₂-C₈ alcoxyalcoxy, un C₂-C₈ alcoxyalkyle, un C₁-C₆ cyanoalkyle, un C₁-C₆ cyanoalcoxy, un C₁-C₆ alkylthio, un C₁-C₆ haloalkylthio, un C₁-C₆ alkylsulfinyle, un C₁-C₆ haloalkylsulfinyle, un C₁-C₆ alkylsulfonyle ou un C₁-C₆ haloalkylsulfonyle;
R² est un halogène, un cyano, un hydroxy, un nitro, un amino, un C₁-C₆, alkyle, un C₃-C₆ cycloalkyle, un C₄-C₁₀ cycloalkylalkyle, un C₂-C₆ alcényle, un C₂-C₆ alcynyle, un C₁-C₆ haloalkyle, un C₁-C₆ alcoxy, un C₁-C₆ haloalcoxy, un C₃-C₆ cycloalcoxy, un C₃-C₆ halocycloalcoxy, un C₄-C₇ cycloalkylalcoxy, un C₂-C₆ alcényloxy, un C₂-C₆ haloalcényloxy, un C₂-C₆ alcynyloxy, un C₃-C₆ haloalcynyloxy, un C₂-C₈ alcoxyalcoxy, un C₂-C₈ alcoxyalkyle, un C₁-C₆ cyanoalkyle, un C₁-C₆ cyanoalcoxy, un C₁-C₆ alkylthio, un C₁-C₆ haloalkylthio, un C₁-C₆ alkylsulfinyle, un C₁-C₆ haloalkylsulfinyle, un C₁-C₆ alkylsulfonyle ou un C₁-C₆ haloalkylsulfonyle;
chaque R³ est indépendamment un halogène, un cyano, un C₁-C₃ alkyle, un C₁-C₃ haloalkyle, un C₁-C₃ alcoxy ou un C₁-C₃ haloalcoxy; et
n est 0, 1 ou 2.

2. Composé selon la revendication 1, dans lequel:
A est un radical choisi dans le groupe constitué de A¹, A² et A³;
Q est CH;
R¹ est un halogène, un cyano, un nitro, un C₁-C₆ alkyle, un C₃-C₆ cycloalkyle, un C₁-C₆ haloalkyle, un C₁-C₆ alcoxy, un C₁-C₆ haloalcoxy, un C₃-C₆ cycloalcoxy, un C₂-C₆ alcényloxy ou un C₂-C₆ alcynyloxy;
R² est un halogène, un cyano, un nitro, un C₁-C₆ alkyle, un C₃-C₆ cycloalkyle, un C₁-C₆ haloalkyle, un C₁-C₆ alcoxy, un C₁-C₆ haloalcoxy, un C₃-C₆ cycloalcoxy, un C₂-C₆ alcényloxy ou un C₂-C₆ alcynyloxy;
R³ est un halogène ou un C₁-C₃ alkyle; et
n est 0 ou 1.

3. Composé selon la revendication 2, dans lequel:
R¹ est un C₁-C₆ alkyle, un C₁-C₆ alcoxy ou un C₁-C₆ haloalcoxy;
R² est un C₁-C₆ alkyle, un C₁-C₆ alcoxy ou un C₁-C₆ haloalcoxy; et
n est 0.

4. Composé selon la revendication 3, dans lequel:
R¹ est un C₁-C₃ alkyle ou un C₁-C₃ alcoxy; et
R² est un C₁-C₃ alkyle ou un C₁-C₃ alcoxy.

5. Composé selon la revendication 1, qui est choisi dans le groupe constitué de:
N-[[5-[1-(4-méthoxy-2-méthylphényl)-1H-pyrazol-3-yl]-2-méthylphényl]-méthyl]carbamate de méthyle;
N-[[5-[3-(4-méthoxy-2-méthylphényl)-1H-pyrazol-1-yl]-2-méthylphényl]-méthyl]carbamate de méthyle;
N-[[5-[1-(2-chloro-4-méthoxyphényl)-1H-pyrazol-3-yl]-2-méthylphényl]-méthyl]carbamate de méthyle;
N-[[5-[3-(4-chloro-2-méthoxyphényl)-1H-pyrazol-1-yl]-2-méthylphényl]-méthyl]carbamate de méthyle;
N-[[5-[4-(2,4-diméthoxyphényl)-2H-1,2,3-triazol-2-yl]-2-méthylphényl]-méthyl]carbamate de méthyle;
N-[[5-[3-(2,4-diméthoxyphényl)-1H-pyrazol-1-yl]-2-méthylphényl]-méthyl]carbamate de méthyle;
N-[[5-[1-(4-chloro-2-méthoxyphényl)-1H-pyrazol-3-yl]-2-méthylphényl]-méthyl]carbamate de méthyle;
N-[[5-[4-(4-méthoxy-2-méthylphényl)-2H-1,2,3-triazol-2-yl]-2-méthylphényl]méthyl]carbamate de méthyle;
N-[[5-[3-(2,4-dichlorophényl)-1H-pyrazol-1-yl]-2-méthylphényl]-méthyl]carbamate de méthyle;
N-[[5-[1-(2,4-diméthylphényl)-1H-pyrazol-3-yl]-2-méthylphényl]-méthyl]carbamate de méthyle;
N-[[5-[3-(2,4-diméthylphényl)-1H-pyrazol-1-yl]-2-méthylphényl]-méthyl]carbamate de méthyle;
N-[[5-[4-(2,4-diméthylphényl)-2H-1,2,3-triazol-2-yl]-2-méthylphényl]-méthyl]carbamate de méthyle;
N-[[5-[3-(2,6-diméthoxy-3-pyridinyl)-1H-pyrazol-1-yl]-2-méthylphényl]-méthyl]carbamate de méthyle;
N-[[5-[3-(6-méthoxy-2-méthyl-3-pyridinyl)-1H-pyrazol-1-yl]-2-méthylphényl]méthyl]carbamate de méthyle;
N-[[5-[1-(2,6-diméthoxy-3-pyridinyl)-1H-pyrazol-3-yl]-2-méthylphényl]-méthyl]carbamate de méthyle;
N-[[5-[1-(6-méthoxy-2-méthyl-3-pyridinyl)-1H-pyrazol-3-yl]-2-méthylphényl]-méthyl]carbamate de méthyle;
N-[[5-[4-(2,6-diméthoxy-3-pyridinyl)-2H-1,2,3-triazol-2-yl]-2-méthylphényl]méthyl]carbamate de méthyle;
N-[[5-[4-(6-méthoxy-2-méthyl-3-pyridinyl)-2H-1,2,3-triazol-2-yl]-2-méthylphényl]méthyl]-carbamate de méthyle;
N-[[5-[1-(2,4-diméthylphényl)-1H-1,2,4-triazol-3-yl]-2-méthylphényl]méthyl]carbamate de méthyle;
N-[[5-[1-(6-méthoxy-2-méthyl-3-pyridinyl)-1H-1,2,4-triazol-3-yl]-2-méthylphényl]méthyl]-carbamate de méthyle;
N-[[5-[1-(2,6-diméthoxy-3-pyridinyl)-1H-1,2,4-triazol-3-yl]-2-méthylphényl]méthyl]carbamate de méthyle;
N-[[2-méthyl-5-[1-[2-méthyl-4-(trifluorométhoxy)phényl]-1H-pyrazol-3-yl]phényl]méthyl]-carbamate de méthyle;
N-[[5-[1-[4-(difluorométhoxy)-2-méthylphényl]-1H-pyrazol-3-yl]-2-méthylphényl]méthyl]-carbamate de méthyle;
N-[[2-méthyl-5-[3-[2-méthyl-4-(trifluorométhoxy)phényl]-1H-pyrazol-1-yl]phényl]méthyl]-carbamate de méthyle;
N-[[5-[3-[4-(difluorométhoxy)-2-méthylphényl]-1H-pyrazol-1-yl]-2-méthylphényl]méthyl]-carbamate de méthyle;
N-[[2-méthyl-5-[1-[2-méthyl-4-(trifluorométhoxy)phényl]-1H-1,2,4-triazol-3-yl]phényl]méthyl]-carbamate de méthyle;
N-[[5-[1-[4-(difluorométhoxy)-2-méthylphényl]-1 H-1 ,2,4-triazol-3-yl]-2-méthylphényl]méthyl]-carbamate de méthyle;
N-[[2-méthyl-5-[4-[2-méthyl-4-(trifluorométhoxy)phényl]-2H-1,2,3-triazol-2-yl]phényl]méthyl]-carbamate de méthyle; et
N-[[5-[4-[4-(difluorométhoxy)-2-méthylphényl]-2H-1,2,3-triazol-2-yl]-2-méthylphényl]méthyl]-carbamate de méthyle.

6. Composition fongicide comprenant (a) un composé selon l'une quelconque des revendications 1 à 5; et (b) au moins un autre fongicide.

7. Composition fongicide comprenant (a) un composé selon l'une quelconque des revendications 1 à 5; et (b) au moins un composant supplémentaire choisi dans le groupe constitué de tensioactifs, de diluants solides et de diluants liquides.

8. Méthode pour un contrôle des maladies de plantes occasionnées par des pathogènes de plantes fongiques comprenant l'application sur la plante ou une portion de celle-ci, ou sur la semence de la plante, d'une quantité à effet fongicide d'un composé selon l'une quelconque des revendications 1 à 5.
